# EUROPEAN PATENT APPLICATION

(11) **EP 3 741 752 A1**
(43) Date of publication of application: **25.11.2020**
(21) Application number: 19741754.6
(22) Date of filing: 17.01.2019
(51) Int. Cl.: C07D 405/14, C07D 401/12, C07D 491/04, A61K 31/501, A61P 35/00

(54) **TAM FAMILY KINASE /AND CSF1R KINASE INHIBITOR AND USE THEREOF**

(30) Priority: 17.01.2018 CN 201810043348; 07.08.2018 CN 201810891111; 14.11.2018 CN 201811354582
(71) Applicant: Nanjing Transthera Biosciences Co., Ltd., Nanjing, Jiangsu 210032 (CN)
(72) Inventor: WU, Frank, Nanjing, Jiangsu 210032 (CN); LI, Lin, Nanjing, Jiangsu 210032 (CN); WAN, Zhonghui, Nanjing, Jiangsu 210032 (CN)
(74) Representative: Grünecker Patent- und Rechtsanwälte PartG mbB
(86) International application number: PCT/CN2019/072083
(87) International publication number: WO 2019/141202

(57) **Abstract**

The present invention provides a novel inhibitor compound of general formula (I), exhibiting excellent kinase inhibitory activity. The compound of the present invention can be used to prevent and/or treat diseases mediated by abnormal expression of TAM family kinase receptors and/or ligands thereof. In addition, the compound of the present invention can also target CSF1R kinase, and thus can be used to prevent and/or treat diseases mediated by abnormal expression of TAM family kinase receptors and/or CSF1R kinase receptors and/or ligands thereof.

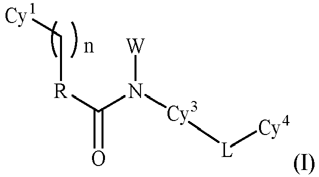

## Description

### TECHNICAL FIELD

The present invention belongs to the field of medicine, and particularly relates to the TAM family kinase and/or CSF1R kinase inhibitor compound of general formula (I), pharmaceutically acceptable salt, ester, stereoisomer and tautomer thereof, pharmaceutical composition and pharmaceutical formulation containing the same, and use thereof. The compound of the present invention can selectively inhibit tyrosine kinase TAM family and/or CSF1R kinase, and can be used for treating diseases mediated by abnormal expression of TAM family kinase and/or CSF1R kinase receptors and/or ligands thereof.

### BACKGROUND

TAM family includes three members, Axl, Mer and Tyro-3, and the family includes an extracellular domain, a transmembrane domain, and a conserved intracellular kinase domain. The extracellular domain consists of two immunoglobulin-like domains linked to two type III fibronectin repeating units. The conserved amino acid sequence KW(I/L)A(I/L)ES of the intracellular kinase domain is a unique structural feature of the TAM family. The family has a common ligand, *i.e.,* growth arrest-specific 6 protein (Gas6), which can bind to all TAM receptors, but with different binding strengths. In addition, the TAM family also includes related receptors, such as vitamin K dependent protein S (ProS), stubby-like protein, recombinant human Tubby-like protein 1 (Tulp1) and galectin-3 (Wu Yanjun, Chinese Journal of New Drugs, 2016; Lu Ping, Chinese Journal of Practical Diagnosis and Therapy, 2016).

Axl (also referred to as UFO, Ark, Tyro-7 or JTK1), Mer (also referred to as c-Mer, Mertk, Eyk, Nyk or Tyro-12), Tyro-3 (also referred to as Sky, Byk, Rse, Dtk, *etc.),* galectin-3, Gas6 and ProS are abnormally expressed in various solid tumors, such as lung cancer, gastric cancer and liver cancer, and in various hematological tumors, such as AML, ALL and CML, and thus closely correlate to poor prognosis of a disease, disease progression, tumor metastasis, tumor drug resistance, *etc.* (Douglas K, Nature Reviews, 2014). In particular, Axl, a tyrosine kinase, has been proved to be one of the causes of resistance to EGFR inhibitors in NSCLC, and is closely related to the metastasis of various solid tumors. The medicaments developed by targeting Axl also confirmed that inhibiting Axl could delay the resistance to EGFR inhibitors and the metastasis of tumors (T. Jimbo, Annals of Oncology, 2017; Sacha J. Holland, American Association for Cancer Research 2010). Moreover, Axl, Mer, Tryo-3 and TAM ligands also play a major role in tumor immunology. Inhibiting the TAM family and ligands thereof can reverse the immunosuppression in the tumor microenvironment and enhance the tumor cell killing effect of the immune system by promoting polarization of macrophages to M1-type macrophages, increasing the activation and function of effector T cells, enhancing the anti-tumor activity of NK cells, *etc.* (Yemsratch T. Akalu, Immunological Reviews, 2017; Greg Lemke, Nature Reviews Immunology, 2008). Therefore, such inhibitors can be developed to strongly inhibit and treat various solid and hematological tumors induced by the family, such as lung cancer, liver cancer, breast cancer, glioma, melanoma, AML, ALL and CML.

In addition to use in the field of tumor diseases, TAM family receptors and ligands thereof can regulate vascular smooth muscle homeostasis, platelet aggregation, thrombus stabilization, erythropoiesis, oligodendrocyte survival, osteoclast function, phagocytosis of apoptotic cells, inflammation, innate immunity and many other physiological functions. Therefore, TAM family inhibitors can also be used to treat endometriosis, vascular disease/trauma, psoriasis, visual defect/lesion (caused by macular degeneration, diabetes, premature delivery, *etc.),* kidney disease, rheumatoid arthritis, osteoporosis and other related diseases caused by the disturbance of signaling pathways of the TAM family.

Colony stimulating factor 1 receptor (CSF1R), also referred to as c-FMS, FMS, FIM2, MCSF and CD115, is a single-chain transmembrane glycoprotein composed of 972 amino acid residues, which belongs to the type III receptor tyrosine kinase (RTK) family, together with FLT-3, PDGFR and KIT. CSF-1R is only expressed on the surface of a monocyte cell line, such as macrophages. Currently, it has been reported that CSF1R ligands include colony stimulating factor 1 (CSF-1, macrophage colony-stimulating factor, also referred to as M-CSF) and interleukin-34 (IL-34). CSF-1 affects various cell functions of a monocyte cell line by binding to CSF1R; and IL-34 can tightly bind to CSF-1R and thus promote the survival, proliferation and differentiation of a monocyte cell line. Therefore, the CSF-1/IL-34/CSF-1R pathway is established.

In the CSF-1/CSF-1R pathway, the signal axis formed by CSF-1 and CSF1R can promote the growth of macrophages in the body and regulate the normal development and homeostasis of tissues and organs, and homeostatic imbalance can cause a variety of diseases, such as inflammation, immune system disease and tumor. Although macrophages have the potential to kill tumor cells, tumor-associated macrophages (TAMs) mostly play an immunosuppressive role. In particular, myeloid-derived suppressor cells (MDSCs) can promote macrophages to selectively ignore the presence of tumor cells by high expression of CSF1R, further promoting the development and metastasis of the tumor. At present, increased expression of CSF-1/CSF1R has been found in various tumors, such as breast cancer, ovarian cancer, colorectal cancer, prostate cancer, lung cancer and Hodgkin's lymphoma (O'Brien J, Am J Pathol, 2010). Overexpression of CSF-1/CSF-1R is associated with the malignant invasion and poor prognosis of a tumor. High expression of CSF1R can be detected in fibroblasts and epithelial cells induced by CSF-1, and eventually tumors are formed in nude mice, demonstrating that the CSF-1/CSF1R axis promotes the proliferation and survival of tumor cells, and plays an important role in the occurrence and development of tumors. In addition, in bone metastasis, CSF1R plays a role in osteolytic bone destruction (Ohno, Mol. Cancer Ther, 2006). Therefore, inhibiting the CSF-1/IL-34/CSF-1R signaling pathway can alleviate the immunosuppressive effect of tumors and improve the activity of the immune system, thereby inhibiting the development and metastasis of tumors. Furthermore, it has been reported that CSF-1R inhibitors can significantly reduce the size of tumors such as glioblastoma, and reduce the invasion and proliferation of tumors (Pyonteck Stephanie M, Nature Medicine, 2013). In summary, inhibiting the CFS1R pathway has become one of the main ways for treating cancers.

Both TAM and CSF1R inhibitors can improve the immunosuppression in the tumor microenvironment and enhance the tumor cell killing ability of the immune system. However, based on the complexity of the tumor pathogenesis, a double effect can be achieved if these two targets can be targeted simultaneously. At present, CSF1R inhibitors are more commonly used in combination with PD-1 clinically, and there are no inhibitors targeting TAM and/or CSF1R on the market.

### SUMMARY

The present invention provides a novel inhibitor compound, and pharmaceutically acceptable salt, ester, stereoisomer or tautomer thereof (hereinafter, sometimes referred to as the compound of the present invention). The compound of the present invention has inhibitory effect on TAM family kinases. In addition, the compound of the present invention can also target CSF1R kinase and exhibits inhibitory effect on CSF1R kinase. The compound of the present invention can be used to treat and/or prevent diseases mediated by abnormal expression of TAM family kinase receptors and/or ligands thereof. In addition, the compound of the present invention can be used to treat and/or prevent diseases mediated by abnormal expression of TAM family kinase and/or CSF1R kinase receptors and/or ligands thereof. The compound of the present invention reverses the immunosuppression in the tumor microenvironment and inhibits the growth, migration and/or drug resistance of the tumor by inhibiting TAM family kinase and/or CSF1R kinase, thereby exerting the tumor immunology effect and anti-tumor efficacy.

Specifically, the present invention provides the following technical solutions.

A compound of general formula (I), or a pharmaceutically acceptable salt, ester, stereoisomer or tautomer thereof is provided: 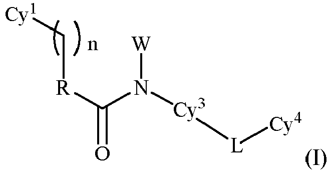
wherein, W is selected from hydrogen and optionally substituted C₁₋₆ alkyl;
R represents a group shown as the following general formula (a), (b) or (c), 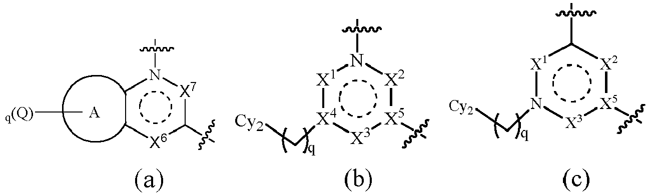
in formula (a), ring A represents 6-10 membered aromatic ring, 5-6 membered heteroaromatic ring having 1-3 heteroatoms selected from NR^{b}, O and S, or 5-6 membered heterocyclic ring having 1-4 heteroatoms selected from NR^{b}, O and S;
Q is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl; and
q is an integer of 0 to 4;
in formula (a), 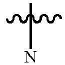 is linked to Cy¹ via a linking group; in formula (a), X⁶ and X⁷ are each independently selected from CR^{a}R^{a}, C=O and NR^{b};
in formula (b), 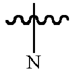 is linked to Cy¹ via a linking group;
in formula (c), 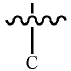 is linked to Cy¹ via a linking group;
X¹, X² and X³ are each independently selected from CR^{a}R^{a}, C=O, NR^{b} and O, and at least one of X¹, X² and X³ is C=O;
X⁴ and X⁵ are each independently selected from CR^{a} and N;
Cy¹ is selected from 3-12 membered heterocyclyl optionally substituted with one or more R¹ and 3-12 membered cycloalkyl optionally substituted with one or more R¹, and R¹ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, - NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy² is selected from 6-14 membered aryl optionally substituted with one or more R² and 5-10 membered heteroaryl optionally substituted with one or more R², and R² is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, - NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy³ is selected from the group consisting of 3-12 membered cycloalkyl optionally substituted with one or more R³, 3-14 membered heterocyclyl optionally substituted with one or moreR³, 5-10 membered heteroaryl optionally substituted with one or more R³ and 6-14 membered aryl optionally substituted with one or more R³, and R³ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy⁴ is selected from 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl, or, two R⁴, together with the atoms attached thereto, can form 5-14 membered cyclic group;
L is selected from the group consisting of -NRb-, -O-, -S-, -(CR^{a}R^{a})ₘ-, optionally substituted 3-12 membered cycloalkyl, optionally substituted 3-14 membered heterocyclyl, optionally substituted 5-14 membered heteroaryl and optionally substituted 6-14 membered aryl, wherein m is an integer of 0 to 3;
R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', -SO₂-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R' is selected from the group consisting of optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
the substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo; 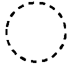
in formula (a), (b) and (c) represents a double bond optionally present in the ring structure;
with the proviso that when the ring in formula (b) carries two carbonyls, Cy¹ represents 3-12 membered heterocyclyl that is optionally substituted with one or more R¹ and contains 1-3 heteroatoms selected from O, S, S(O) and S(O)₂, and Cy³-L does not form aryloxy;
when, in group shown as formula (c), at least one of X¹ and X³ represents NR^{b}, Cy¹ represents 3-12 membered heterocyclyl that is optionally substituted with one or more R¹ and contains 1-3 heteroatoms selected from O, S, S(O) and S(O)₂;
when, in group shown as formula (b), X¹ and X² are CH, X³ is C=O, and X⁴ and X⁵ are C, L does not represent heteroaryl;
when, in group shown as formula (c), X¹ and X² are CH, X³ is C=O, and X⁵ is C, Cy¹ represents 3-12 membered heterocyclyl that is optionally substituted with one or more R¹ and contains 1-3 heteroatoms selected from O, S, S(O) and S(O)₂, and Cy² represents 6-14 membered aryl optionally substituted with one or more R²;
the case where two carbonyls are directly bonded in the ring structure is impossible; and
n is an integer of 0 to 4.

In another embodiment, a compound of formula (I) or a pharmaceutically acceptable salt, ester, stereoisomer or tautomer thereof is provided: 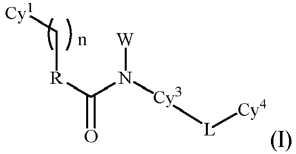
wherein, W is selected from hydrogen and optionally substituted C₁₋₆ alkyl;
R represents a group shown as general formula (a), 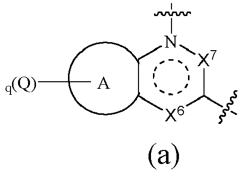
in formula (a), ring A represents 6-10 membered aromatic ring, 5-6 membered heteroaromatic ring having 1-3 heteroatoms selected from NR^{b}, O and S, or 5-6 membered heterocyclic ring having 1-4 heteroatoms selected from NR^{b}, O and S;
Q is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
q is an integer of 0 to 4;
in formula (a), 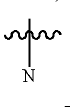 is linked to Cy¹ via a linking group;
in formula (a), X⁶ and X⁷ are each independently selected from CR^{a}R^{a}, C=O and NR^{b}, and at least one of X⁶ and X⁷ is C=O;
Cy¹ is selected from 3-12 membered heterocyclyl optionally substituted with one or more R¹ and 3-12 membered cycloalkyl optionally substituted with one or more R¹, and R¹ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, - NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy³ is selected from the group consisting of 3-12 membered cycloalkyl optionally substituted with one or more R³, 3-14 membered heterocyclyl optionally substituted with one or more R³, 5-10 membered heteroaryl optionally substituted with one or more R³ and 6-14 membered aryl optionally substituted with one or more R³, and R³ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy⁴ is selected from the group consisting of 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl, or, two R⁴, together with the atoms attached thereto, can form 5-6 membered cyclic group;
L is selected from the group consisting of -NRb-, -O-, -S-, -(CR^{a}R^{a})ₘ-, optionally substituted 3-12 membered cycloalkyl, optionally substituted 3-14 membered heterocyclyl, optionally substituted 5-14 membered heteroaryl and optionally substituted 6-14 membered aryl, wherein m is an integer of 0 to 3;
R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', -SO₂-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R' is selected from the group consisting of optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
the substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo; 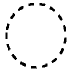
in formula (a) represents a double bond optionally present in the ring structure; and
n is an integer of 0 to 4.

In one embodiment of the present invention, Cy¹ represents 3-12 membered heterocyclyl optionally substituted with one or more R¹, and ring A represents 6-10 membered aromatic ring, or 5-6 membered heteroaromatic ring having 1-4 heteroatoms selected from NR^{b}, O and S.

In one embodiment of the present invention, Cy¹ represents 3-12 membered heterocyclyl that is optionally substituted with one or more R¹ and contains 1-3 heteroatoms selected from O, NR^{b}, S, S(O) and S(O)₂, and ring A represents 6-10 membered aromatic ring.

In one embodiment of the present invention, the above ring A represents benzene ring, furan ring, thiophene ring, pyrrole ring, oxazole ring, isoxazole ring, thiazole ring, isothiazole ring, pyrazole ring, imidazole ring, pyridine ring, pyridazine ring, pyrimidine ring, pyrazine ring, pyran ring, thiopyran ring, pyrrolidine ring, pyrroline ring, tetrahydrofuran ring, tetrahydrothiophene ring, piperidine ring or tetrahydropyran ring.

In one embodiment of the present invention, Q is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', - SO₂-R', -NR^{b}C(O)-R', optionally substituted 3-10 membered cycloalkyl, optionally substituted 3-10 membered heterocyclyl, optionally substituted 6-10 membered aryl and optionally substituted 5-10 membered heteroaryl;

In one embodiment of the present invention, in the formula (a), X⁶ represents C=O and X⁷ represents CR^{a}R^{a}.

In one embodiment of the present invention, in the formula (a), X⁶ represents C=O, X⁷ represents CR^{a}R^{a}, and there is a double bond between X⁷ and an adjacent C atom.

In one embodiment of the present invention, in the formula (a), X⁶ represents CR^{a}R^{a} and X⁷ represents C=O.

In one embodiment of the present invention, q is 0, 1, 2 or 3.

In one embodiment of the present invention, a compound of formula (I) or a pharmaceutically acceptable salt, ester, stereoisomer or tautomer thereof is provided, 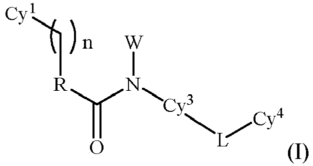
wherein, W is selected from hydrogen and optionally substituted C₁₋₆ alkyl;
R represents a group shown as the following formula (b) or (c), 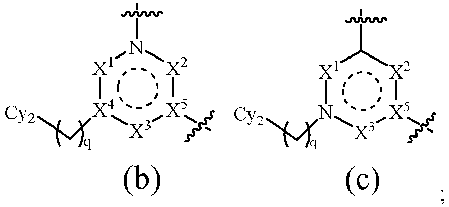
in formula (b), 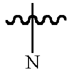 is linked to Cy¹ via a linking group;
in formula (c), 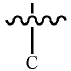 is linked to Cy¹ via a linking group;
q represents 0, 1, 2 or 3;
X¹, X² and X³ are each independently selected from CR^{a}R^{a}, C=O, NR^{b} and O, and at least one of X¹, X² and X³ is C=O;
X⁴ and X⁵ are each independently selected from CR^{a} and N;
Cy¹ is selected from 3-12 membered heterocyclyl optionally substituted with one or more R¹ and 3-12 membered cycloalkyl optionally substituted with one or more R¹, and R¹ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, - NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy² is selected from 6-14 membered aryl optionally substituted with one or more R² and 5-10 membered heteroaryl optionally substituted with one or more R², and R² is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, - NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy³ is selected from the group consisting of 3-12 membered cycloalkyl optionally substituted with one or more R³, 3-14 membered heterocyclyl optionally substituted with one or more R³, 5-10 membered heteroaryl optionally substituted with one or more R³ and 6-14 membered aryl optionally substituted with one or more R³, and R³ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy⁴ is selected from the group consisting of 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
L is selected from the group consisting of -NRb-, -O-, -S-, -(CR^{a}R^{a})ₘ-, optionally substituted 3-12 membered cycloalkyl, optionally substituted 3-14 membered heterocyclyl, optionally substituted 5-14 membered heteroaryl and optionally substituted 6-14 membered aryl, wherein m is an integer of 0 to 3;
R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', -SO₂-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R' is selected from the group consisting of optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
the substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂ aminocarbonyl, C₁₋₆ alkylcarbonyl, C₁₋₆ alkylcarbonyloxy, C₁₋₆ alkylcarbonylamino, C_{1- 6} alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo; 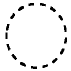
in formula (b) and (c) represents a double bond optionally present in the ring structure;
with the proviso that when the ring in formula (b) carries two carbonyls, Cy¹ represents 3-12 membered heterocyclyl that is optionally substituted with one or more R¹ and contains 1-3 heteroatoms selected from O, S, S(O) and S(O)₂, and Cy³-L does not form aryloxy;
when, in group shown as formula (c), at least one of X¹ and X³ represents NR^{b}, Cy¹ represents 3-12 membered heterocyclyl that is optionally substituted with one or more R¹ and contains 1-3 heteroatoms selected from O, S, S(O) and S(O)₂;
when, in group shown as formula (b), X¹ and X² are CH, X³ is C=O, and X⁴ and X⁵ are C, L does not represent heteroaryl;
when, in group shown as formula (c), X¹ and X² are CH, X³ is C=O, and X⁵ is C, Cy¹ represents 3-12 membered heterocyclyl that is optionally substituted with one or more R¹ and contains 1-3 heteroatoms selected from O, S, S(O) and S(O)₂, and Cy² represents 6-14 membered aryl optionally substituted with one or more R²;
the case where two carbonyls are directly bonded in the ring structure is impossible; and
n is an integer of 0 to 4.

In one embodiment of the present invention, X¹, X² and X³ are each independently selected from CR^{a}R^{a}, C=O and NR^{b}, and at least one of X¹, X² and X³ is C=O.

In one embodiment of the present invention, X³ represents C=O.

In one embodiment of the present invention, there is at least one double bond in the ring of formula (b).

In one embodiment of the present invention, there is at least one double bond in the ring of formula (c).

In one embodiment of the present invention, two of X¹, X² and X³ represent C=O, and the one other than C=O is linked to an adjacent group via a double bond.

In one embodiment of the present invention, when, in group shown as formula (b), X¹ and X² are CH, X³ is C=O, and X⁴ and X⁵ are C, Cy¹ represents 3-12 membered heterocyclyl that is optionally substituted with one or more R¹ and contains 1-3 heteroatoms selected from O, S, S(O) and S(O)₂, Cy² represents 6-14 membered aryl optionally substituted with one or more R², and L does not represent heteroaryl.

In one embodiment of the present invention, when, in group shown as formula (b), X¹ and X² are CH, X³ is C=O, and X⁴ and X⁵ are C, L does not represent heteroaryl and heterocyclyl.

In one embodiment of the present invention, when, in group shown as formula (b), X³ is C, and at least one of X¹ and X² is C=O, Cy¹ represents 3-12 membered heterocyclyl that is optionally substituted with one or more R¹ and contains 1-3 heteroatoms selected from O, S, S(O) and S(O)₂, and Cy² represents 6-14 membered aryl optionally substituted with one or more R².

In one embodiment of the present invention, in group shown as formula (c), Cy¹ represents 3-12 membered heterocyclyl that is optionally substituted with one or more R¹ and contains 1-3 heteroatoms selected from O, S, S(O) and S(O)₂.

In one embodiment of the present invention, a compound of formula (I) or a pharmaceutically acceptable salt, ester, stereoisomer or tautomer thereof is provided, having a structure of general formula (II), 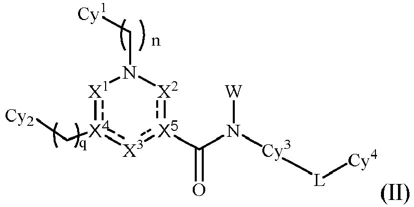
wherein, -̅-̅-̅-̅ represents a single bond or a double bond;
q represents 0, 1, 2 or 3;
X¹, X² and X³ are each independently selected from CR^{a}R^{a}, C=O and NR^{b}, and at least one of X¹, X² and X³ is C=O; and
X⁴ and X⁵ are each independently selected from CR^{a} and N.

In one embodiment of the present invention, a compound of formula (I) or a pharmaceutically acceptable salt, ester, stereoisomer or tautomer thereof is provided, having a structure of general formula (III), 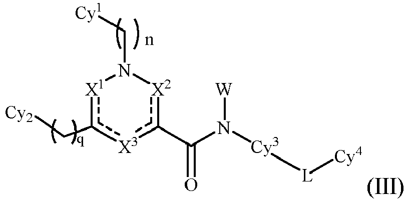 wherein,
-̅-̅-̅-̅ represents a single bond or a double bond;
q represents 0, 1, 2 or 3; and
X¹, X² and X³ are each independently selected from CR^{a}R^{a}, C=O and NR^{b}, and at least one of X¹, X² and X³ is C=O.

In one embodiment of the present invention, a compound of formula (I) or a pharmaceutically acceptable salt, ester, stereoisomer or tautomer thereof is provided, having a structure of general formula (IV), 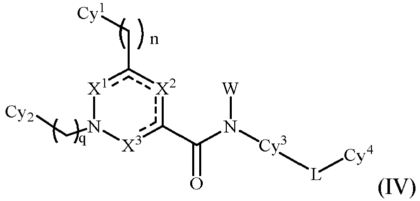 wherein,
-̅-̅-̅-̅ represents a single bond or a double bond;
q represents 0, 1, 2 or 3; and
X¹, X² and X³ are each independently selected from CR^{a}R^{a}, C=O and NR^{b}, and at least one of X¹, X² and X³ is C=O.

In one embodiment of the present invention, Cy¹ is selected from 4-10 membered heterocyclyl optionally substituted with one or more R¹.

In one embodiment of the present invention, Cy¹ is selected from 3-10 membered cycloalkyl optionally substituted with one or more R¹.

In one embodiment of the present invention, R¹ is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted - C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl.

In one embodiment of the present invention, Cy¹ is selected from 4-8 membered heterocyclyl optionally substituted with one or more R¹.

In one embodiment of the present invention, heterocyclyl of Cy¹ contains 1-3 heteroatoms selected from O, NR^{b}, S, S(O) and S(O)₂.

In one embodiment of the present invention, Cy² is selected from 6-10 membered aryl optionally substituted with one or more R².

In one embodiment of the present invention, Cy² is selected from 5-6 membered heteroaryl optionally substituted with one or more R².

In one embodiment of the present invention, R² is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted - C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl.

In one embodiment of the present invention, heteroaryl of Cy² contains 1-3 heteroatoms selected from O, NR^{b}, S, S(O) and S(O)₂.

In one embodiment of the present invention, Cy² is selected from phenyl and naphthyl optionally substituted with one or more R².

In one embodiment of the present invention, Cy³ is selected from 3-8 membered cycloalkyl optionally substituted with one or more R³.

In one embodiment of the present invention, Cy³ is selected from 3-6 membered cycloalkyl optionally substituted with one or R³.

In one embodiment of the present invention, Cy³ is selected from 5-6 membered heteroaryl optionally substituted with one or more R³.

In one embodiment of the present invention, Cy³ is selected from 6-10 membered aryl optionally substituted with one or more R³.

In one embodiment of the present invention, Cy³ is selected from phenyl or naphthyl optionally substituted with one or more R³.

In one embodiment of the present invention, R³ is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted - C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl.

In one embodiment of the present invention, heteroaryl of Cy³ contains 1-3 heteroatoms selected from O, NR^{b} and S.

In one embodiment of the present invention, Cy⁴ is selected from 5-10 membered heteroaryl optionally substituted with one or more R⁴.

In one embodiment of the present invention, Cy⁴ is selected from 9-10 membered heteroaryl optionally substituted with one or more R⁴.

In one embodiment of the present invention, Cy⁴ is selected from 6-10 membered aryl optionally substituted with one or more R⁴.

In one embodiment of the present invention, Cy⁴ is selected from phenyl or naphthyl optionally substituted with one or more R⁴.

In one embodiment of the present invention, R⁴ is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted - C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl.

In one embodiment of the present invention, two R⁴, together with the atoms attached thereto, can form 5-14 membered cyclic group.

In one embodiment of the present invention, two R⁴, together with the atoms attached thereto, can form 5-10 membered cyclic group.

In one embodiment of the present invention, two R⁴, together with the atoms attached thereto, can form 5-6 membered cyclic group.

In one embodiment of the present invention, two R⁴, together with the atoms attached thereto, can form 5-6 membered oxygen-containing cyclic group.

In one embodiment of the present invention, heteroaryl of Cy⁴ contains 1-4 atoms selected from O, NR^{b} and S.

In one embodiment of the present invention, L is selected from -NRb-, -O-, -S- and optionally substituted 5-10 membered heteroaryl.

In one embodiment of the present invention, heteroaryl of L contains 1-4 atoms selected from O, NR^{b} and S.

In one embodiment of the present invention, when L is selected from heteroaryl and heterocyclyl, at least one of X¹ and X² in formula (b) is C=O.

In one embodiment of the present invention, when L is selected from heteroaryl and heterocyclyl, at least one of X¹ and X³ in formula (c) is C=O.

In one embodiment of the present invention, R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl.

In one embodiment of the present invention, R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', - C(O)-R', -SO₂-R', optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl.

In one embodiment of the present invention, R' is selected from the group consisting of optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl.

In one embodiment of the present invention, R' is selected from the group consisting of optionally substituted 4-6 membered cycloalkyl, optionally substituted 4-6 membered cycloalkenyl, optionally substituted 4-10 membered heterocyclyl, optionally substituted 6-10 membered aryl and optionally substituted 5-6 membered heteroaryl.

In one embodiment of the present invention, the substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-10 membered aryl, 3-8 membered heterocyclyl, 5-6 membered heteroaryl and oxo.

In one embodiment of the present invention, substituents involved in the phrase "optionally substituted" are each independently selected from the group consisting of hydroxyl, mercapto, amino, carboxyl, cyano, nitro, halogen, C₁₋₄ alkyl, C₁₋₄ alkoxy C₁₋₄ alkyl, C₁₋₄ alkoxy, C₁₋₄ alkyl C₁₋₄ alkoxy, C₁₋₄ alkoxy C₁₋₄ alkoxy, C₁₋₄ alkylamino, (C₁₋₄ alkyl)₂amino, C₁₋₄ alkylester, C₁₋₄ alkylaminocarbonyl, (C₁₋₄ alkylaminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, cyclopropyl, cyclobutyl, cyclopentyl, cyclohexyl, cycloheptyl, phenyl, naphthyl, oxa-cyclopropyl, oxa-cyclobutyl, oxacyclopentyl, oxa-cyclohexyl, oxa-cycloheptyl, pyrrolyl, furanyl, thienyl, oxazolyl, isoxazolyl, pyrazolyl, thiazolyl, pyridyl, pyrimidinyl, pyridazinyl and oxo.

In one embodiment of the present invention, n is 0, 1, 2 or 3.

In one embodiment of the present invention, X¹ is N, X² is CR^{a}, and X³ is C=O.

In one embodiment of the present invention, X¹ is CR^{a}, X² is N, and X³ is C=O.

In one embodiment of the present invention, X¹ is C=O, X² is CR^{a}, and X³ is C=O.

In one embodiment of the present invention, X¹ is CR^{a}, X² is CR^{a}, and X³ is C=O.

In one embodiment of the present invention, among X¹, X² and X³, the one other than C=O is linked to an adjacent group via a double bond.

In one embodiment of the present invention, the ring in the group shown as formula (b) is represented by any one of the following formulae, and in these formulae, -̅-̅-̅-̅ represents a single bond or a double bond,

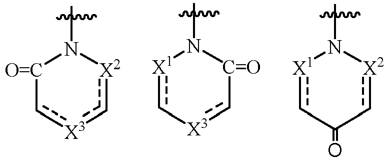

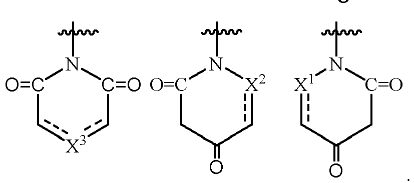

In one embodiment of the present invention, the ring in the group shown as formula (c) is represented by any one of the following formulae, and in these formulae, -̅-̅-̅-̅ represents a single bond or a double bond,

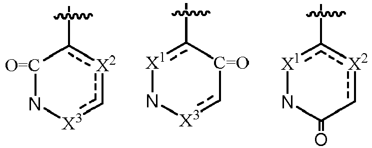

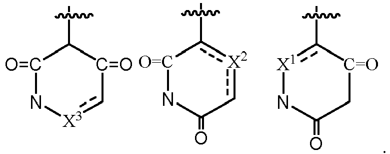

In one embodiment of the present invention, in formula (b), X¹, X² and X³ are each independently selected from CR^{a}, C=O and NR^{b}, and at least one of X¹, X² and X³ is C=O; and
X⁴ is selected from C and N, and X⁵ is selected from C.

In one embodiment of the present invention, X¹ and X³ are each independently selected from CR^{a}, C=O and NR^{b}, and X¹ and X³ cannot be both C=O; and X² is CR^{a} or NR^{b}.

In one embodiment of the present invention, Cy¹ is selected from 4-6 membered heterocyclyl optionally substituted with one or more R¹.

In one embodiment of the present invention, Cy¹ is selected from 4-6 membered heterocyclyl that is optionally substituted with one or more R¹ and contains 1-3 heteroatoms selected from O, NR^{b}, S, S(O) and S(O)₂.

In one embodiment of the present invention, Cy² is selected from phenyl or naphthyl optionally substituted with one or more R² and pyridyl optionally substituted with one or more R².

In one embodiment of the present invention, Cy³ is a group shown as 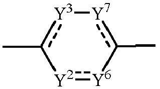 optionally substituted with one or more R³, and in the formula, -̅-̅-̅ represents a single bond or a double bond, and Y², Y³, Y⁶ and Y⁷ are each independently selected from CR^{a}R^{a} and NR^{b}.

In one embodiment of the present invention, at least one of Y², Y³, Y⁶ and Y⁷ is NR^{b}.

In one embodiment of the present invention, Cy³ is a group shown as 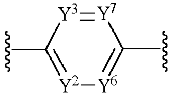 optionally substituted with one or more R³, and in the formula, Y², Y³, Y⁶ and Y⁷ are each independently selected from CH and N, and at least one of Y², Y³, Y⁶ and Y⁷ is N.

In one embodiment of the present invention, Cy³ is selected from cyclohexyl optionally substituted with one or more R³.

In one embodiment of the present invention, Cy³ is selected from thienyl optionally substituted with one or more R³.

In one embodiment of the present invention, Cy⁴ is a group shown as 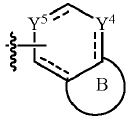 optionally substituted with one or more R⁴, and in the formula, -̅-̅-̅-̅ represents a single bond or a double bond, Y⁴ and Y⁵ are each independently selected from CR^{a}R^{a} and NR^{b} and at least one of Y⁴ and Y⁵ is NR^{b}, and ring B is benzene ring, naphthalene ring or 5-10 membered heteroaromatic ring.

In one embodiment of the present invention, Cy⁴ is a group shown as 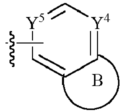 optionally substituted with one or more R⁴, and in the formula, Y⁴ and Y⁵ are each independently selected from C and N, and at least one of Y⁴ and Y⁵ is N.

In one embodiment of the present invention, ring B is benzene ring or 5-6 membered heteroaromatic ring having 1-3 heteroatoms selected from NR^{b}, O and S.

In one embodiment of the present invention, Cy¹ is selected from the following groups optionally substituted with one or more R¹:

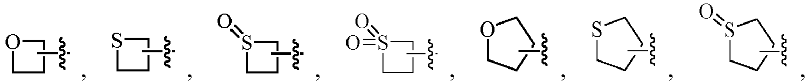

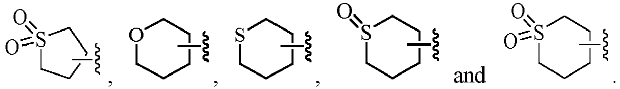

In one embodiment of the present invention, Cy² is selected from the following groups optionally substituted with one or more R²:

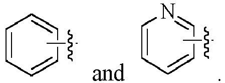

In one embodiment of the present invention, Cy³ is selected from the following groups optionally substituted with one or more R³: 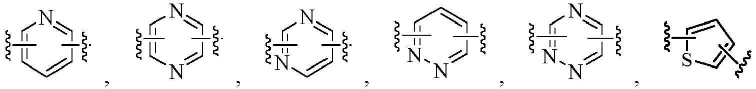 and

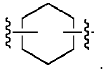

In one embodiment of the present invention, Cy⁴ is selected from the following groups optionally substituted with one or more R⁴:

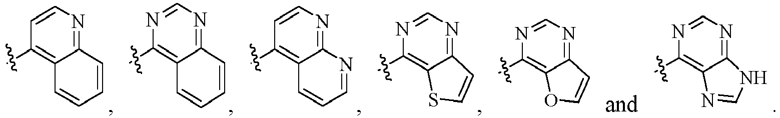

In one embodiment of the present invention, Cy¹ is selected from the following groups optionally substituted with one or more R¹:

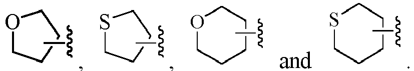

In one embodiment of the present invention, Cy² is selected from the following group optionally substituted with one or more R²:

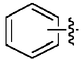

In one embodiment of the present invention, Cy³ is selected from the following groups optionally substituted with one or more R³:

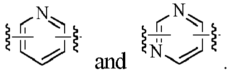

In one embodiment of the present invention, Cy³ is selected from the following groups optionally substituted with one or more R³: 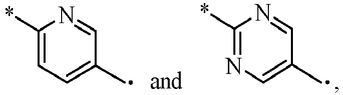 wherein * terminal is linked to N, and • terminal is linked to L.

In one embodiment of the present invention, Cy⁴ is selected from the following group optionally substituted with one or more R⁴:

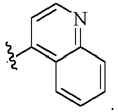

In one embodiment of the present invention, Cy¹ represents 3-12 membered heterocyclyl optionally substituted with one or more R¹, and Cy² represents 6-14 membered aryl optionally substituted with one or more R².

In one embodiment of the present invention, R¹ in Cy¹ is each independently selected from the group consisting of hydrogen, hydroxyl, mercapto, halogen, carboxyl, nitro, -NR^{b}R^{c}, - C(O)R^{d}, -C(O)OR^{d}, -C(O)NR^{b}R^{c}, -NR^{b}C(O)R^{d}, optionally substituted C₁₋₆ alkyl and optionally substituted C₁₋₆ alkoxy.

In one embodiment of the present invention, R¹ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy.

In one embodiment of the present invention, R¹ is each independently selected from hydrogen, hydroxyl, fluorine, chlorine, bromine and C₁₋₄ alkyl.

In one embodiment of the present invention, R² in Cy² is each independently selected from the group consisting of hydrogen, hydroxyl, mercapto, halogen, carboxyl, nitro, -NR^{b}R^{c}, - C(O)R^{d}, -C(O)OR^{d}, -C(O)NR^{b}R^{c}, -NR^{b}C(O)R^{d}, optionally substituted C₁₋₆ alkyl and optionally substituted C₁₋₆ alkoxy.

In one embodiment of the present invention, R² is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy.

In one embodiment of the present invention, R² is each independently selected from hydrogen, hydroxyl, fluorine, chlorine, bromine and C₁₋₄ alkyl.

In one embodiment of the present invention, R³ in Cy³ is each independently selected from the group consisting of hydrogen, hydroxyl, mercapto, halogen, carboxyl, nitro, -NR^{b}R^{c}, - C(O)R^{d}, -C(O)OR^{d}, -C(O)NR^{b}R^{c}, -NR^{b}C(O)R^{d}, optionally substituted C₁₋₆ alkyl and optionally substituted C₁₋₆ alkoxy.

In one embodiment of the present invention, R³ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy.

In one embodiment of the present invention, R³ is each independently selected from hydrogen, hydroxyl, fluorine, chlorine, bromine and C₁₋₄ alkyl.

In one embodiment of the present invention, R⁴ in Cy⁴ is each independently selected from the group consisting of hydrogen, hydroxyl, mercapto, halogen, carboxyl, nitro, -NR^{b}R^{c}, - C(O)R^{d}, -C(O)OR^{d}, -C(O)NR^{b}R^{c}, -NR^{b}C(O)R^{d}, optionally substituted C₁₋₆ alkyl and optionally substituted C₁₋₆ alkoxy, or, two R⁴, together with the atoms attached thereto, can form 5-6 membered cyclic group.

In one embodiment of the present invention, R⁴ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy and C₁₋₆ alkoxy C₁₋₆ alkoxy, or, two R⁴, together with the atoms attached thereto, can form 5-6 membered oxygen-containing cyclic group.

In one embodiment of the present invention, R⁴ is selected from the group consisting of hydrogen, hydroxyl, mercapto, halogen, C₁₋₄ alkyl, halogenated C₁₋₄ alkyl, C₁₋₄ alkoxy, C₁₋₄ alkoxy C₁₋₄ alkyl, C₁₋₄ alkyl C₁₋₄ alkoxy, C₁₋₄ alkoxy C₁₋₄ alkoxy and halogenated C₄ alkoxy.

In one embodiment of the present invention, two R⁴, together with the atoms attached thereto, can form 5-6 membered oxygen-containing cyclic group.

In one embodiment of the present invention, L is selected from -NRb-, -O-, -S- and optionally substituted 5-10 membered heteroaryl.

In one embodiment of the present invention, L is selected from -O-, -S- and optionally substituted 5-6 membered heteroaryl containing 1-3 heteroatoms selected from NR^{b}, O and S.

In one embodiment of the present invention, L is -O-.

In one embodiment of the present invention, L represents optionally substituted pyridyl.

In one embodiment of the present invention, R^{a} is present or absent, and when present, R^{a} is each independently selected from the group consisting of hydrogen, cyano, hydroxyl, mercapto, halogen, carboxyl, nitro, C₁₋₆ alkyl, C₁₋₆ alkoxy and halogenated C₁₋₆ alkyl.

In one embodiment of the present invention, R^{a} is present or absent, and when present, R^{a} is each independently selected from hydrogen, C₁₋₆ alkyl and C₁₋₆ alkoxy.

In one embodiment of the present invention, R^{b}, R^{c} and R^{d} are each present or absent, and when present, R^{b}, R^{c} and R^{d} are each independently selected from the group consisting of hydrogen, cyano, hydroxyl, mercapto, halogen, carboxyl, nitro, C₁₋₆ alkyl, C₁₋₆ alkoxy and halogenated C₁₋₆ alkyl.

In one embodiment of the present invention, R^{b}, R^{c} and R^{d} are each present or absent, and when present, R^{b}, R^{c} and R^{d} are each independently selected from hydrogen, C₁₋₆ alkyl and C_{1- 6} alkoxy.

In one embodiment of the present invention, a compound of formula (I) or a pharmaceutically acceptable salt, ester, stereoisomer or tautomer thereof is provided, having a structure of general formula (V), 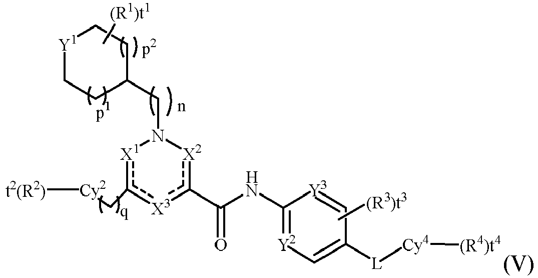
wherein, -̅-̅-̅-̅ represents a single bond or a double bond, X¹, X² and X³ are each independently selected from CR^{a}R^{a}, C=O and NR^{b}, and at least one of X¹, X² and X³ is C=O;
Y¹ is selected from O, S, S(O) and S(O)₂;
Cy² is selected from phenyl and 5-6 membered heteroaryl;
Y² and Y³ are each independently selected from C and N, and at least one of Y² and Y³ is N;
Cy⁴ is a group shown as 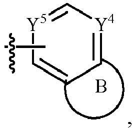 Y⁴ and Y⁵ are each independently selected from C and N, and at least one of Y⁴ and Y⁵ is N, and ring B is benzene ring or 5-6 membered heteroaromatic ring;
R¹ is selected from the group consisting of hydrogen, hydroxyl, mercapto, halogen, carboxyl, nitro, -NR^{b}R^{c}, -C(O)R^{d}, -C(O)OR^{d}, -C(O)NR^{b}R^{c}, -NR^{b}C(O)R^{d}, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy;
R² is selected from the group consisting of hydrogen, hydroxyl, mercapto, halogen, carboxyl, nitro, -NR^{b}R^{c}, -C(O)R^{d}, -C(O)OR^{d}, -C(O)NR^{b}R^{c}, -NR^{b}C(O)R^{d}, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy;
R³ is selected from the group consisting of hydrogen, hydroxyl, mercapto, halogen, carboxyl, nitro, -NR^{b}R^{c}, -C(O)R^{d}, -C(O)OR^{d}, -C(O)NR^{b}R^{c}, -NR^{b}C(O)R^{d}, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy;
R⁴ is selected from the group consisting of hydrogen, hydroxyl, mercapto, halogen, carboxyl, nitro, -NR^{b}R^{c}, -C(O)R^{d}, -C(O)OR^{d}, -C(O)NR^{b}R^{c}, -NR^{b}C(O)R^{d}, C₁₋₆ alkyl, hydroxy C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy and C₁₋₆ alkoxy C₁₋₆ alkoxy, or, two R⁴, together with the atoms attached thereto, can form 5-6 membered oxygen-containing cyclic group;
L is selected from -NRb-, -O- and -S-;
R^{a} is present or absent, and when present, R^{a} is each independently selected from hydrogen, C₁₋₆ alkyl and halogenated C₁₋₆ alkyl;
R^{b}, R^{c} and R^{d} are each present or absent, and when present, R^{b}, R^{c} and R^{d} are each independently selected from hydrogen and C₁₋₆ alkyl;
n is an integer of 0 to 2; q is an integer of 0 to 2;
t¹, t², t³ and t⁴ are each independently selected from integers of 0 to 5; and
p¹ and p² are each independently selected from integers of 0 to 2.

In one embodiment of the present invention, a compound of formula (I) or a pharmaceutically acceptable salt, ester, stereoisomer or tautomer thereof is provided, having a structure of general formula (VI), 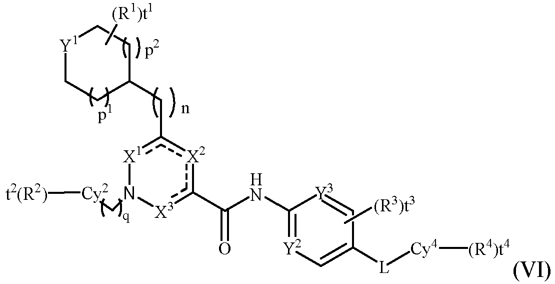 wherein, each group is defined as the same as that in the formula (V).

Preferably, in the formula (V), X¹ is N, X² is CR^{a}, and X³ is C=O.

Preferably, in the formula (V), X¹ is CR^{a}, X² is N, and X³ is C=O.

Preferably, in the formula (V), X¹ is C=O, X² is CR^{a}, and X³ is C=O.

Preferably, in the formula (V), X¹ is CR^{a}, X² is CR^{a}, and X³ is C=O.

Preferably, in the formula (VI), X¹ is N, X² is CR^{a}, and X³ is C=O.

Preferably, in the formula (VI), X¹ is CR^{a}, X² is N, and X³ is C=O.

Preferably, in the formula (VI), X¹ is C=O, X² is CR^{a}, and X³ is C=O.

Preferably, in the formula (VI), X¹ is CR^{a}, X² is CR^{a}, and X³ is C=O.

In one embodiment of the present invention, when the ring in formula (b) carries two carbonyls, Cy² represents 6-14 membered aryl optionally substituted with one or more R², Cy¹ represents 3-12 membered heterocyclyl that is optionally substituted with one or more R¹ and contains 1-3 heteroatoms selected from O, S, S(O) and S(O)₂, and Cy³-L does not form aryloxy.

In one embodiment of the present invention, when, in group shown as formula (b), at least one of X¹ and X² represents NR^{b}, Cy¹ represents 3-12 membered heterocyclyl that is optionally substituted with one or more R¹ and contains 1-3 heteroatoms selected from O, S, S(O) and S(O)₂.

In one embodiment of the present invention, when, in group shown as formula (c), at least one of X¹ and X³ represents NR^{b}, Cy¹ represents 3-12 membered heterocyclyl that is optionally substituted with one or more R¹ and contains 1-3 heteroatoms selected from O, S, S(O) and S(O)₂.

In one embodiment of the present invention, when, in group shown as formula (b), X¹ and X² are CH, and X³ is C=O, Cy¹ represents 3-12 membered heterocyclyl that is optionally substituted with one or more R¹ and contains 1-3 heteroatoms selected from O, S, S(O) and S(O)₂, Cy² represents 6-14 membered aryl optionally substituted with one or more R², and L does not represent heteroaryl.

In one embodiment of the present invention, when, in group shown as formula (c), X¹ and X² are CH, and X³ is C=O, Cy¹ represents 3-12 membered heterocyclyl that is optionally substituted with one or more R¹ and contains 1-3 heteroatoms selected from O, S, S(O) and S(O)₂, and Cy² represents 6-14 membered aryl optionally substituted with one or more R².

In one embodiment of the present invention, the case where two carbonyls are directly bonded in the compound structure of the present invention is impossible.

In one embodiment of the present invention, ring A is selected from phenyl, 5-6 membered heteroaryl and 5-6 membered heterocyclyl.

In one embodiment of the present invention, Q is selected from the group consisting of hydrogen, cyano, hydroxyl, halogen, carboxyl, nitro, -NR^{b}R^{c}, -C(O)R^{d}, -C(O)NR^{b}R^{c}, - OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl, and q is an integer of 0 to 4.

In one embodiment of the present invention, Cy¹ is selected from 3-12 membered heterocyclyl optionally substituted with one or more R¹, and R¹ is selected from the group consisting of hydrogen, cyano, hydroxyl, halogen, carboxyl, nitro, -NR^{b}R^{c}, -C(O)R^{d}, - C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, C_{1- 6} alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl.

In one embodiment of the present invention, Cy² is selected from 3-12 membered cycloalkyl, 3-12 membered cycloalkenyl, 3-12 membered heterocyclyl, 6-14 membered aryl and 5-10 membered heteroaryl that are optionally substituted with one or more R², and R² is selected from the group consisting of hydrogen, cyano, hydroxyl, halogen, carboxyl, nitro, - NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, - SO₂R^{d}, -NR^{b}SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', - O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl.

In one embodiment of the present invention, Cy³ is selected from 3-12 membered cycloalkyl, 3-14 membered heterocyclyl, 5-10 membered heteroaryl and 6-14 membered aryl that are optionally substituted with one or more R³, and R³ is selected from the group consisting of hydrogen, cyano, hydroxyl, halogen, carboxyl, nitro, -NR^{b}R^{c}, -C(O)R^{d}, -C(O)NR^{b}R^{c}, - OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl.

In one embodiment of the present invention, Cy⁴ is selected from 3-14 membered heterocyclyl and 5-14 membered heteroaryl that are optionally substituted with one or more R⁴, and R⁴ is selected from the group consisting of hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl, or, two R⁴, together with the atoms attached thereto, can form 5-6 membered cyclic group.

In one embodiment of the present invention, R^{a} is present or absent, and when present, R^{a} is independently selected from the group consisting of hydrogen, cyano, hydroxyl, halogen, carboxyl, nitro, -NR^{b}R^{c}, -C(O)R^{d}, -C(O)NR^{b}R^{c}, -OC(O)NR^{b}R^{c}, -NR^{b}C(O)OR^{d}, -NR^{b}C(O)R^{d}, - SO₂-NR^{b}R^{c}, -SO₂R^{d}, -NR^{b}SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, C₂₋₈ alkenyl, C₂₋₈ alkynyl, -C₁₋₆ alkyl-R', -C₁₋₆ alkoxy-R', -O-R', -C(O)-R', -SO₂-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl.

In one embodiment of the present invention, R^{b} and R^{c} are present or absent, and when present, R^{b} and R^{c} are each independently selected from the group consisting of hydrogen, hydroxyl, -C(O)R^{d}, -C(O)NR^{b}R^{c}, -SO₂-NR^{b}R^{c}, -SO₂R^{d}, C₁₋₆ alkyl, hydroxy C₁₋₆ alkyl, cyano C₁₋₆ alkyl, amino C₁₋₆ alkyl, C₁₋₆ alkylamino C₁₋₆ alkyl, halogenated C₁₋₆ alkyl, C₁₋₆ alkoxy C₁₋₆ alkyl, -C₁₋₆ alkyl-R', -C(O)-R', -SO₂-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl or 5-10 membered heteroaryl.

In one embodiment of the present invention, R^{d} is present or absent, and when present, R^{d} is independently selected from the group consisting of hydrogen, -NR^{b}R^{c}, -NR^{b}C(O)OR^{d}, - NR^{b}C(O)R^{d}, -NR^{b}SO₂R^{d}, C₁₋₆ alkyl, hydroxy C₁₋₆ alkyl, cyano C₁₋₆ alkyl, amino C₁₋₆ alkyl, C_{1- 6} alkylamino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkoxy C₁₋₆ alkoxy, -C₁₋₆ alkyl-R', -C₁₋₆ alkoxy-R', -O-R', -NR^{b}C(O)-R', 3-12 membered cycloalkyl, 3-12 membered cycloalkenyl, 3-12 membered heterocyclyl, 6-14 membered aryl and 5-10 membered heteroaryl, and R' is 3-12 membered cycloalkyl, 3-12 membered cycloalkenyl, 3-12 membered heterocyclyl, 6-14 membered aryl and 5-10 membered heteroaryl.

Some compounds of the present invention are exemplarily listed below, but the compounds of the present invention are not limited thereto.

| No. | Structure | No. | Structure |
|---|---|---|---|
| 1 | 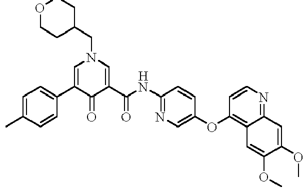 | 2 | 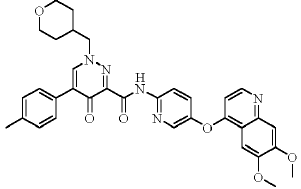 |
| 3 | 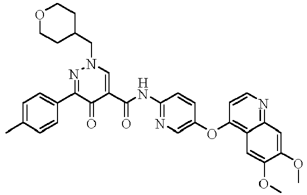 | 4 | 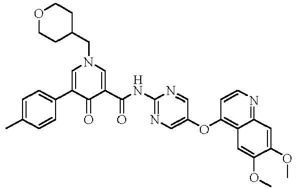 |
| 5 | 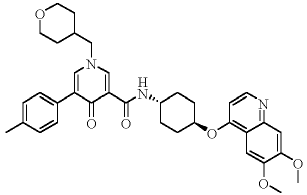 | 6 | 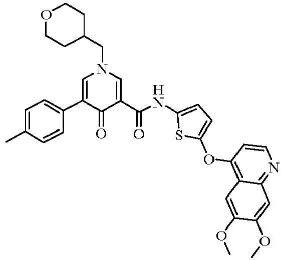 |
| 7 | 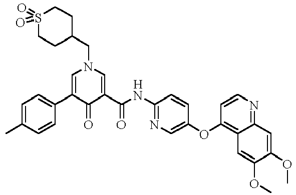 | 8 | 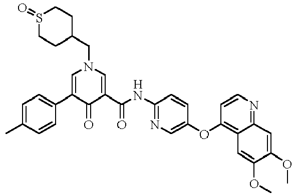 |
| 9 | 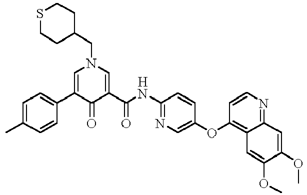 | 10 | 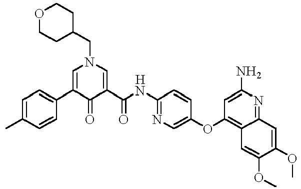 |
| 11 | 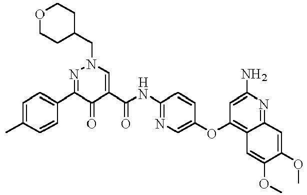 | 12 | 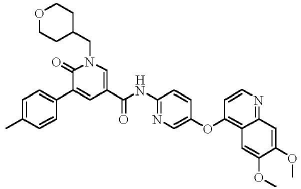 |
| 13 | 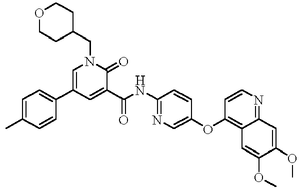 | 14 | 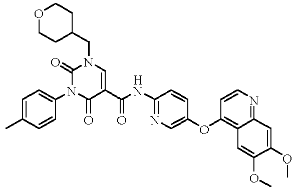 |
| 15 | 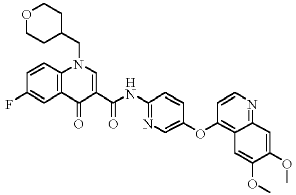 | 16 | 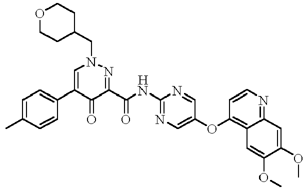 |
| 17 | 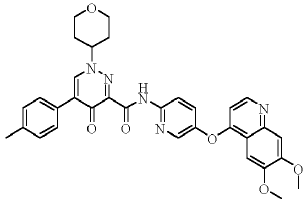 | 18 | 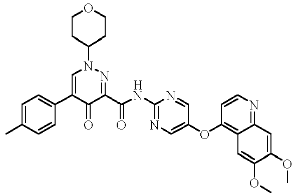 |
| 19 | 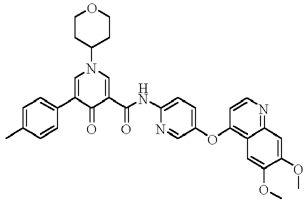 | 20 | 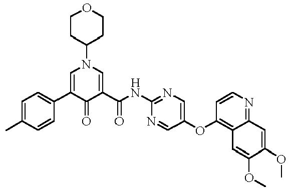 |
| 21 | 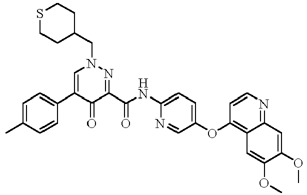 | 22 | 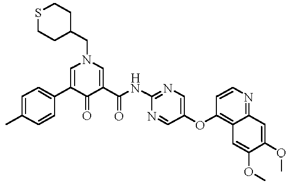 |
| 23 | 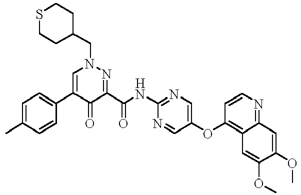 | 24 | 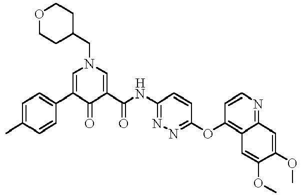 |
| 25 | 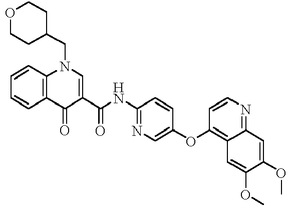 | 26 | 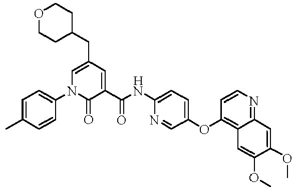 |
| 27 | 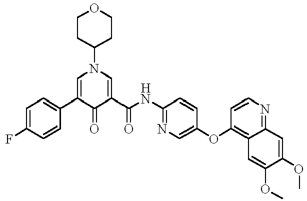 | 28 | 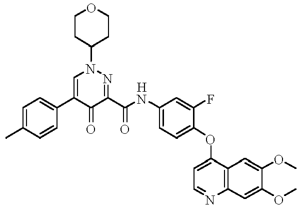 |
| 29 | 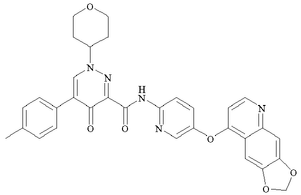 | 30 | 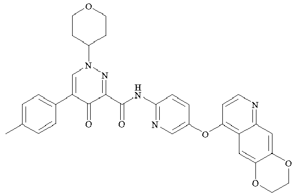 |
| 31 | 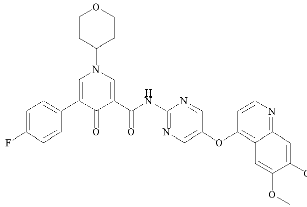 | 32 | 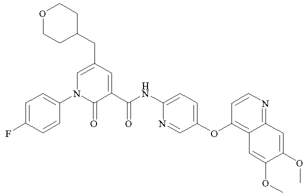 |
| 33 | 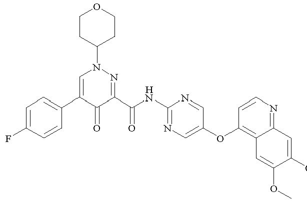 | 34 | 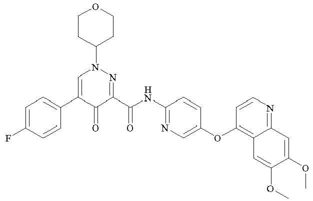 |
| 35 | 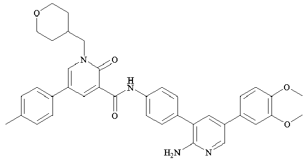 | 36 | 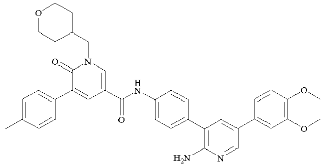 |
| 37 | 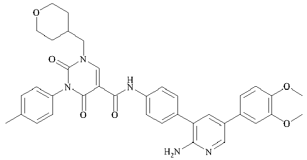 | 38 | 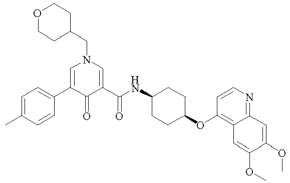 |
| 39 | 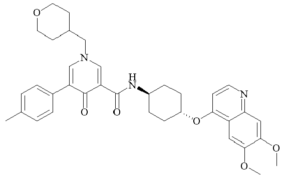 | 40 | 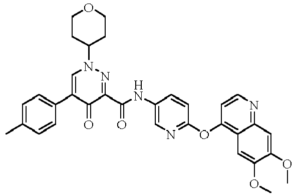 |
| 41 | 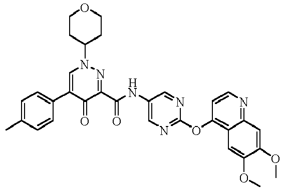 | 42 | 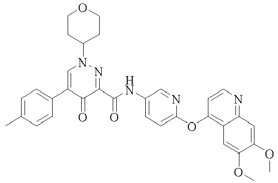 |
| 43 | 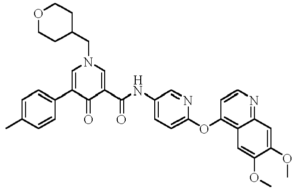 | 44 | 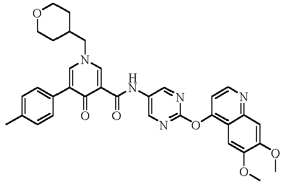 |
| 45 | 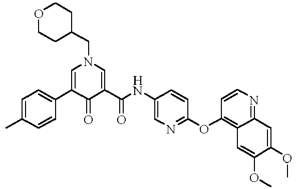 | 46 | 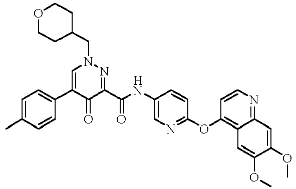 |
| 47 | 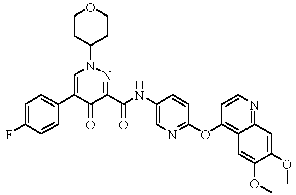 | 48 | 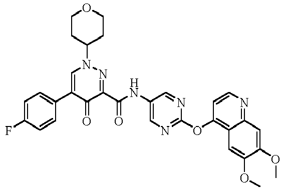 |
| 49 | 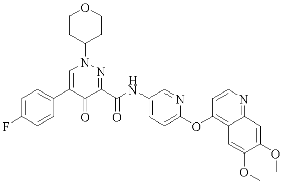 | | |

The present invention also provides a pharmaceutical composition, including at least one of a compound of any one of formula (I), (II), (III), (IV), (V) and (VI), and a pharmaceutically acceptable salt, ester, stereoisomer or tautomer thereof.

The present invention also provides a pharmaceutical composition including a compound of any one of formula (I), (II), (III), (IV), (V) and (VI), or a pharmaceutically acceptable salt, ester, stereoisomer or tautomer thereof, and the pharmaceutical composition may optionally include one or more pharmaceutical carriers.

The present invention also provides a pharmaceutically acceptable formulation including a compound of any one of formula (I), (II), (III), (IV), (V) and (VI), or a pharmaceutically acceptable salt, ester, stereoisomer or tautomer thereof, and the pharmaceutically acceptable formulation optionally includes one or more pharmaceutical carriers.

In one embodiment of the present invention, the aforementioned pharmaceutical composition or formulation may further include one or more second therapeutically active agents.

In one embodiment of the present invention, it provides a pharmaceutical composition including a compound of any one of formula (I), (II), (III), (IV), (V) and (VI), or a pharmaceutically acceptable salt, ester, stereoisomer or tautomer thereof, and the pharmaceutical composition may optionally include at least one second therapeutically active agent.

In one embodiment of the present invention, the second therapeutically active agent refers to at least one selected from the group consisting of antimetabolite, growth factor inhibitor, mitotic inhibitor, anti-tumor hormone, alkylating agent, metal formulation, topoisomerase inhibitor, hormone drug, immunomodulator, tumor suppressor gene, cancer vaccine, immune checkpoint- or tumor immunotherapy-related antibody, small molecule drug and cytotherapeutic agent.

In one embodiment of the present invention, the pharmaceutical composition or formulation can be administered to a patient or subject in need of prophylaxis and/or treatment in any suitable manner well known in the art, for example, oral, parenteral (including subcutaneous, intramuscular, intravenous, intra-arterial, intradermal, intrathecal, and epidural), transdermal, rectal, nasal, transpulmonary, topical (including buccal and sublingual), vaginal, intraperitoneal, intrapulmonary and intranasal administration.

In one embodiment of the present invention, the pharmaceutical composition or formulation can be formulated into a conventional solid formulation, such as tablet, capsule, pill and granule, and can also be formulated into an oral liquid formulation, such as oral solution, oral suspension and syrup. In the preparation of an oral formulation, an appropriate filler, binder, disintegrant, lubricant and the like may be added. For parenteral administration, the pharmaceutical composition can be formulated as an injection, a sterile powder for injection and a concentrated solution for injection. The injection can be made by a conventional method existing in the pharmaceutical field, and during the preparation process, no additive may be added, or an appropriate additive may be added according to the property of the medicine. For rectal administration, the pharmaceutical composition can be made into a suppository and the like. For transpulmonary administration, the pharmaceutical composition can be made into an inhalant, spray or the like.

In one embodiment of the present invention, it provides use of the compound of any one of formula (I), (II), (III), (IV), (V) and (VI) or a pharmaceutically acceptable salt, ester, stereoisomer or tautomer thereof, the pharmaceutical composition, or the formulation described above in the preparation of a medicament for treating and/or preventing diseases mediated by the abnormal expression of TAM family kinase receptors and/or ligands thereof, and the diseases mediated by the abnormal expression of TAM family kinase receptors and/or ligands thereof include at least one of tumor, immune dysregulation, endometriosis, vascular disease/trauma, psoriasis, visual defect/lesion (caused by macular degeneration, diabetes, premature delivery, *etc.),* kidney disease, rheumatoid arthritis, osteoporosis and related diseases.

In one embodiment of the present invention, the compound of any one of formula (I), (II), (III), (IV), (V) and (VI) or a pharmaceutically acceptable salt, ester, stereoisomer or tautomer thereof, the pharmaceutical composition or the formulation described above is used for treating and/or preventing diseases mediated by the abnormal expression of TAM family kinase receptors and/or ligands thereof, and the diseases mediated by the abnormal expression of TAM family kinase receptors and/or ligands thereof include at least one of tumor, immune dysregulation, endometriosis, vascular disease/trauma, psoriasis, visual defect/lesion (caused by macular degeneration, diabetes, premature delivery, *etc.),* kidney disease, rheumatoid arthritis, osteoporosis and related diseases.

In one embodiment of the present invention, it provides use of the compound of any one of formula (I), (II), (III), (IV), (V) and (VI) or a pharmaceutically acceptable salt, ester, stereoisomer or tautomer thereof, the pharmaceutical composition, or the formulation described above in the formulation of a medicament for treating and/or preventing diseases mediated by the abnormal expression of TAM family kinase and/or CSF1R kinase receptors and/or ligands thereof, and the diseases mediated by the abnormal expression of TAM family kinase and/or CSF1R kinase receptors and/or ligands thereof include at least one of tumor, immune dysregulation, endometriosis, vascular disease/trauma, psoriasis, visual defect/lesion (caused by macular degeneration, diabetes, premature delivery, *etc.),* kidney disease, rheumatoid arthritis, osteoporosis and related diseases.

In one embodiment of the present invention, the compound of any one of formula (I), (II), (III), (IV), (V) and (VI) or a pharmaceutically acceptable salt, ester, stereoisomer or tautomer thereof, the pharmaceutical composition or the formulation described above is used for treating and/or preventing diseases mediated by the abnormal expression of TAM family kinase and/or CSF1R kinase receptors and/or ligands thereof, and the diseases mediated by the abnormal expression of TAM family kinase and/or CSF1R kinase receptors and/or ligands thereof include at least one of tumor, immune dysregulation, endometriosis, vascular disease/trauma, psoriasis, visual defect/lesion (caused by macular degeneration, diabetes, premature delivery, *etc.),* kidney disease, rheumatoid arthritis, osteoporosis and related diseases.

In one embodiment of the present invention, the tumor includes sarcoma, lymphoma and cancer, and may specifically be lung cancer, squamous cell carcinoma, bladder cancer, gastric cancer, ovarian cancer, peritoneal cancer, breast cancer, breast ductal carcinoma, head and neck cancer, endometrial cancer, corpus carcinoma, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma, melanoma, cytoma and sarcoma.

### EFFECT OF THE INVENTION

The compound of the present invention has inhibitory effect on TAM family kinases. In addition, the compound of the present invention can also target CSF1R kinase and exhibits inhibitory effect on CSF1R kinase. The compound of the present invention can be used to treat and/or prevent diseases mediated by abnormal expression of TAM family kinase receptors and/or ligands thereof. In addition, the compound of the present invention can be used to treat and/or prevent diseases mediated by abnormal expression of TAM family kinase and/or CSF1R kinase receptors and/or ligands thereof. The compound of the present invention reverses the immunosuppression in the tumor microenvironment and inhibits the growth, migration and/or drug resistance of the tumor by simultaneously inhibiting TAM family kinase and CSF1R kinase, thereby exerting the tumor immunology effect and anti-tumor efficacy.

In addition, with a long half-life and excellent metabolic stability in the body, the compound of the present invention can improve the medicament efficacy, reduce the medication burden on a patient, and improve the compliance of a patient.

### DETAILED DESCRIPTION OF THE INVENTION

The embodiments of the present invention will be described in more detail in conjunction with specific implementations below, but those skilled in the art will understand that the specific implementations described below are only used to illustrate the present invention and should not be regarded as limiting the protection scope of the present invention. On the contrary, the present invention is intended to encompass all alternatives, modifications and equivalents that can be included within the scope of the invention as defined by the claims. Unless otherwise specified, the various embodiments of the present invention can be combined in any manner, and the conversions, modifications, and changes of the technical solutions thus obtained are also included in the scope of the present invention, and do not exceed the scope of the present invention.

In the context of the present invention, unless clearly defined otherwise, or the meaning is beyond the understanding of those skilled in the art, a hydrocarbon or hydrocarbon derivative group with 3 or more carbon atoms (such as propyl, propoxy, butyl, butane, butene, butenyl and hexane) is deemed as normal either with or without the prefix of "normal". For example, propyl is generally considered as *n*-propyl, and butyl is generally considered as *n*-butyl, unless otherwise specified.

All publications, patent applications, patents, and other references mentioned in this specification are hereby incorporated by reference. Unless otherwise defined, all technical and scientific terms used in this specification have the meanings conventionally understood by those skilled in the art. In case of conflict, the definition in this specification shall control.

In the context of this specification, in addition to the contents clearly stated, any matter or item not mentioned is directly applicable to those known in the art without any change. Moreover, any embodiment described herein can be freely combined with one or more other embodiments described herein, and the resulting technical solutions or technical ideas are regarded as part of the original disclosure or original record of the present invention, and should not be regarded as new contents that have not been disclosed or anticipated herein, unless those skilled in the art believe that the combination is obviously unreasonable.

In the present invention, the expression of "C_{a-b} group" (a and b represent an integer ≥ 1, and a < b) means that the "group" has a-b carbon atoms, for example, C₁₋₄ alkyl represents alkyl with 1-4 carbon atoms, C₁₋₄ alkoxy represents alkoxy with 1-4 carbon atoms, C₃₋₁₀ cycloalkyl represents cycloalkyl with 3-10 carbon atoms, and C₁₋₄ alkoxy C₁₋₄ alkyl represents a group formed by bonding alkoxy having 1-4 carbon atoms with alkyl having 1-4 carbon atoms.

In the present invention, "group" represents a monovalent group or a divalent or higher group that conforms to the valence as required, for example, "cycloalkyl (also referred to as cycloalkyl group)" includes a monovalent group obtained by removing one hydrogen atom therefrom, and also includes a divalent or higher group obtained by removing one or more hydrogen atoms from the same carbon atom or from two or more different carbon atoms. As a terminal group, "cycloalkyl" is monovalent; and as a linking group in the structure, "cycloalkyl" is divalent or higher. A person skilled in the art can definitely determine the valence of a "group".

The "halogen" in the present invention refers to fluorine, chlorine, bromine and iodine. It is preferably fluorine, chlorine and bromine.

The "halogenated" in the present invention means that one or more hydrogens on any carbon atom in the substituent may be substituted with one or more halogens that are the same or different. "Halogen" is as defined above.

"C₁₋₆ alkyl" in the present invention refers to linear or branched alkyl obtained by removing one or more hydrogen atoms from alkane containing 1-6 carbon atoms, for example, methyl, ethyl, *n*-propyl, isopropyl, *n*-butyl, isobutyl, sec-butyl, *tert-butyl, n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl and 1-methyl-2-methylpropyl. "C₁₋₄ alkyl" refers to the above instance containing 1 to 4 carbon atoms.

"C₁₋₆ alkylamino", "(C₁₋₆ alkyl)₂ amino", "C₁₋₆ alkylester", "C₁₋₆ alkylamino carbonyl", "C_{1- 6} alkyl carbonyl", "C₁₋₆ alkylcarbonyloxy", "C₁₋₆ alkylsulfonylamino", "C₁₋₆ alkylsulfonyl", "C_{1- 6} alkylthio" and the like that contain "C₁₋₆ alkyl" described in the present invention refer to groups obtained by linking C₁₋₆ alkyl to corresponding groups, such as -NH₂, -CO-NH₂-, -COO-, -CO-, -SO₂NH₂-, -SO₂- and -S-. For example, it may include groups obtained by linking each of "C₁₋₆ alkyl" groups listed above to corresponding groups, such as -NH₂, -CO-NH₂-, - CO-O-, -CO-, -SO₂NH₂-, -SO₂- and -S-.

"C₂₋₈ alkenyl" mentioned in the present invention refers to linear or branched alkenyl obtained by removing one or more hydrogen atoms from alkene that contains 2 to 8 carbon atoms and at least one carbon-carbon double bond, for example, vinyl, 1-propenyl, 2-propenyl, 1-butenyl, 2-butenyl, 1,3-butadien-1-yl, 1-penten-3-yl, 2-penten-1-yl, 3-penten-1-yl, 3-penten-2-yl, 1,3-pentadien-1-yl, 1,4-pentadien-3-yl, 1-hexen-3-yl and 1,4-hexadien-1-yl. Preferably, "C₂₋₈ alkenyl" contains a carbon-carbon double bond.

"C₂₋₈ alkynyl" mentioned in the present invention refers to linear or branched alkynyl obtained by removing one or more hydrogen atoms from alkyne that contains 2 to 8 carbon atoms and at least one carbon-carbon triple bond, for example, ethynyl, propynyl, 2-butyn-1-yl, 2-pentyn-1-yl, 3-pentyn-1-yl, 4-methyl-2-pentyn-1-yl, 2-hexyn-1-yl, 2-hexyn-2-yl, 3-hexyn-1-yl and 3-hexyn-2-yl. Preferably, "C₂₋₈ alkynyl" contains a carbon-carbon triple bond.

"C₁₋₆ alkoxy" in the present invention refers to the group obtained by linking an oxygen atom to the parent structure of "C₁₋₆ alkyl" defined above, *i.e.,* "C₁₋₆ alkyl-O-" group, for example, methoxy, ethoxy, *n*-propoxy, isopropoxy, *n*-butoxy, tert-butoxy, *n*-pentyloxy, neopentyloxy and *n*-hexyloxy. "C₁₋₄ alkoxy" refers to the above instance containing 1-4 carbon atoms, *i.e.,* "C₁₋₄ alkyl-O-" group.

"Halogenated C₁₋₆ alkoxy", "C₁₋₆ alkoxy C₁₋₆ alkoxy" and "C₁₋₆ alkyl C₁₋₆ alkoxy" that contain "C₁₋₆ alkoxy" described in the present invention refer to groups obtained by substituting at least one hydrogen atom on C₁₋₆ alkoxy independently with at least one halogen, C₁₋₆ alkoxy and C₁₋₆ alkyl.

The "polycyclic ring" in the present invention refers to a polycyclic system structure formed by two or more cyclic structures linked by fusing, spiro or bridging. The "ortho-fused ring" refers to a polycyclic ring structure formed by two or more ring structures sharing two adjacent ring atoms (*i.e.,* sharing a bond) with each other. The "bridged ring" refers to a polycyclic ring structure formed by two or more ring structures sharing two non-adjacent ring atoms with each other. The "spiro ring" refers to a polycyclic ring structure formed by two or more ring structures sharing a ring atom with each other.

The "cycloalkyl" or "cycloalkyl group" (hereinafter, collectively referred to as "cycloalkyl") in the present invention refers to a monovalent, divalent or higher (as required) group derived from cycloalkane. The cycloalkane includes monocyclic cycloalkane or polycyclic cycloalkane, and may have 3, 4, 5, 6, 7, 8, 9, 10, 11 or 12 carbon atoms. Unless otherwise specified, a certain membered cycloalkyl encompasses all possible monocyclic or polycyclic cycloalkyls (including ortho-fused, spiro and bridged). Cycloalkyl may be a 3-12 membered monovalent, divalent or higher (as required) group, a 3-10 membered monovalent, divalent or higher (as required) group, a 3-8 membered monovalent, divalent or higher (as required) group, a 3-6 membered monovalent, divalent or higher (as required) group, or a 4-6 membered monovalent, divalent or higher (as required) group.

(Monovalent, divalent or higher) monocyclic cycloalkyl may be 3-12 membered cycloalkyl, 3-10 membered cycloalkyl, 3-8 membered cycloalkyl, 3-6 membered cycloalkyl or 4-6-membered cycloalkyl. Examples include, but are not limited to: cyclopropyl, cyclobutyl, cyclopentyl, cyclohexyl, cycloheptyl, cyclooctyl, cyclopentyl-1, 3-diyl, cyclohexyl-1, 4-diyl, cycloheptyl-1, 4-diyl, *etc.*

(Monovalent, divalent or higher) polycyclic cycloalkyl includes ortho-fused cycloalkyl, bridged cycloalkyl and spiro cycloalkyl.

(Monovalent, divalent or higher) ortho-fused cycloalkyl may be 6-11 membered ortho-fused cycloalkyl or 7-10 membered ortho-fused cycloalkyl, and representative examples include, but are not limited to, monovalent, divalent or higher groups derived from bicyclo[3.1.1]heptane, bicyclo[2.2.1]heptane, bicycle
[2.2.2]octane, bicyclo[3.2.2]nonane, bicyclo[3.3.1]nonane and bicyclo[4.2.1] nonane.

The "cycloalkenyl" in the present invention refers to a group obtained by forming at least one double bond in the above cycloalkyl, and may have 3, 4, 5, 6, 7, 8, 9, 10, 11 or 12 ring carbon atoms. Unless otherwise specified, a certain membered cycloalkenyl encompasses all possible monocyclic or polycyclic cycloalkenyls (including ortho-fused, spiro and bridged). Cycloalkenyl may be 3-12 membered cycloalkenyl, 3-8 membered cycloalkenyl, 4-6 membered cycloalkenyl, 7-11 membered spiro cycloalkenyl, 7-11 membered ortho-fused cycloalkenyl, 6-11 membered bridged cycloalkenyl, *etc.* Examples of cycloalkenyl include, but are not limited to, cyclobutenyl, cyclopentenyl, cyclohexenyl, 1,4-cyclohexadien-1-yl, cycloheptenyl, 1,4-cycloheptadien-1-yl, cyclooctenyl and 1,5-cyclooctadien-1-yl.

"Cycloalkyl" and "cycloalkenyl" may also be monovalent groups obtained by removing one hydrogen atom from 7-12 membered spiro ring or 7-11 membered spiro ring, or divalent or higher groups obtained by removing two or more hydrogen atoms from the same carbon atom or from different carbon atoms. Examples of spiro ring include, but are not limited to:

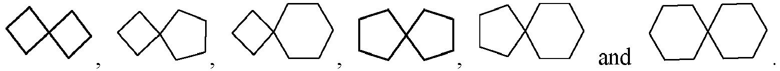

"Cycloalkyl" and "cycloalkenyl" may also be monovalent groups obtained by removing one hydrogen atom from 6-12 membered bridged ring or 7-11 membered bridged ring, or divalent or higher groups obtained by removing two or more hydrogen atoms from the same carbon atom or from different carbon atoms. Examples of bridged ring include, but are not limited to:

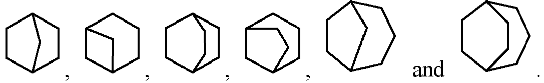

The "heterocyclic ring" in the present invention includes non-aromatic cyclic hydrocarbon containing at least one (may be 1-5, 1-4, 1-3, 1-2 or 1) heteroatom selected from O, S and N as a ring atom in the ring. It may be a heterocyclic ring having 3, 4, 5, 6, 7, 8, 9, 10, 11, 12, 13 or 14 ring atoms. There may optionally be at least one double bond in the ring. The heterocyclic ring of the present invention may be a monocyclic or polycyclic system (ortho-fused, spiro or bridged). Examples of heterocyclic ring may be pyrroline, pyrrolidine, piperidine, piperazine, morpholine, thiomorpholine, tetrahydropyran, dihydropyridine, dihydropyridazine, dioxane, oxathiolane, thiacyclopentane, tetrahydrofuran, tetrahydropyran, tetrahydrothiazole, tetrahydroisothiazole and other monoheterocyclic rings; and indoline, isoindoline, benzopyran, benzodioxane, tetrahydroquinoline, benzo[*d*]oxazol-2(3*H*)-one, tetrahydrobenzothiophene and other polyheterocyclic rings. Furthermore, it may be a heterocyclic ring obtained by replacing at least one ring carbon atom in the 7-12 membered spiro ring, 7-11 membered spiro ring, 6-12 membered bridged ring or 7-11 membered bridged ring with a heteroatom selected from O, S and N. Furthermore, it may be the 6-12 membered spiro heterocyclic ring, 7-11 membered spiro heterocyclic ring, 6-12 membered saturated spiro ring, 7 membered saturated spiro heterocyclic ring, 6-12 membered bridged heterocyclic ring, 7-11 membered bridged heterocyclic ring, 6-12 membered saturated bridged ring, and 7-8 membered saturated bridged ring.

The "heterocyclyl" or "heterocyclyl group" (hereinafter, collectively referred to as "heterocyclyl") in the present invention refers to a monovalent, divalent or higher group derived from the "heterocyclic ring". In addition, the "heterocyclyl" described in the present invention may also be a monovalent, divalent or higher non-aromatic cyclic group obtained by substituting at least one ring carbon atom in the cycloalkyl or cycloalkenyl with at least one heteroatom selected from O, S, S(O), S(O)₂ and N, and preferably by substitution with 1-4 heteroatoms. More specifically, "heterocyclyl" may be a group having 3, 4, 5, 6, 7, 8, 9, 10, 11, 12, 13 or 14 ring atoms. It may be 3-14 membered heterocyclyl, 3-12 membered heterocyclyl, 3-10 membered heterocyclyl, 4-10 membered heterocyclyl, 3-8 membered heterocyclyl, 4-8 membered heterocyclyl, or 4-6 membered heterocyclyl.

In addition, "heterocyclyl" includes a monovalent, divalent or higher (as required) monocyclic heterocyclyl system, or a monovalent, divalent or higher (as required) polycyclic heterocyclyl system (also referred to as polycyclic system), including saturated or partially saturated heterocyclyl, but excluding aromatic cyclyl. Unless otherwise specified, it may encompass all possible monocyclic, polycyclic (including ortho-fused, spiro and bridged), saturated or partially saturated heterocyclyls.

The monovalent, divalent or higher (as required) monocyclic heterocyclyl may be 3-14 membered heterocyclyl, 3-12 membered heterocyclyl, 3-10 membered heterocyclyl, 4-10 membered heterocyclyl, 3-8 membered heterocyclyl, 4-8 membered heterocyclyl, or 4-6 membered heterocyclyl. Furthermore, it may also be 3-14 membered oxygen-containing heterocyclyl, 3-14 membered nitrogen-containing heterocyclyl, 3-12 membered oxygen-containing heterocyclyl, 3-12 membered sulfur-containing heterocyclyl, 3-12 membered sulfonyl-containing (S(O)₂) heterocyclyl, 3-12 membered sulfinyl-containing (S(O)) heterocyclyl, *etc.* Examples of "heterocyclyl" include, but are not limited to, aza-cyclopropyl, oza-cyclopropyl, thiocyclopropyl, aza- cyclobutyl, oxa-cyclobutyl, thiocyclobutyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1, 3-thiazolidinyl, tetrahydro-2*H*-pyranyl, tetrahydro-2*H*- thiopyranyl, piperidyl, piperazinyl, morpholinyl, 1,4-dioxanyl, 1,4-oxathianyl, 4,5-dihydroisoxazolyl, 4,5-dihydrooxazolyl, 2,5-dihydrooxazolyl, 2,3- dihydrooxazolyl, 3,4-dihydro-2*H*-pyrrolyl, 2,3-dihydro-1*H*-pyrrolyl, 2,5-dihydro- 1*H*-imidazolyl, 4,5-dihydro-1*H*-imidazolyl, 4,5-dihydro-1*H-*pyrazolyl, 4,5- dihydro-3*H*-pyrazolyl, 4,5-dihydrothiazolyl, 2,5-dihydrothiazolyl, 2*H*-pyranyl, 4*H*-pyranyl, 2*H*-thiopyranyl, 4*H*-thiopyranyl, 2,3,4,5-tetrahydropyridyl, 1,2- isoxazinyl, 1,4-isoxazinyl, 6*H*-1,3-oxazinyl or the like.

The monovalent, divalent or higher (as required) polycyclic heterocyclyl includes ortho-fused heterocyclyl, spiro heterocyclyl and bridged heterocyclyl, which may be saturated, partially saturated or unsaturated, but are not aromatic. Polycyclic heterocyclyl may be heterocyclyl obtained by linking above heterocyclyl to 6-14 membered aryl, 3-12 membered cycloalkyl, 3-12 membered cycloalkenyl, 3-14 membered heterocyclyl or 3-14 membered heteroaryl.

The ortho-fused heterocyclyl may be 6-12 membered ortho-fused cyclyl, 7-10 membered ortho-fused cyclyl, 6-10 membered ortho-fused cyclyl or 6-12 membered saturated ortho-fused cyclyl, and representative examples include, but are not limited to: 3-azabicyclo[3.1.0]hexyl, 3,6-diazabicyclo[3.2.0]heptyl, 3,8-diazabicyclo[4.2.0]octyl, 3,7-dizabicyclo[4.2.0]octyl, octahydropyrrolo [3,4-c] pyrrolyl, octahydropyrrolo[3,4-*b*]pyrrolyl, octahydropyrrolo[3,4-*b*][1,4] oxazinyl, octahydro-1*H*-pyrrolo[3,4-*c*]pyridyl, 2,3-dihydrobenzofuran-2-yl, 2,3- dihydrobenzofuran - 3-yl, indolin-1-yl, indolin-2-yl, indolin-3-yl, 2,3- dihydrobenzothiophen-2-yl, octahydro-1*H*-indolyl and octahydrobenzofuranyl.

The spiro heterocyclyl may be a monovalent group obtained by removing one hydrogen atom from 6-12 membered spiro heterocyclic ring, 7-11 membered spiro heterocyclic ring, 6-12 membered saturated spiro ring or 7 membered saturated spiro heterocyclic ring, or a divalent or higher group obtained by removing two or more hydrogen atoms from the same carbon atom or from different carbon atoms as required. Examples of spiro heterocyclyl include, but are not limited to:

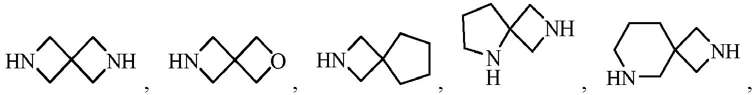

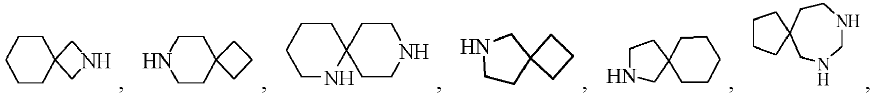

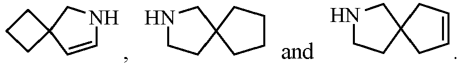

The bridged heterocyclyl may be a monovalent group obtained by removing one hydrogen atom from 6-12 membered bridged heterocyclic ring, 7-11 membered bridged heterocyclic ring, 6-12 membered saturated bridged ring or 7-8 membered saturated bridged ring, or a divalent or higher group obtained by removing two or more hydrogen atoms from the same carbon atom or from different carbon atoms as required. Examples of bridged heterocyclyl include, but are not limited to:

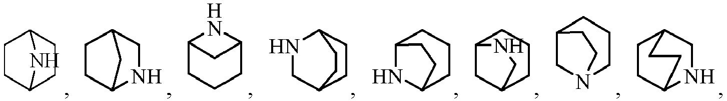

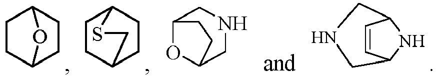

The "aromatic ring" in the present invention refers to a carbocyclic hydrocarbon having aromaticity. It may be 6-14 membered aromatic ring or 6-10 membered aromatic ring. Specifically, 6 membered aromatic ring is benzene ring, 10 membered aromatic ring is naphthalene ring, and 14 membered aromatic ring is anthracene or phenanthrene ring.

The "aryl" or "aromatic group" (hereinafter, collectively referred to as "aryl") in the present invention refers to a monovalent, divalent or higher (as required) group derived from an aromatic carbocyclic hydrocarbon. It includes 6-14 membered aryl and 6-10 membered aryl. 6-14 membered aryl is, for example, phenyl, naphthyl, phenanthrenyl or anthracenyl. 6-10 membered aryl is, for example, phenyl or naphthyl. Divalent aryl may include, for example, phenylene, naphthylene and the like.

The "heteroaromatic ring" in the present invention refers to an aromatic cyclic hydrocarbon containing at least one heteroatom selected from O, S and N (may be 1-5, 1-4, 1-3, 1-2 or 1) as a ring atom in the ring. The heteroaromatic ring may be 5, 6, 7, 8, 9, 10, 11, 12, 13 or 14 membered heteroaromatic ring. In addition, the heteroaromatic ring of the present invention may be a monocyclic or polycyclic system (ortho-fused, spiro or bridged). Specifically, examples may include pyrrole, pyrazine, pyrazole, indole, tetrazole, furan, thiophene, pyridine, imidazole, triazole, tetrazole, triazine, pyridazine, pyrimidine, pyrazine, isoxazole, thiazole, isothiazole, thiadiazole, oxazole, oxadiazole and other monocyclic heteroaromatic rings. Examples may also include isoindole, indazole, indolizine, isoindoline, quinoline, isoquinoline, cinnoline, 2,3-diazanaphthalene, quinazoline, naphthyridine, quinoxaline, purine, pteridine, benzimidazole, benzisoxazole, benzoxazole, benzoxadiazole, benzisothiazole, benzothiazole, benzothiadiazole, benzofuran, isobenzofuran, benzothiophene, benzotriazole, imidazopyridine, triazolopyridine, imidazothiazole, pyrazinopyridazine, benzimidazoline and other polycyclic heteroaromatic rings.

The "heteroaryl" or "heteroaryl group" (hereinafter, collectively referred to as "heteroaryl") in the present invention refers to a monovalent, divalent or higher group derived from the "heteroaromatic ring". In addition, the "heteroaryl" in the present invention may also be aromatic cyclic hydrocarbyl containing 5, 6, 7, 8, 9, 10, 11, 12, 13 or 14 ring atoms and at least one heteroatom selected from O, S and N. That is, heteroaryl may be 5-14 membered heteroaryl, 5-10 membered heteroaryl or 5-6 membered heteroaryl. Heteroaryl may have 1, 2, 3, 4 or 5 heteroatoms as ring atoms. In addition, the case where a carbon or sulfur atom is replaced with O or N is also included in heteroaryl, for example, a carbon atom is replaced with C(O), and a sulfur atom is replaced with S(O) or S(O)₂. Heteroaryl includes monocyclic and polycyclic heteroaryl. Unless otherwise specified, a certain membered heteroaryl includes all possible monocyclic, polycyclic, fully aromatic or partially aromatic heteroaryl.

Monocyclic heteroaryl may be 5-6 membered heteroaryl, and examples include, but are not limited to, furyl, imidazolyl, isoxazolyl, thiazolyl, isothiazolyl, oxadiazolyl, oxazolyl, pyridyl, pyridazinyl, pyrimidinyl, pyrazinyl, pyrazolyl, pyrrolyl, tetrazolyl, thiadiazolyl, thienyl, triazolyl and triazinyl. In certain embodiments, polycyclic heteroaryl refers to a group formed by linking a monocyclic heteroaromatic ring to phenyl, cycloalkenyl, heteroaryl, cycloalkyl and heterocyclyl. Polycyclic heteroaryl may be 8-14 membered ortho-fused heteroaryl or 9-10 membered ortho-fused heteroaryl, and examples include, but are not limited to, benzimidazolyl, benzofuranyl, benzothienyl, benzoxadiazolyl, benzothiadiazolyl, benzothiazolyl, cinnolinyl, 5,6-dihydroquinolin-2-yl, 5,6-dihydroisoquinolin-1-yl, furopyridinyl, indazolyl, indolyl, isoindolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4 -yl, 5,6,7,8-tetrahydroisoquinolin-1-yl, thienopyridyl, 4,5,6,7-tetrahydro[c][1,2,5] oxadiazolyl and 6,7-dihydro[*c*][1,2,5]oxadiazol-4(5*H*)keto. The heteroaryl may also be a divalent group derived from a group described above.

The "5-14 membered cyclic group" in the present invention refers to a group having 5, 6, 7, 8, 9, 10, 11, 12, 13 or 14 ring atoms, and may be the cycloalkyl, cycloalkenyl, heterocyclyl, aryl and heteroaryl of the present invention having 5 to 14 ring atoms. Specifically, the "5-14 membered cyclic group" may be 5-10 membered cyclic group or 5-6 membered cyclic group. Examples include, but are not limited to, groups derived from pyrroline, pyrrolidine, piperidine, piperazine, morpholine, thiomorpholine, tetrahydropyran, dihydropyridine, dihydropyridazine, dioxane, oxathiolane, thiacyclopentane, tetrahydrofuran, tetrahydropyran, tetrahydrothiazole, tetrahydroisothiazole, pyrrole, pyrazine, pyrazole, indole, tetrazole, furan, thiophene, pyridine, imidazole, triazole, tetrazole, triazine, pyridazine, pyrimidine, pyrazine, isoxazole, thiazole, isothiazole, thiadiazole, oxazole, oxadiazole, benzene, *etc.* Preferably, it is 5-6 membered oxygen-containing cyclic group, *i.e*., cyclic group having at least one O and 5 or 6 ring atoms.

The term "at least one" in the present invention refers to 1 to the number of all groups able to be chemically substituted, preferably 1-6, more preferably 1-5, more preferably 1-4, more preferably 1-3, more preferably 1-2 or more preferably 1.

The ester in the present invention refers to a pharmaceutically acceptable ester formed by the compound of the present invention, and more specifically to formate, acetate, propionate, butyrate, acrylate and ethyl succinate of the compound of the present invention, but not limited thereto.

The "pharmaceutically acceptable salt" in the present invention refers to a pharmaceutically acceptable addition salt of acid and base or a solvate thereof. Such pharmaceutically acceptable salts include salts of the following acids: hydrochloric acid, phosphoric acid, hydrobromic acid, sulfuric acid, sulfurous acid, formic acid, toluenesulfonic acid, methanesulfonic acid, nitric acid, benzoic acid, citric acid, tartaric acid, maleic acid, hydroiodic acid, alkanoic acid (such as acetic acid, HOOC-(CH₂)ₙ-COOH (wherein n is 0-4)), *etc.* It also includes alkali salts: sodium salt, potassium salt, calcium salt, ammonium salt, *etc.* Those skilled in the art know a variety of pharmaceutically acceptable non-toxic addition salts.

The hydrogen atom, fluorine atom, carbon atom, nitrogen atom, oxygen atom, sulfur atom and the like in the present invention also include the respective radioactive isotopes or stable isotopes thereof.

The tumor described in the invention includes sarcoma, lymphoma and cancer, and may specifically include lung cancer, squamous cell carcinoma, bladder cancer, gastric cancer, ovarian cancer, peritoneal cancer, breast cancer, breast ductal carcinoma, head and neck cancer, endometrial cancer, corpus carcinoma, rectal cancer, liver cancer, kidney cancer, renal pelvis cancer, esophageal cancer, esophageal adenocarcinoma, glioma, prostate cancer, thyroid cancer, female reproductive system cancer, carcinoma in situ, lymphoma, neurofibromatosis, bone cancer, skin cancer, brain cancer, colon cancer, testicular cancer, gastrointestinal stromal tumor, oral cancer, pharyngeal cancer, multiple myeloma, leukemia, non-Hodgkin's lymphoma, villous adenoma, melanoma, cytoma and sarcoma.

In the present invention, the expression "A and/or B" refers to A alone or both A and B. For example, "TAM family kinase and/or CSF1R kinase" refers to "TAM family kinase" alone or both "TAM family kinase" and "CSF1R kinase".

The "stereoisomers" of the compounds of formula (I), (II), (III), (IV), (V) and (VI) of the present invention refer to enantiomers produced when there is a chiral carbon atom in the compounds of formula (I), (II), (III), (IV), (V) and (VI), and *cis-trans* isomers produced when there is a carbon-carbon double bond or cyclic structure in the compounds. All enantiomers, diastereomers, racemtes, geometric isomers, epimers and mixtures thereof of the compounds of formula (I) are included in the scope of the present invention. The definition of the compounds of the present invention encompasses all possible stereoisomers and mixtures thereof. In particular, it includes racemic forms and isolated optical isomers with specified activities. Racemic forms can be resolved by physical methods, such as fractional crystallization, separation or crystallization of diastereomeric derivatives, or chiral column chromatography. An individual optical isomer can be obtained from the racemate by conventional methods (*e.g*., salifying with an optically active acid, followed by crystallization).

The "tautomers" of the compounds of formula (I), (II), (III), (IV), (V) and (VI) of the present invention refer to the functional isomers produced when an atom in the compounds of formula (I), (II), (III), (IV), (V) and (VI) moves rapidly between two positions. When the hydrogen at the alpha position of the carbonyl-containing functional group is on the alpha carbon, a keto tautomer is produced; and when the hydrogen at the alpha position of the carbonyl-containing functional group is on the oxygen of the carbonyl, an enol tautomer is produced.

The pharmaceutical composition of the present invention includes at least one of the compound of formula I, II, III, IV, V or VI, and the pharmaceutically acceptable salt, ester, stereoisomer or tautomer thereof.

The pharmaceutical composition of the present invention includes the compound of formula I, II, III, IV, V or VI, or the pharmaceutically acceptable salt, ester, stereoisomer or tautomer thereof, and optionally one or more pharmaceutical carriers.

The pharmaceutical composition of the invention can be administered to a patient or subject in need of prophylaxis and/or treatment in any suitable manner well known in the art, for example, oral, parenteral (including subcutaneous, intramuscular, intravenous, intra-arterial, intradermal, intrathecal, and epidural), transdermal, rectal, nasal, transpulmonary, topical (including buccal and sublingual), vaginal, intraperitoneal, intrapulmonary and intranasal administration.

The pharmaceutical composition of the invention can be formulated into a conventional solid formulation, such as tablet, capsule, pill and granule, and can also be formulated into an oral liquid formulation, such as oral solution, oral suspension and syrup. In the preparation of an oral formulation, one or more of suitable excipient, diluent, sweetener, solubilizer, lubricant, binder, tablet disintegrant, stabilizer, preservative and encapsulating material may be added. For parenteral administration, the pharmaceutical composition can be formulated as an injection, a sterile powder for injection and a concentrated solution for injection. The injection can be made by a conventional method existing in the pharmaceutical field, and during the preparation process, no additive may be added, or an appropriate additive may be added according to the property of the medicine. For rectal administration, the pharmaceutical composition can be made into a suppository and the like. For transpulmonary administration, the pharmaceutical composition can be made into an inhalant, spray or the like. In the present invention, suitable solid carriers include, but are not limited to, cellulose, glucose, lactose, mannitol, magnesium stearate, magnesium carbonate, sodium carbonate, sodium saccharin, sucrose, dextrin, talc, starch, pectin, gelatin, tragacanth, arabic gum, sodium alginate, p-hydroxybenzoate, methylcellulose, sodium carboxymethyl cellulose, low-melting wax, cocoa butter, *etc.* Suitable liquid carriers include, but are not limited to, water, ethanol, polyol *(e.g.,* glycerin, propylene glycol, and liquid polyethylene glycol), vegetable oil, glyceride, and mixtures thereof.

Methods for preparing the pharmaceutical composition of the present invention are generally known. The pharmaceutical composition of the present invention is prepared by known methods, including conventional mixing, granulating, tableting, coating, dissolving or lyophilizing.

The pharmaceutical formulation is preferably in the form of unit dose. In this form, the formulation is subdivided into unit dosages containing an appropriate amount of active component. The unit dosage form can be packaged into a package containing a discrete quantity of formulation, such as a packaged tablet, capsule, or powder in a vial or ampoule.

Dosage of a medicament depends on various factors, including the age, weight and state of a patient, and the route of administration. The precise dosage administered is determined based on the judgment of a treating physician. The usual dosage for administration of the active compound may be, for example, about 0.01 to about 100 mg/day, about 0.05 to about 75 mg/day, about 0.1 to about 50 mg/day, or about 5 to about 10 mg/day. The desired dosage also depends on the specific compound employed, the severity of a disease, the route of administration, the weight and health status of a patient, and the judgment of a treating physician.

### Examples

If specific reaction conditions are not noted in the examples, conventional conditions or conditions recommended by the manufacturers shall be adopted. The adopted reagents or instruments, without manufacturers, are all commercially-available conventional products.

In the present invention, unless otherwise stated, (i) the temperature is expressed in Celsius (°C), and unless otherwise stated, the operation is performed at room temperature; (ii) the progress of the reaction is tracked by thin-layer chromatography (TLC) or LC-MS; and (iii) the final product has clear proton nuclear magnetic resonance spectroscopy (¹H-NMR) data and mass spectrometry (MS) data. The abbreviations and English expressions used in the present invention have the following meanings:
DAST: diethylaminosulfur trifluoride
DCM: dichloromethane
-Boc: *tert*-butyloxycarbonyl
TEA: triethylamine
TBSC1: *tert*-Butyldimethylsilyl chloride
TBS-: *tert*-butyldimethylsilyl
DMSO: dimethyl sulfoxide
NaHMDS: sodium bis(trimethylsilyl)amide
TBAF: tetra-n-butylammonium fluoride
MsCl: methanesulfonyl chloride
TFA: trifluoroacetic acid
DMF: *N, N*-dimethylformamide
Pd₂(dba)₃: tris(dibenzylideneacetone)dipalladium(0)
conc.HCl: concentrated hydrochloric acid
NBS: N-bromosuccinimide
AIBN: azobisisobutyronitrile
THF: tetrahydrofuran
TMSCN: trimethylsilyl cyanide
CPBA: *m*-chloroperoxybenzoic acid
TMSI: trimethylsilane imidazole
BHT: dibutylhydroxytoluene
Pd(PPh₃)₂Cl₂: bis (triphenylphosphine) dichloropalladium
EA: ethyl acetate
MTBE: methyl *tert-butyl* ether
PE: petroleum ether
HATU: 2-(7-azabenzotriazol-1-yl)-*N*,*N*,*N'*,*N'*-tetramethyluronium hexafluorophosphate PBu3: tributylphosphine
DMF-DMA: *N*, N-dimethylformamide dimethyl acetal
DIPEA/DIEA: *N, N*-diisopropylethylamine DEC: dichloroethane
Pd(dppf)Cl₂•CH₂Cl₂: [1,1'-bis(diphenylphosphino)ferrocene] dichloropalladium dichloromethane adduct
rt: room temperature

### Example 1: synthesis of 4-oxo-1-((tetrahydro-2H-pyran-4-yl)methyl)-5-(p-tolyl)-1,4-dihydropyridin-3-formic acid

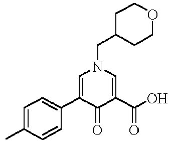

### Steps

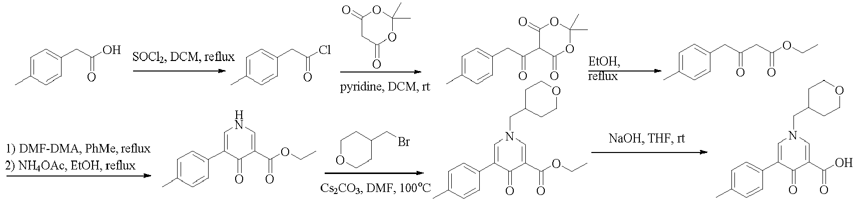

### Step 1: synthesis of 2-(p-tolyl)acetyl chloride

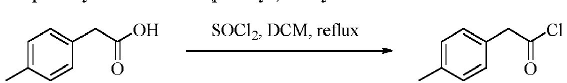

Under a nitrogen atmosphere, 2-(*p*-tolyl)acetic acid (25 g, 0.17 mol, 1.0 eq) was added to dichloromethane (250 mL), and oxalyl chloride (42.25 g, 0.34 mol, 2.0 eq) and DMF (0.25 mL) were then successively added dropwise at room temperature. The reaction solution reacted at reflux for 1 hr. The reaction solution was concentrated to remove the solvent. The concentrate was re-dissolved in dichloromethane (100 mL), and then concentrated once again (this process was repeated twice). The obtained crude product was directly used in the next step without purification.

### Step 2: synthesis of 2,2-dimethyl-5-(2-(p-tolyl)acetyl)-1,3-dioxan-4,6-dione

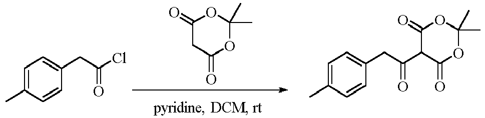

2,2-dimethyl-1,3-dioxan-4,6-dione (36 g, 0.25 mol, 1.05 eq) and pyridine (31 g, 0.39 mol, 2.3 eq) were added to dichloromethane (350 mL), and the acetyl chloride prepared in the previous step was added dropwise at 0 °C. The reaction solution was stirred at 0 °C for 30 min. After the reaction was completed, the reaction solution was washed successively with 10% hydrochloric acid (100 mL × 2) and water (100 mL × 2), dried, and concentrated to obtain 2,2-dimethyl-5-(2-(*p*-tolyl)acetyl)-1,3-dioxan-4,6-dione (53 g crude), which was directly used in the next step.

### Step 3: synthesis of ethyl 3-oxo-4-(p-tolyl)butyrate

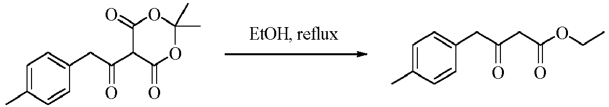

2,2-dimethyl-5-(2-(*p*-tolyl)acetyl)-1,3-dioxan-4,6-dione (53 g crude) was added to absolute ethanol (500 mL), and the reaction solution was heated to reflux in an oil bath and stirred overnight. After the reaction was completed, as detected by TLC, the reaction solution was concentrated. The crude product was separated by silica gel column chromatography to obtain ethyl 3-oxo-4-(*p*-tolyl)butyrate (yellow oily substance, 15 g, three-step yield: 60%).

### Step 4: synthesis of ethyl 4-oxo-5-(p-tolyl)-1,4-dihydropyridin-3-carboxylate

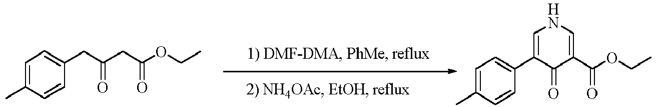

Ethyl 3-oxo-4-(*p*-tolyl)butyrate (5 g, 22.7 mmol, 1.0 eq) was added to toluene (75 mL), and DMF-DMA (8.1 g, 68.1 mmol, 3.0 eq) was then added. The reaction solution was heated to reflux for 2 hrs. After the reaction was completed, as detected by TLC, the reaction solution was concentrated. The crude product was added to absolute ethanol (50 mL), ammonium acetate (8.75 g, 113.5 mmol, 5.0 eq) was then added, and the reaction solution reacted at reflux for 1.5 hrs. After the reaction was completed, as detected by TLC, the reaction solution was cooled to room temperature, and filtered under vacuum. The filter cake was washed with ethanol (5 mL × 3) and dried to obtain a white solid (1.5 g, yield: 26%).

### Step 5: synthesis of ethyl 4-oxo-1-((tetrahydro-2H-pyran-4-yl)methyl)-5-(p-tolyl)-1,4-dihydropyridin-3 -formate

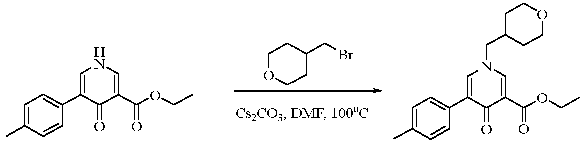

Ethyl 4-oxo-5-(*p*-tolyl)-1,4-dihydropyridin-3-carboxylate (1.56 g, 6.1 mmol, 1.0 eq) was added to DMF (45 mL), and cesium carbonate (3.96 g, 12.2 mmol, 2.0 eq) and 4-(bromomethyl)tetrahydro-2*H*-pyran (1.2 g, 6.7 mmol, 1.1 eq) were then added. The reaction solution was heated to 100 °C and reacted overnight. After the reaction was completed, as detected by TLC and LC-MS, the reaction solution was cooled to room temperature. Water (100 mL) was added and the mixture was separated. Ethyl acetate (50 mL × 5) was added to the aqueous phase for extraction, and the extract was washed with saturated brine (50 mL × 3), dried, filtered and concentrated. The crude product was purified by silica gel column chromatography to obtain a yellow solid (1.2 g, 77%).

### Step 6: synthesis of 4-oxo-1-((tetrahydro-2H-pyran-4-yl)methyl)-5- (p-tolyl)-1,4-dihydropyridin-3-formic acid

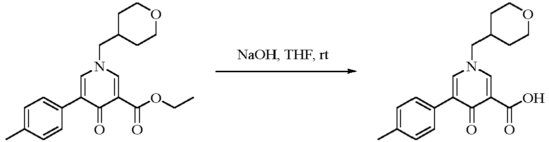

Ethyl 4-oxo-1-((tetrahydro-2*H*-pyran-4-yl)methyl)-5-(*p*-tolyl)-1,4- dihydropyridin-3 - formate (500 mg, 1.41 mmol, 1.0 eq) was added to a mixture solution of methanol (2.5 mL) and tetrahydrofuran (2.5 mL), and then a solution (2.5 mL) of sodium hydroxide (112 mg, 2.82 mmol, 2.0 eq) in water was added. Then the reaction solution was stirred at room temperature for 1 hr. After the reaction was completed, as detected by HPLC and LC-MS, the reaction solution was concentrated to remove most of methanol and tetrahydrofuran, and methyl *tert-*butyl ether (2.5 mL × 3) was added for extraction. The aqueous phase was kept at a temperature below 10 °C, and the pH was adjusted to 2 with 1 mol/L hydrochloric acid. After the solids were precipitated, the reaction mixture was filtered under vacuum. The filter cake was washed with a small amount of water (2 mL x 3), and dried to obtain a white solid (410 mg, yield: 89%).

¹HNMR (300 MHz, DMSO-*d₆*) δ ppm: δ 8.76 (s, 1H), 8.37 (s, 1H), 7.60 (d, *J* = 8.1 Hz, 2H), 7.28 (d*, J* = 7.8 Hz, 2H), 4.15 (d*, J* = 7.2 Hz, 2H), 3.86 (d*, J* = 10.8 Hz, 2H), 3.24 (m, 2H), 2.35 (s, 3H), 2.10 (m, 1H), 1.40 (m, 2H), 1.25-1.23 (m, 2H).

LC-MS(*m*/*z*)= 328.0 [M+H]⁺.

### Example 2: synthesis of N-(5-((6,7-dimethoxyquinolin-4-yl)oxy)pyridin-2-yl)-4-oxo-1-((tetrahydro-2H-pyran-4-yl)methyl)-5-(p-tolyl)-1,4-dihydropyridin-3-carboxamide (compound 1)

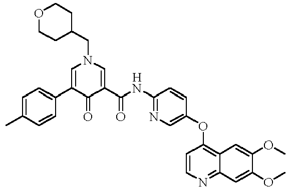

### Steps

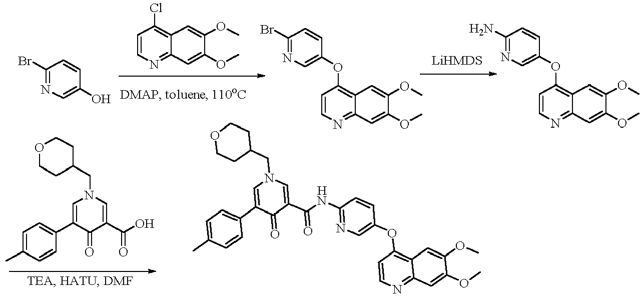

### Step 1: synthesis of 4-((6-bromopyridin-3-yl)oxy)-6,7-dimethoxyquinoline

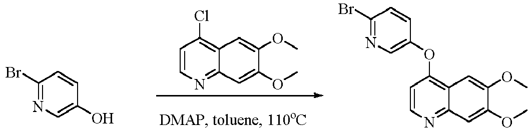

6-bromopyridin-3-ol (1.74 g, 10.0 mmol, 1.0 eq), 4-chloro-6,7- dimethoxybenzopyridine (2.24 g, 10.0 mmol, 1.0 eq) and 4- dimethylaminopyridine (3.67 g, 30.0 mmol, 3.0 eq) were dissolved in toluene (50 mL), and the reaction solution was heated to 100 °C and reacted for 16 hrs. After the reaction was completed, as detected by LC-MS, the reaction solution was directly concentrated, and the crude product was purified by silica gel column chromatography (DCM : MeOH = 50:1 ∼ 20:1) to obtain 4-((6-bromopyridin-3-yl)oxy)-6,7-dimethoxyquinoline (white solid, 1.70 g, yield: 47%).

### Step 2: synthesis of 5-((6,7-dimethoxyquinolin-4-yl)oxy)pyridin-2-amine

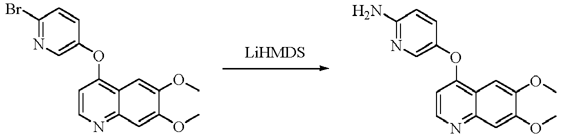

4-((6-bromopyridin-3-yl)oxy)-6,7-dimethoxyquinoline (700 mg, 2 mmol, 2.0 eq) and [1,1'-biphenyl]-2-yldicyclohexylphosphine (70 mg, 0.2 mmol, 0.1 eq) were dissolved in tetrahydrofuran (10 mL), lithium bis(trimethylsilyl) amide (4 mL, 4 mmol, 2.0 eq) was added, and the reaction solution was heated to 65 °C and reacted for 2 hrs under a nitrogen atmosphere. After the reaction was completed, as detected by LC-MS, the reaction solution was poured into water (30 mL). Ethyl acetate (30 mL × 3) was added for extraction, and the organic phases were combined, dried, filtered and concentrated. The crude product was purified by silica gel column chromatography (DCM : MeOH = 50:1 ∼ 10:1) to obtain 5-((6,7-dimethoxyquinolin-4-yl)oxy)pyridin-2-amine (light yellow solid, 100 mg, yield: 17%).

### Step 3: synthesis of N-(5-((6,7-dimethoxyquinolin-4-yl)oxy)pyridin-2-yl) -4-oxo-1-((tetrahydro-2H-pyran-4-yl)methyl)-5-(p-tolyl)-1,4-dihydropyridin-3-carboxamide

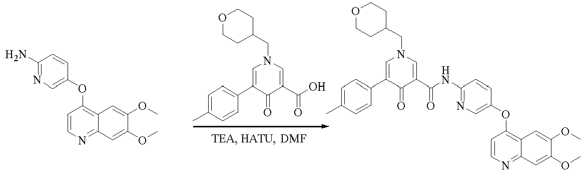

Raw material 4-oxo-1-((tetrahydro-2*H*-pyran-4-yl)methyl)-5-(*p*-tolyl)-1, 4-dihydropyridin-3-carboxylic acid (60 mg , 0.183 mmol, 1.0 eq) was dissolved in *N, N-*dimethylformamide (10 mL), and triethylamine (57 mg, 0.550 mmol, 3.0 eq) and HATU (104 mg, 0.274 mmol, 1.5 eq) were added successively. The reaction solution was stirred at room temperature for 1 hr. 5-((6,7-dimethoxyquinolin-4-yl)oxy)pyridin-2-amine (100 mg, 0.336 mmol, 1.8 eq) was added, and the reaction solution was stirred at room temperature for 1 hr. After the reaction was completed, as detected by LC-MS, the reaction solution was poured into water (30 mL). Ethyl acetate (30 mL × 3) was added for extraction, and the organic phases were combined, dried, filtered and concentrated. The crude product was purified by silica gel column chromatography (DCM : MeOH = 50:1 ∼ 10:1) to obtain N-(5-((6,7- dimethoxyquinolin-4-yl)oxy)pyndin-2-yl)-4-oxo-1-((tetrahydro-2*H*-pyran-4-yl)methyl)-5-(*p*-tolyl)-1,4-dihydropyridin-3-carboxamide (light yellow solid, 19 mg, yield: 17%).

¹HNMR(400MHz, DMSO-*d₆*) δ(ppm): 13.45 (s, 1H), 8.50-8.73 (d, 1H), 8.44-8.49 (d, 1H), 8.38-8.42 (d, 1H), 8.37 (s, 1H), 8.16-8.17 (d, 1H), 7.83-7.86 (m, 2H), 7.61 (s, 1H), 7.59 (s, 1H), 7.25-7.42 (m, 2H), 6.55-6.56 (d, 1H), 4.10-4.12 (d, 2H), 3.96 (s, 3H), 3.95 (s, 3H), 3.86-3.88 (m, 2H), 3.24-3.30 (m, 2H), 2.36 (s, 3H), 2.11-2.12 (m, 1H), 1.45-1.48 (m, 2H), 1.34-1.37 (m, 2H).

Molecular formula: C₃₅H₃₄N₄O₆ Molecular weight: 606.25 LC-MS(Pos, *m*/*z*)=607.25 [M+H]⁺.

### Example 3: synthesis of intermediate ethyl 4-oxo-5-(p-tolyl)-1,4- dihydropyridazin- 3-carboxylate hydrochloride

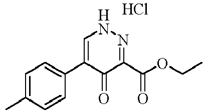

### Step 1: synthesis of 2-(p-tolyl)acetyl chloride

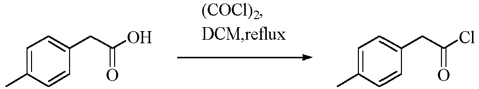

2-(*p*-tolyl)acetic acid (9.0 g, 60 mmol, 1.0 eq) was dissolved in DCM (90 mL), and oxalyl chloride (15.2 g, 120 mmol, 2.0 eq) and DMF (0.1 mL) were then successively added dropwise at room temperature. The reaction solution was heated to reflux for 2 hrs. The reaction solution was cooled to room temperature, and concentrated to obtain a product (10 g crude), which was directly used in the next step.

### Step 2: synthesis of ethyl 2-diazo-3-oxo-4-(p-tolyl)butyrate

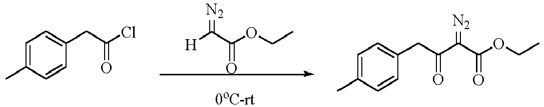

Intermediate 2-(*p*-tolyl)acetyl chloride (10 g crude, 60 mmol, 1.0 eq) was added to a three-necked flask and cooled to 0 °C, and ethyl diazoacetate (13.7 g, 120 mmol, 2.0 eq) was slowly added dropwise. Then the reaction solution was warmed to room temperature and stirred overnight. After the reaction was completed, as detected by TLC and LC-MS, the reaction solution was diluted with EA, washed twice with saturated potassium carbonate aqueous solution, and then washed with saturated brine. The resulting solution was dried and concentrated. The crude product was purified by silica gel column chromatography (100-200 mesh silica gel, EA/PE = 0 ∼ 10%) to obtain a yellow oily product (6.0 g, two-step yield: 41%).

### Step 3: synthesis of ethyl 2-hydrazono-3-oxo-4-(p-tolyl)butyrate

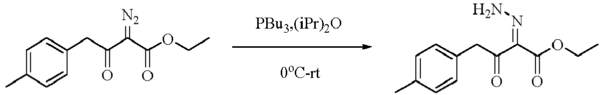

Intermediate ethyl 2-diazo-3-oxo-4-(*p*-tolyl)butyrate (6.0 g, 24.4 mmol, 1.0 eq) was dissolved in isopropyl ether (60 mL), the resulting solution was cooled to 0 °C, and tributylphosphine (5.4 g, 26.8 mmol, 1.1 eq) was added dropwise. Then the reaction solution was warmed to room temperature and reacted for 2 hrs. After the reaction was completed, as detected by TLC and LC-MS, EA (50 mL) was added, and the reaction solution was washed twice with water, and then washed with saturated brine. The resulting solution was dried and concentrated. The crude product was purified by silica gel column chromatography (100-200 mesh silica gel, EA/PE = 0-20%) to obtain a white solid product (4.1 g, yield: 67%).

### Step 4: synthesis of tert-butyl 2-(1-ethoxy-1,3-dioxo-4-(p-tolyl)but-2-enyl)hydrazin-1-carboxylate

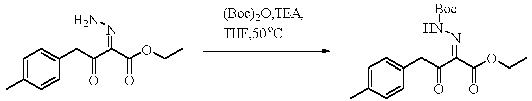

Intermediate ethyl 2-hydrazono-3-oxo-4-(*p*-tolyl)butyrate (5.4 g, 21.75 mmol, 1.0 eq) was dissolved in THF (60 mL), and Boc anhydride (5.2 g, 23.92 mmol, 1.1 eq), triethylamine (6.6 g, 65.24 mmol, 3.0 eq) and DMAP (20 mg) were added successively. Then the reaction solution reacted overnight at room temperature. After the reaction was completed, as detected by TLC and LC-MS, the reaction solution was concentrated under reduced pressure to remove most of the solvent. The residue was added with EA and water, and the liquid was separated. The organic phase was washed with saturated brine, dried and concentrated. The crude product was purified by silica gel column chromatography (100-200 mesh silica gel, EA/PE = 0-10%) to obtain a yellow oily product (5.1 g, yield: 67%).

### Step 5: synthesis of ethyl 4-oxo-5-(p-tolyl)-1,4-dihydropyridazin-3- carboxylate hydrochloride

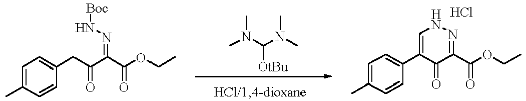

1-*tert*-butoxy-*N*, *N, N'*, *N*'-tetramethylmethanediamine (2.54 g, 14.6 mmol, 1.0 eq) was dissolved in THF (20 mL), and the resulting solution was heated to reflux. The solution of *tert-*butyl 2-(1-ethoxy-1,3-dioxo-4-(*p*-tolyl) but-2-enyl)hydrazin-1-carboxylate (5.1 g, 14.6 mmol, 1.0 eq) in THF (30 mL) was added dropwise, and then the reaction solution reacted for 30 min. After the reaction was completed, as detected by TLC, the reaction solution was cooled to 20 °C. The solution of 4 mol/L hydrogen chloride in 1,4-dioxane (30 mL) was added dropwise, and then the reaction solution was stirred for 30 min. After the reaction was completed, as detected by LC-MS, the reaction system was concentrated. EA was added to the residue, and the mixture was stirred for 15 min. The Solids were precipitated, The solids were filtered out under vacuum, and dried to obtain a light yellow product (4.2 g, yield: 97.7%).

¹H NMR(300 MHz, DMSO-*d₆*) δ(ppm): 14.14 (brs, 1H), 8.97 (brs, 2H), 8.56 (s, 1H), 7.74 (d, *J* = 7.5 Hz, 2H), 7.23 (d, *J* = 7.8 Hz, 2H), 4.30 (q, *J* = 7.2 Hz, 2H), 2.33 (s, 3H), 1.28 (t, *J* = 6.9 Hz, 3H).

### Example 4: synthesis of intermediate 4-oxo-1-((tetrahydro-2H-pyran-4-yl) methyl)-5-(p-tolyl)-1,4-dihydropyridazin-3-carboxylic acid

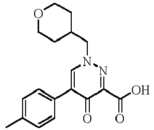

### Step 1: synthesis of ethyl 4-oxo-1-((tetrahydro-2H-pyran-4-yl)methyl)- 5-(p-tolyl)-1,4-dihydropyridazin-3 -carboxylate

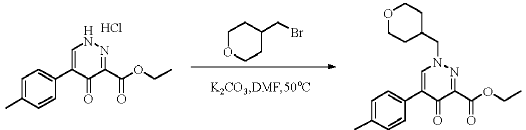

Ethyl 4-oxo-5-(*p*-tolyl)-1,4-dihydropyridazin-3-carboxylate hydrochloride (4.2 g, 14.3 mmol, 1.0 eq) was dissolved in DMF (30 mL), potassium carbonate (7.88 g, 57.2 mmol, 4.0 eq) and 4-(bromomethyl)tetrahydro-2*H*-pyran (3.06 g, 17.1 mmol, 1.2 eq) were added, and the reaction solution was heated to 50 °C and stirred for 5 hrs. After the reaction was completed, as detected by LC-MS, the reaction solution was filtered under vacuum to remove the insolubles. The crude product was firstly purified by silica gel column chromatography (100-200 mesh silica gel, EA/PE = 10-50%), and then recrystallized with a mixed solvent of DCM and MTBE (1:10) to obtain a product (1.6 g, yield: 32%).

### Step 2: synthesis of 4-oxo-1-((tetrahydro-2H-pyran-4-yl)methyl)-5-(p- tolyl)-1,4-dihydropyridazin-3-carboxylic acid

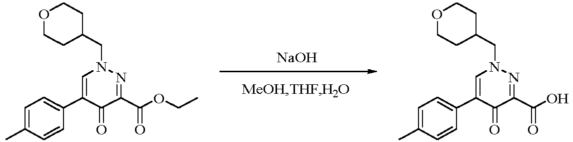

Ethyl 4-oxo-1-((tetrahydro-2*H*-pyran-4-yl)methyl)-5-(*p*-tolyl)-1,4-dihydropyridazin-3-carboxylate (1.6 g, 4.5 mmol, 1.0 eq) was dissolved in a mixture solution of MeOH, THF and H₂O (1:1:1, 20 mL), then NaOH (899 mg, 22.4 mmol, 5.0 eq) was added, and the reaction solution was stirred at room temperature for 2 hrs. After the reaction was completed, as detected by LC-MS, the reaction solution was concentrated to remove MeOH and THF. pH of the residue was adjusted to 3 with a 1 mol/L HCl aqueous solution, and a large number of white solids were precipitated. The mixture was filtered under vacuum, and the filter cake was washed twice with water, and dried to obtain a white solid product (1.35 g, yield: 91%).

¹H NMR(300 MHz, DMSO-*d₆*) δ(ppm): 15.98 (brs, 1H), 9.06 (s, 1H), 7.78 (d, *J* = 7.5 Hz, 2H), 7.32 (d, *J* = 7.2 Hz, 2H), 4.27 (d, *J* = 7.2 Hz, 2H), 3.87-3.83 (m, 2H), 3.27-3.23 (m, 2H), 2.37 (s, 3H), 2.25-2.15 (m, 1H), 1.52-1.48 (m, 2H), 1.35-1.32 (m, 2H).

### Example 5: synthesis of intermediate 4-oxo-1-(tetrahydro-2H-pyran-4-yl)-5-(p-tolyl)-1,4-dihydropyridazin-3-carboxylic acid

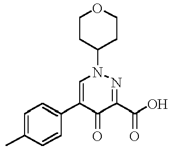

### Step 1: synthesis of ethyl 4-oxo-1-(tetrahydro-2H-pyran-4-yl)-5-(p-tolyl)-1,4-dihydropyridazin-3 -carboxylate

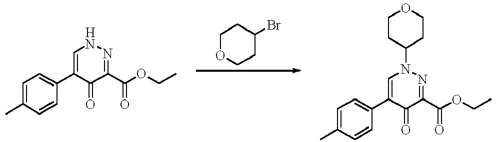

Ethyl 4-oxo-5-(*p*-tolyl)-1,4-dihydropyridazin-3-carboxylate (1.15 g, 4.452 mmol, 1.0 eq) and 4-bromotetrahydro-2*H*-pyran (882.0 mg, 5.343 mmol, 1.2 eq) were dissolved in *N, N-*dimethylformamide (10.0 mL), then cesium carbonate (4.352 g, 13.356 mmol, 3.0 eq) was added, and the reaction solution was heated to 100 °C and reacted for 2 hrs. After the reaction was completed, as detected by TLC, the reaction solution was filtered, concentrated under reduced pressure to remove *N, N*-dimethylformamide. Ethyl acetate was added, and the resulting mixture was washed four times with water, dried and concentrated under reduced pressure. The crude product was purified by silica gel column chromatography (DCM : MeOH = 200: 1 ∼ 30: 1) to obtain a product (1.0 g, yield: 87.0%).

### Step 2: synthesis of 4-oxo-1-(tetrahydro-2H-pyran-4-yl)-5-(p-tolyl)-1, 4-dihydropyridazin-3-carboxylic acid

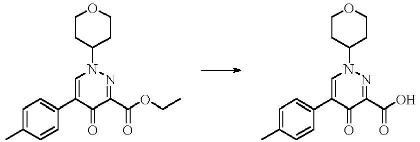

Ethyl 4-oxo-1-(tetrahydro-2*H-*pyran-4-yl)-5-(*p*-tolyl)-1,4-dihydropyndazin -3-carboxylate (1.0 g, 2.92 mmol, 1.0 eq) was dissolved in tetrahydrofuran (10.0 mL), then a 1 mol/L lithium hydroxide aqueous solution (5.0 mL) was added, and the reaction solution was stirred for 2 hrs. After the reaction was completed, as detected by TLC, the reaction solution was concentrated under reduced pressure to remove tetrahydrofuran. The pH of the remaining aqueous solution was adjusted to 4-5, and a large number of solids were precipitated. The mixture was filtered under vacuum, and the filter cake was dried to obtain a white solid product (800.0 mg, yield: 80%).

### Example 6: synthesis of intermediate 4-oxo-1-(tetrahydro-2H-pyran-4-yl)-5-(p-tolyl)-1,4-dihydropyridin-3-carboxylic acid

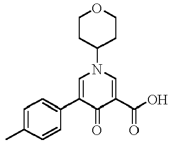

### Step 1: synthesis of 2-(p-tolyl)acetyl chloride

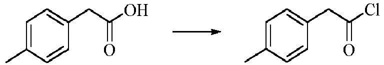

2-(p-tolyl)acetic acid (5.0 g, 33.293 mmol, 1.0 eq) and thionyl chloride (7.92 g, 66.587 mmol, 2.0 eq) were dissolved in dichloromethane (50.0 mL), and the reaction solution was heated to 60 °C and reacted at reflux for 3 hrs under a nitrogen atmosphere. After the reaction was completed, as detected by TLC, the reaction solution was concentrated under reduced pressure. An appropriate amount of dichloromethane was added, and the resulting mixture was concentrated, and this process was repeated twice to obtain a yellow oily product.

### Step 2: synthesis of 2,2-dimethyl-5-(2-(p-tolyl)acetyl)-1,3-dioxan-4,6-dione

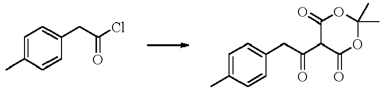

2,2-dimethyl-1,3-dioxan-4,6-dione (5.758 g, 33.952 mmol, 1.2 eq) and triethylamine (8.085 g, 79.903 mmol, 2.4 eq) were dissolved in dichloromethane (40.0 mL), and the mixture was cooled to 0 °C under a nitrogen atmosphere. 2-(*p*-tolyl)acetyl chloride diluted in dichloromethane (10.0 mL) was slowly added dropwise, and then the reaction solution was warmed to room temperature and reacted for 3 hrs. After the reaction was completed, as detected by TLC, the reaction solution was washed three times with 1 mol/L hydrochloric acid, and then washed twice with a saturated sodium chloride solution. The resulting solution was dried, and concentrated under reduced pressure to obtain a yellow oily product (crude product).

### Step 3: synthesis of ethyl 3-oxo-4-(p-tolyl)butyrate

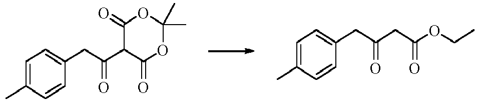

2,2-dimethyl-5-(2-(*p*-tolyl)acetyl)-1,3-dioxan-4,6-dione (crude) was dissolved in ethanol (50.0 mL), and the reaction solution was heated to 100 °C and reacted at reflux for 3 hrs. The reaction was not completed, as detected by TLC, and concentrated hydrochloric acid (1.5 mL) was added. The reaction solution reacted for 2 hrs. After the reaction was completed, as detected by TLC, the reaction solution was concentrated under reduced pressure. The crude product was purified by silica gel column chromatography (PE : EA = 75: 1 ∼ 10: 1) to obtain a yellow oily product (2.9 g, three-step yield: 58%).

### Step 4: synthesis of ethyl 5-(dimethylamino)-2-((dimethylamino) methylene)-3-oxo-4-(p-tolyl)pent-4-enoate

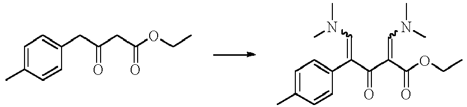

Ethyl 3-oxo-4-(*p*-tolyl)butyrate (2.9 g, 13.166 mmol, 1.0 eq) was dissolved in toluene (75.0 mL), DMF-DMA (4.706 g, 39.497 mmol, 3.0 eq) was added, and the reaction solution was heated to 100 °C and reacted at reflux for 3 hrs. After the reaction was completed, as detected by TLC, the reaction solution was concentrated under reduced pressure to obtain a yellow oily product (crude).

### Step 5: synthesis of ethyl 4-oxo-1-(tetrahydro-2H-pyran-4-yl)-5-(p-tolyl)-1, 4-dihydropyridin-3 -carboxylate

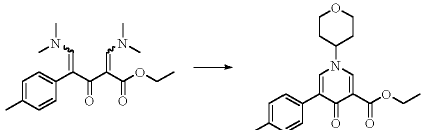

Ethyl 5-(dimethylamino)-2-((dimethylamino)methylene)-3-oxo-4-(*p*-tolyl) pent-4-enoate (crude) was dissolved in ethanol (50.0 mL), tetrahydro-2*H*- pyran-4-amine (1.998 g, 19.749 mmol, 1.5 eq) was added, and the reaction solution was warmed to 100 °C and reacted at reflux for 3 hrs. After the reaction was completed, as detected by TLC, the reaction solution was concentrated under reduced pressure to obtain a yellow oily product (crude).

### Step 6: synthesis of 4-oxo-1-(tetrahydro-2H-pyran-4-yl)-5-(p-tolyl)-1, 4-dihydropyridin-3-carboxylic acid

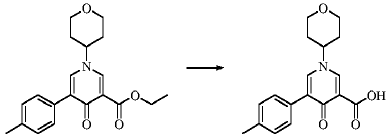

Ethyl 4-oxo-1-(tetrahydro-2*H*-pyran-4-yl)-5-(*p*-tolyl)-1,4-dihydropyridin- 3-carboxylate was dissolved in tetrahydrofuran (20.0 mL), then a 1 mol/L lithium hydroxide aqueous solution (10.0 mL) was added, and the reaction solution was stirred at room temperature for 2 hrs. After the reaction was completed, as detected by TLC, the reaction solution was concentrated under reduced pressure to remove tetrahydrofuran. The pH was adjusted to 4-5, and a large number of solids were precipitated. The mixture was filtered under vacuum, and the filter cake was slurried with ethyl acetate (3.0 mL), and was filtered under vacuum. The filter cake was dried to obtain a white solid product (1.6 g, three-step yield: 55%).

### Example 7: synthesis of intermediate 5-((6,7-dimethoxyquinolin-4-yl)oxy) pyrimidin-2-amine

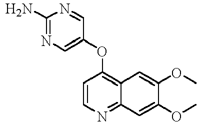

### Step 1: synthesis of tert-butyl (5-hydroxypyrimidin-2-yl)carbamate

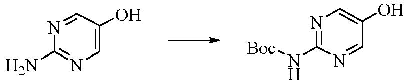

2-aminopyrimidin-5-ol (3.0 g, 27.0 mmol, 1.0 eq) was dissolved in tetrahydrofuran (25 mL), and triethylamine (2.7 g, 27.0 mmol, 1.0 eq) and di-*tert*-butyl dicarbonate (7.0 g, 32.0 mmol, 1.2 eq) were added under an ice bath. Then 4-dimethylaminopyridine (0.6 g, 5.4 mmol, 0.2 eq) was added in batches, and the reaction solution was gradually heated to room temperature and stirred overnight. After the reaction was completed, as detected by TLC, a saturated ammonium chloride aqueous solution (20 mL) and ethyl acetate (30 mL) were added to the system, and the liquid was separated. The organic phase was dried over anhydrous sodium sulfate and concentrated. The crude product was slurried with methyl *tert-butyl* ether (15 mL) for 2 hrs, filtered under vacuum and dried to obtain a product (4.4 g, yield: 77.1%).

### Step 2: synthesis of 5-((6,7-dimethoxyquinolin-4-yl)oxy)pyridin-2-amine

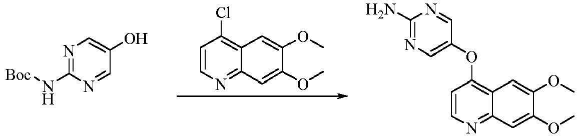

*Tert*-butyl (5-hydroxypyrimidin-2-yl)carbamate (4.4 g, 20.8 mmol, 1.0 eq) was dissolved in diphenyl ether (70 mL), then 4-chloro-6,7-dimethoxyquinoline (5.6 g, 24.9 mmol, 1.2 eq) and 4-dimethylaminopyridine (5.0 g, 41.6 mmol, 2.0 eq) were added, and the reaction solution was stirred overnight at 140 °C. After the reaction was completed, as detected by TLC, the reaction system was cooled to room temperature and poured into petroleum ether (200 mL). Solids were precipitated, filtered out under vacuum, and dried to obtain a product (2.4 g, yield: 32.2%).

### Example 8: synthesis of N-(5-((6,7-dimethoxyquinolin-4-yl)oxy)pyridin-2-yl)-4-oxo-1-((tetrahydro-2H-pyran-4-yl)methyl)-5-(p-tolyl)-1,4-dihydropyridazin-3-carboxamide (compound 2)

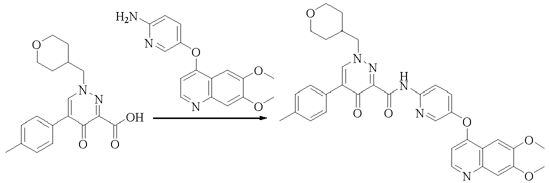

4-oxo-1-((tetrahydro-2*H*-pyran-4-yl)methyl)-5-(*p*-tolyl)-1,4-dihydropyridazin-3-carboxylic acid (150.0 mg, 0.457 mmol, 1.0 eq) and triethylamine (139.5 mg, 0.457 mmol, 3.0 eq) were dissolved in *N*, *N*-dimethylformamide (2.5 mL), and the reaction solution was cooled to 0 °C. HATU (261.0 mg, 0.685 mmol, 1.5 eq) was added, and the reaction solution was stirred for 0.5 hr under a nitrogen atmosphere. 5-((6,7-dimethoxyquinolin-4-yl)oxy)pyridin-2- amine (135.0 mg, 0.457 mmol, 1.0 eq) was added, and the reaction solution was slowly warmed to room temperature and stirred overnight. After the reaction was completed, as detected by TLC, the reaction solution was concentrated under reduced pressure to remove *N, N-*dimethylformamide. Ethyl acetate (30 mL) was added, and the resulting mixture was washed with water (3.0 mL × 4), dried and concentrated. The crude product was purified by silica gel column chromatography (DCM : MeOH = 150: 1 ∼ 40: 1) to obtain a product (120 mg, yield: 32.4%).

¹H NMR(400 MHz, DMSO-*d₆*) δ(ppm): 13.11 (s, 1H), 8.91 (s, 1H), 8.51-8.50 (d, 1H), 8.44-8.41 (m, 2H), 7.91-7.88 (d, 1H), 7.81-7.79 (d, 2H), 7.55 (s, 1H), 7.43 (s, 1H), 7.33-7.31 (d, 2H), 6.59-6.57 (d, 1H), 4.26-4.25 (d, 1H), 3.96-3.95 (d, 6H), 3.88-3.86 (d, 2H), 3.33-3.30 (m, 2H), 2.38 (s, 3H), 2.25-2.22 (m, 1H), 1.58-1.55 (m, 2H), 1.39-1.32 (m, 2H).

Molecular formula: C₃₄H₃₃N₅O₆ Molecular weight: 607.67 LC-MS(Pos, m/z)=608.39 [M+H]⁺.

### Example 9: synthesis of N-(5-((6,7-dimethoxyquinolin-4-yl)oxy)pyrimidin-2-yl)-4-oxo-1-((tetrahydro-2H-pyran-4-yl)methyl)-5-(p-tolyl)-1,4-dihydropyridin-3-carboxamide (compound 4)

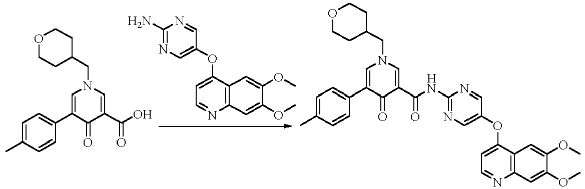

4-oxo-1-((tetrahydro-2*H*-pyran-4-yl)methyl)-5-(*p*-tolyl)-1,4-dihydropyridin-3-carboxylic acid (770.0 mg, 2.3 mmol, 1.0 eq) and 5-((6,7-dimethoxyquinolin-4-yl)oxy)pyrimidin-2-amine (701.7 mg, 2.3 mmol, 1.0 eq) were dissolved in pyridine (12 mL), and phosphorus oxychloride (36.0 mg, 0.2 mmol, 0.1 eq) was added dropwise. Then the reaction solution was stirred at room temperature for 2 hrs. After the reaction was completed, as monitored by TLC, the reaction system was concentrated, and the crude product was purified by preparative thin-layer chromatography (dichloromethane: methanol = 10: 1) to obtain a product (133.0 mg, yield: 9.5%).

¹HNMR (400 MHz,DMSO-*d₆*) δ(ppm): 13.71 (s, 1H), 8.80 (s, 2H), 8.70-8.71 (m, 1H), 8.51-8.52 (m, 1H), 8.17-8.18 (m, 1H), 7.59-7.61 (m, 2H), 7.56 (m, 1H),7.43 (s, 2H), 7.25-7.27 (m, 1H), 6.69-6.71 (s, 1H), 4.12-4.13 (m, 2H), 3.95-3.96 (s, 6H), 3.85-3.88 (m, 2H), 3.25-3.28 (m, 2H), 2.36 (s, 3H), 2.08-2.12 (s, 1H), 1.45-1.48 (m, 2H), 1.33-1.37 (m, 2H).

Molecular formula: C₃₄H₃₃N₅O₆ Molecular weight: 607.67 LC-MS(Pos, *m*/*z*)=608.1 [M+H]⁺.

### Example 10: synthesis of N-(5-((6,7-dimethoxyquinolin-4-yl)oxy)pyrimidin-2-yl)-4-oxo-1-((tetrahydro-2H-pyran-4-yl)methyl)-5-(p-tolyl)-1,4-dihydropyridazin-3-carboxamide (compound 16)

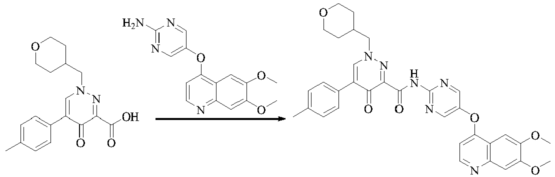

4-oxo-1-((tetrahydro-2*H*-pyran-4-yl)methyl)-5-(*p*-tolyl)-1,4-dihydropyridazin-3-carboxylic acid (507.4 mg, 1.5 mmol, 1.0 eq) and 5-((6,7- dimethoxyquinolin-4-yl)oxy)pyrimidin-2-amine (460.9 mg, 1.5 mmol, 1.0 eq) were dissolved in pyridine (6 mL), and phosphorus oxychloride (36.0 mg, 0.2 mmol, 0.1 eq) was added dropwise. Then the reaction solution was stirred at room temperature for 2 hrs. After the reaction was completed, as monitored by TLC, the reaction system was concentrated and purified by preparative thin-layer chromatography (dichloromethane: methanol = 10:1) to obtain a product (109.8 mg, yield: 12.0%).

¹HNMR (400 MHz, DMSO-*d₆*) δ(ppm): 12.97 (s, 1H), 8.86 (m, 1H), 8.79 (m, 2H), 8.49-8.51 (m, 1H), 7.77-7.79 (m, 2H), 7.54 (s, 1H), 7.43 (s, 1H), 7.29-7.31 (m, 2H), 6.67-6.68 (m, 1H), 4.21-4.22 (m, 2H), 3.95-3.96 (m, 6H), 3.84-3.88 (m, 2H), 3.25-3.28 (m, 2H), 2.37 (s, 3H), 2.18 (s, 1H), 1.50-1.53 (m, 2H), 1.32-1.38 (m, 2H).

Molecular formula: C₃₃H₃₂N₆O₆ Molecular weight: 608.66 LC-MS(Pos, *m*/*z)* =609. 1[M+H]⁺.

### Example 11: synthesis of N-(5-((6,7-dimethoxyquinolin-4-yl)oxy)pyridin-2-yl)-4-oxo-1-(tetrahydro-2H-pyran-4-yl)-5-(p-tolyl)-1,4-dihydropyridazin-3-carboxamide (compound 17)

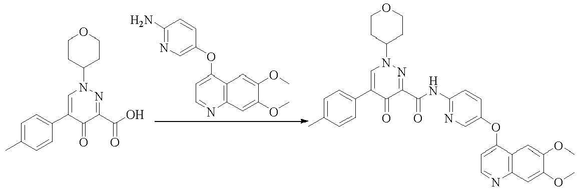

4-oxo-1-(tetrahydro-2*H*-pyran-4-yl)-5-(*p*-tolyl)-1,4-dihydropyridazin-3-carboxylic acid (300.0 mg, 0.954 mmol, 1.0 eq) and 5-((6,7-dimethoxyquinolin -4-yl)oxy)pyridin-2-amine (284.0 mg, 0.954 mmol, 1.0 eq) were dissolved in pyridine (2.5 mL), then phosphorus oxychloride (0.5 mL) was added, and the reaction solution reacted overnight at room temperature. After the reaction was completed, as detected by TLC, water and ethyl acetate were added, and the liquid was separated. The organic phase was dried and concentrated under reduced pressure. The crude product was first separated by preparative thin-layer chromatography (DCM MeOH = 13:1), and then purified by silica gel column chromatography (DCM : MeOH = 40:1 ∼ 12:1) to obtain a white solid product (130.0 mg, yield: 43.3%).

¹H NMR(400 MHz, DMSO-*d₆*) δ(ppm): 13.69 (s, 1H), 8.81 (s, 1H), 8.75-8.74 (d, 1H), 8.52-8.51 (d, 2H), 8.24-8.23 (d, 1H), 7.63-7.61 (d, 2H), 7.56 (s, 1H), 7.43 (s, 1H), 7.27-7.25 (d, 2H), 6.72-6.70 (d, 1H), 4.61-4.55 (d, 1H), 4.05-4.02 (d, 2H), 3.97-3.96 (d, 6H), 3.48-3.43 (m, 2H), 2.37 (s, 3H), 2.14-2.10 (m, 4H).

Molecular formula: C₃₃H₃₁N₅O₆ Molecular weight: 593.64 LC-MS(Pos, *m*/*z*)=594.2 [M+H]⁺.

### Example 12: synthesis of N-(5-((6,7-dimethoxyquinolin-4-yl)oxy)pyrimidin-2-yl)-4-oxo-1-(tetrahydro-2H-pyran-4-yl)-5-(p-tolyl)-1,4-dihydropyridazin-3-carboxamide (compound 18)

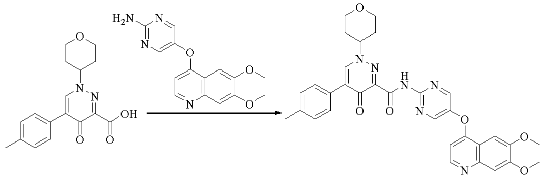

4-oxo-1-(tetrahydro-2*H*-pyran-4-yl)-5-(*p*-tolyl)-1,4-dihydropyndazin-3-carboxylic acid (300.0 mg, 0.954 mmol, 1.0 eq) and 5-((6,7-dimethoxyquinolin -4-yl)oxy)pyrimidin-2-amine (256.0 mg, 0.859 mmol, 0.9 eq) were dissolved in pyridine (3.0 mL), then phosphorus oxychloride (0.75 mL) was added, and the reaction solution was stirred overnight at room temperature. The reaction was not completed, as detected by TLC, and phosphorus oxychloride (0.75 mL) was supplemented. 4 hrs later, the reaction was still not completed, as detected by TLC, and phosphorus oxychloride (0.75 mL) was supplemented again. 0.5 hr later, water was added, and ethyl acetate was added for extraction. The organic phase was dried and concentrated under reduced pressure. The crude product was first separated by preparative thin-layer chromatography (DCM: MeOH = 15:1), and then purified by silica gel column chromatography (DCM: MeOH = 60:1 ∼ 20:1) to obtain a white solid product (50.0 mg, yield: 16.7%).

¹H NMR (400 MHz, DMSO-*d*₆) δ(ppm): 12.99 (s, 1H), 8.88 (s, 1H), 8.81 (s, 2H), 8.52-8.50 (d, 1H), 7.82-7.79 (d, 2H), 7.55 (s, 1H), 7.43 (s, 1H), 7.31-7.29 (d, 2H), 6.70-6.69 (d, 1H), 4.71-4.64 (m, 1H), 4.07-4.03 (d, 2H), 3.96-3.95 (d, 6H), 3.50-3.45 (m, 2H), 2.37 (s, 3H), 2.13-2.03 (m, 4H).

Molecular formula: C₃₂H₃₀N₆O₆ Molecular weight: 594.63 LC-MS(Pos, *m*/*z*)=595.38 [M+H]⁺.

### Example 13: synthesis of N-(5-((6,7-dimethoxyquinolin-4-yl)oxy)pyridin -2-yl)-4-oxo-1-(tetrahydro-2H-pyran-4-yl)-5-(p-tolyl)-1,4-dihydropyridin-3 -carboxamide (compound 19)

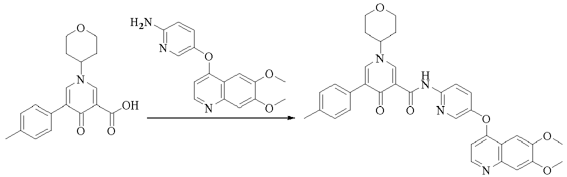

4-oxo-1-(tetrahydro-2*H*-pyran-4-yl)-5-(*p*-tolyl)-1,4-dihydropyridin-3 -carboxylic acid (160.0 mg, 0.511 mmol, 1.0 eq) and triethylamine (155.0 mg, 1.533 mmol, 3.0 eq) were dissolved in N, *N*-dimethylformamide (2.0 mL), and the mixture solution was cooled to 0 °C. HATU (291.0 mg, 0.766 mmol, 1.5 eq) was added under a nitrogen atmosphere, and the reaction solution was stirred at a low temperature for 0.5 hr. 5-((6,7-dimethoxyquinolin-4-yl)oxy) pyridin-2-amine (152.0 mg, 0.511 mmol, 1.0 eq) was added, and the resulting reaction solution was slowly warmed to room temperature and reacted overnight. After the reaction was completed, as detected by TLC, the reaction solution was concentrated under reduced pressure. Ethyl acetate was added, and the resulting mixture was washed twice with a saturated sodium bicarbonate aqueous solution, and then washed four times with water. The resulting solution was dried and concentrated under reduced pressure. The crude product was first separated by preparative thin-layer chromatography (DCM: MeOH = 20:1), and then purified by silica gel column chromatography (DCM: MeOH = 80:1 ∼ 30:1) to obtain a yellow solid product (113.0 mg, yield: 70.6%).

¹H NMR(400 MHz, DMSO-*d*₆) δ(ppm): 13.44 (s, 1H), 8.78-8.77 (d, 1H), 8.51-8.38 (m, 3H), 8.24-8.23 (d, 2H), 7.88-7.85 (m, 1H), 7.63-7.61 (d, 2H), 7.55 (s, 1H), 7.42 (s, 1H), 7.27-7.25 (d, 2H), 6.58-6.56 (d, 1H), 4.57-4.53 (m, 1H), 4.05-4.01 (d, 2H), 3.96-3.95 (d, 6H), 3.47-3.42 (m, 2H), 2.37 (s, 1H), 2.14-2.10 (m, 2H), 2.02-2.00 (m, 2H).

Molecular formula: C₃₄H₃₂N₄O₆ Molecular weight: 592.65 LC-MS (Pos, *m*/*z*)=593.28 [M+H]⁺.

### Example 14: synthesis of N-(5-((6,7-dimethoxyquinolin-4-yl)oxy)pyrimidin -2-yl)-4-oxo-1-(tetrahydro-2H-pyran-4-yl)-5-(p-tolyl)-1,4-dihydropyridin-3-carboxamide (compound 20)

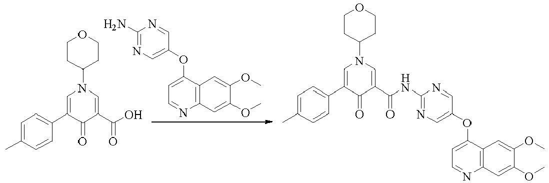

4-oxo-1-(tetrahydro-2*H*-pyran-4-yl)-5-(*p*-tolyl)-1,4-dihydropyridin-3 -carboxylic acid (300.0 mg, 0.957 mmol, 1.0 eq) and 5-((6,7-dimethoxyquinolin -4-yl)oxy)pyrimidin-2-amine (285.6 mg, 0.957 mmol, 1.0 eq) were dissolved in pyridine (2.5 mL), and phosphorus oxychloride (0.5 mL) was added dropwise. 2 hrs later, the reaction was not completed, as detected by TLC, and phosphorus oxychloride (0.1 mL) was supplemented. The resulting reaction solution reacted overnight at room temperature. After the reaction was completed, as detected by TLC, ethyl acetate (30 mL) was added. The resulting mixture was washed with 1 mol/L hydrochloric acid (3.0 mL × 2), dried and concentrated. The crude product was first separated by preparative thin-layer chromatography (DCM: MeOH = 17:1), and then purified by silica gel column chromatography (DCM: MeOH = 20:1 ∼ 15:1) to obtain a white solid product (80.0 mg, yield: 26.7%).

¹H NMR(400 MHz, DMSO-*d*₆) δ(ppm): 13.12 (s, 1H), 8.92 (s, 1H), 8.52-8.50 (d, 1H), 8.45-8.42 (m, 2H), 7.91-7.90 (d, 1H), 7.89-7.81 (m, 2H), 7.55 (s, 1H), 7.43 (s, 1H), 7.33-7.31 (d, 2H), 6.59-6.58 (d, 1H), 4.72-4.66 (m, 1H), 4.08-4.04 (d, 2H), 3.96-3.95 (d, 6H), 3.51-3.46 (m, 2H), 2.38 (s, 1H), 2.18-2.16 (m, 4H).

Molecular formula: C₃₃H₃₁N₅O₆ Molecular weight: 593.64 LC-MS(Pos, *m*/*z*)=594.2 [M+H]⁺.

### Example 15: synthesis of N-(5-((6,7-dimethoxyquinolin-4-yl)oxy)pyridin-2-yl)-4-oxo-1-((tetrahydro-2H-thiopyran-4-yl)methyl)-5-(p-tolyl)-1,4-dihydropyridazin-3-carboxamide (compound 21)

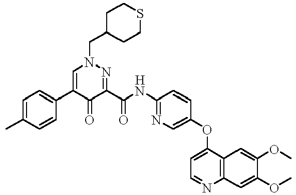

### Step 1: synthesis of ethyl 4-oxo-1-((tetrahydro-2H-thiopyran-4-yl)methyl)-5-(p-tolyl)-1,4-dihydropyridazin-3 -carboxylate

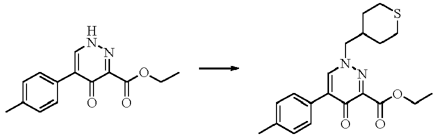

Ethyl 4-oxo-5-(*p*-tolyl)-1,4-dihydropyridazin-3-carboxylate (426.07 mg, 1.65 mmol), 4-(bromomethyl)tetrahydro-2*H*-thiopyran (320.0 mg, 1.65 mmol) and K₂CO₃ (684.14 mg, 4.95 mmol) were added to DMF (5 mL), and the reaction solution was stirred overnight at room temperature. After the reaction was completed, as monitored by TLC, the reaction solution was concentrated under reduced pressure. The residue was added with ethyl acetate and filtered, and the filtrate was concentrated under reduced pressure. The crude product was first purified by silica gel column chromatography (PE : EA = 10:1 ∼ 2:1), and then recrystallized with methyl *tert-butyl* ether/dichloromethane (10/1) to obtain a product (300 mg, yield: 48.9%).

### Step 2: synthesis of 4-oxo-1-((tetrahydro-2H-thlopyran-4-yl)methyl)-5-(p-tolyl)-1,4-dihydropyridazin-3-carboxylic acid

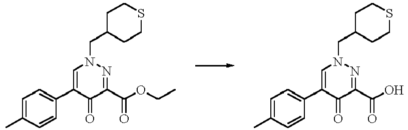

Ethyl 4-oxo-1-((tetrahydro-2*H*-thiopyran-4-yl)methyl)-5-(*p*-tolyl)-1,4-dihydropyridazin-3-carboxylate (300.0 mg, 0.81 mmol) was added to a flask, then tetrahydrofuran (4 mL) and a lithium hydroxide aqueous solution (1 mol/L, 4 mL) were added, and the reaction solution was stirred at room temperature for 3 hrs. After the reaction was completed, as monitored by TLC, the reaction solution was concentrated under reduced pressure, and pH was adjusted to 1-2. Solids were precipitated in the aqueous phase, and filtered out under vacuum to obtain a product (290 mg crude).

### Step 3: synthesis of N-(5-((6,7-dimethoxyquinolin-4-yl)oxy)pyridin-2-yl)-4-oxo-1-((tetrahydro-2H-thiopyran-4-yl)methyl)-5-(p-tolyl)-1,4-dihydropyridazin-3-carboxamide

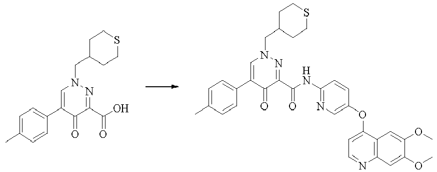

4-oxo-1-((tetrahydro-2*H*-thiopyran-4-yl)methyl)-5-(p-tolyl)-1,4-dihydropyridazin-3-carboxylic acid (60 mg, 0.174 mmol) and 5-((6,7-dimethoxyquinolin -4-yl)oxy)pyridin-2-amine (51.8 mg, 0.174 mmol) were added to a flask, and pyridine (2 mL) was added. Then phosphorus oxychloride (0.5 mL) was added dropwise, and the reaction solution reacted at room temperature for 5 min. After the reaction was completed, as monitored by TLC, water was added, and ethyl acetate was added for extraction. The organic phases were combined, washed with water, and then washed with saturated brine. The resulting solution was dried over anhydrous sodium sulfate, filtered and concentrated. The crude product was purified by silica gel column chromatography (DCM: MeOH = 100:1 ∼ 40:1) to obtain a product (28.0 mg, yield: 25.8 %).

¹H NMR (400 MHz, DMSO-*d*₆) δ(ppm): 13.14 (s, 1H), 8.96 (s, 1H), 8.52-8.51 (d, 1H), 8.44-8.42 (t, 2H), 7.91-7.90 (d, 1H), 7.89-7.79 (m, 2H), 7.56 (s, 1H), 7.43 (s, 1H), 7.33-7.31 (d, 2H), 6.60-6.59 (d, 1H), 4.43-4.39 (t, 2H), 3.96-3.95 (d, 6H), 2.97-2.93 (m, 1H), 2.84-2.77 (m, 2H), 2.65-2.61 (m, 1H), 2.38 (s, 3H), 2.18-2.14 (m, 2H), 2.07-2.01 (m, 2H), 1.24 (s, 1H).

Molecular formula: C₃₄H₃₃N₅O₅S Molecular weight: 623.22 LC-MS(Pos, *m*/*z)=* 624.0[M+H]⁺.

### Example 16: synthesis of N-(5-((6,7-dimethoxyquinolin-4-yl)oxy)pyrimidin -2-yl)-4-oxo-1-((tetrahydro-2H-thiopyran-4-yl)methyl)-5-(p-tolyl)-1,4-dihydropyridin-3-carboxamide (compound 22)

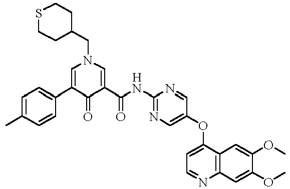

### Step 1: synthesis of ethyl 5-(dimethylamino)-2-((dimethylamino)methylene) -3-oxo-4-(p-tolyl)pentan-4-enoate

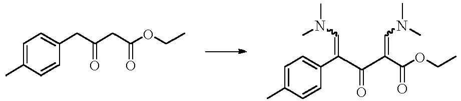

Ethyl 3-oxo-4-(*p*-tolyl)butyrate (5.0 g, 22.7 mmol) was added to a flask, then toluene (75 mL) and DMF-DMA (8.1 g, 68.1 mmol) were added, and the reaction solution was heated to reflux overnight. After the reaction was completed, as monitored by TLC, the reaction solution was concentrated under reduced pressure to obtain a product (8.0 g crude).

### Step 2: synthesis of ethyl 4-oxo-1-((tetrahydro-2H-thiopyran-4-yl)methyl)-5-(p-tolyl)-1,4-dihydropyridin-3 -carboxylate

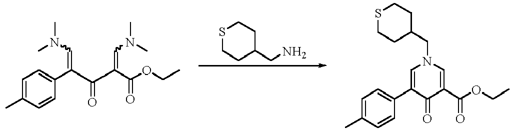

Ethyl 5-(dimethylamino)-2-((dimethylamino)methylene)-3-oxo-4-(*p*-tolyl) pentan-4-enoate (3.3 g crude) was added to a flask, then (tetrahydro-2*H*-thiopyran-4-yl)methylamine (2.6 g, 19.99 mmol) and ethanol (6 mL) were added, and the reaction solution was heated to reflux for 3 hrs. After the reaction was completed, as monitored by TLC, the reaction solution was concentrated under reduced pressure to obtain a product (3.3 g crude).

### Step 3: synthesis of 4-oxo-1-((tetrahydro-2H-thiopyran-4-yl)methyl)-5-(p-tolyl)-1,4-dihydropyridin-3-carboxylic acid

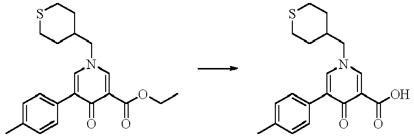

Ethyl 4-oxo-1-((tetrahydro-2*H*-thiopyran-4-yl)methyl)-5-(*p*-tolyl)-1,4- dihydropyridin-3-carboxylate (3.3 g crude) was added to a flask, then tetrahydrofuran (10 mL) and a LiOH aqueous solution (1 mol/L, 10 mL) were added, and the reaction solution was stirred overnight at room temperature. After the reaction was completed, as monitored by TLC, the reaction solution was concentrated under reduced pressure, and pH was adjusted to 1-2 with HCl (1 mol/L). Solids were precipitated and filtered out under vacuum. DCM was added to the filtrate for extraction, and the organic phases were combined, dried over anhydrous sodium sulfate, and concentrated under reduced pressure. The crude product was combined with the filter cake, and slurried with ethyl acetate to obtain a product (1.5 g, three-step yield: 46.7%).

### Step 4: synthesis of N-(5-((6,7-dimethoxyquinolin-4-yl)oxy)pyrimidin-2-yl) -4-oxo-1-((tetrahydro-2H-thiopyran-4-yl)methyl)-5-(p-tolyl)-1,4-dihydropyridin-3-carboxamide

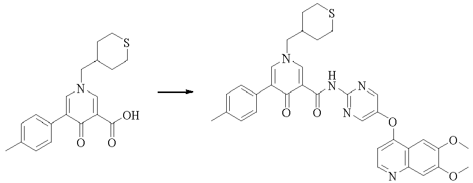

4-oxo-1-((tetrahydro-2*H*-thiopyran-4-yl)methyl)-5-(*p*-tolyl)-1,4-dihydropyridin-3-carboxylic acid (450 mg, 1.31 mmol) and 5-((6,7-dimethoxyquinolin -4-yl)oxy)pyrimidin-2-amine (390.8 mg, 1.31 mmol) were added to a flask, and pyridine (5 mL) was added. Then phosphorus oxychloride (0.25 mL) was added dropwise, and the reaction solution reacted at room temperature for 5 min. After the reaction was completed, as monitored by TLC, water was added, and ethyl acetate was added for extraction. The organic phases were combined, washed with water, and then washed with saturated brine. The resulting solution was dried over anhydrous sodium sulfate, filtered and concentrated. The crude product was purified by silica gel column chromatography (DCM: MeOH = 100:1 ∼ 40:1) to obtain a product (130.0 mg, yield: 15.9%).

¹H NMR (400 MHz, DMSO-*d*₆) δ(ppm): 13.71 (s, 1H), 8.80 (s, 1H), 8.69-8.68 (d, 1H), 8.52-8.51 (d, 1H), 8.17-8.16 (d, 2H), 7.61-7.56 (t, 2H), 7.43 (s, 1H), 7.27-7.25 (d, 2H), 6.71-6.70 (d, 1H), 4.12-4.10 (d, 2H), 3.97-3.96 (d, 6H), 2.67-2.61 (t, 6H), 2.36 (s, 3H), 1.94-1.86 (t, 3H), 1.25 (s, 1H).

Molecular formula: C₃₄H₃₃N₅O₅S Molecular weight: 623.22 LC-MS(Pos, *m*/*z)=* 624.4[M+H]⁺.

### Example 17: synthesis of N-(5-((6,7-dimethoxyquinolin-4-yl)oxy)pyrimidin -2-yl)-4-oxo-1-((tetrahydro-2H-thiopyran-4-yl)methyl)-5-(p-tolyl)-1,4-dihydropyridazin-3-carboxamide (compound 23)

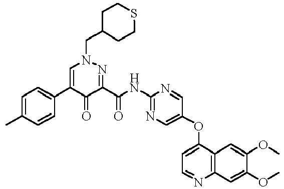

### Step 1: synthesis of N-(5-((6,7-dimethoxyquinolin-4-yl)oxy)pyrimidin-2-yl)-4-oxo-1-((tetrahydro-2H-thiopyran-4-yl)methyl)-5-(p-tolyl)-1,4-dihydropyridazin-3-carboxamide

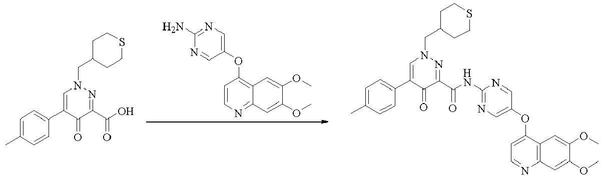

4-oxo-1-((tetrahydro-2*H*-thiopyran-4-yl)methyl)-5-(*p*-tolyl)-1,4-dihydropyridazin-3-carboxylic acid (78.8 mg, 0.22 mmol, 1.0 eq) and 5-((6,7- dimethoxyquinolin-4-yl)oxy)pyrimidin-2-amine (68.2 mg, 0.22 mmol, 1.0 eq) were added to pyridine (1.5 mL), then phosphorus oxychloride (0.1 mL) was slowly added dropwise, and the reaction solution was stirred for 3 min. After the reaction was completed, as detected by TLC, ethyl acetate (10 mL) and water (20 mL) were added, and the liquid was separated. The organic phase was dried over anhydrous sodium sulfate and filtered under vacuum, and the filtrate was concentrated under reduced pressure. The crude product was purified by preparative thin-layer chromatography (dichloromethane: methanol = 15:1) to obtain a product (9.45 mg, yield: 6.7%).

¹H NMR (400MHz, DMSO-*d*₆) δ(ppm): 8.92 (s, 1H), 8.79 (m, 2H), 8.51 (m, 1H), 7.29-7.31 (m, 2H), 7.43 (s, 1H), 7.55 (s, 1H), 7.76-7.80 (m, 2H), 6.68-6.69 (s, 1H), 4.36-4.40 (m, 2H), 3.95-3.96 (m, 6H), 2.91-2.95 (m, 1H), 2.72-2.85 (m, 2H), 2.37(s, 3H), 2.12-2.16 (m, 2H), 1.98-2.06 (m, 2H), 1.56-1.61 (m, 2H).

Molecular formula: C₃₃H₃₂N₆O₅S Molecular weight: 624.72 LC-MS (Pos,m/z)=625.3 [M+H]⁺.

### Example 18: synthesis of N-(6-((6,7-dimethoxyquinolin-4-yl)oxy)pyridazin-3-yl)-4-oxo-1-((tetrahydro-2H-pyran-4-yl)methyl)-5-(p-tolyl)-1,4-dihydropyridin-3-carboxamide (compound 24)

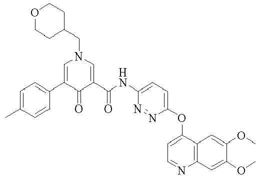

### Steps

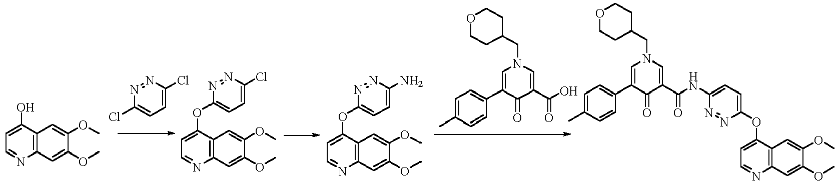

### Step 1: synthesis of 4-((6-chloropyridazin-3-yl)oxy)-6,7-dimethoxyquinoline

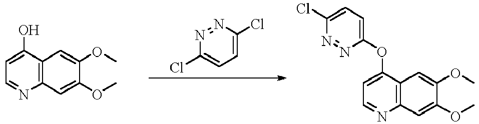

6,7-dimethoxyquinolin-4-ol (2.0 g, 9.75 mmol, 1.0 eq) was dissolved in *N, N-*dimethylformamide (10.0 mL), then potassium carbonate (4.04 g, 29.34 mmol, 3.0 eq) and 3,6-dichloropyridazine (1.74 g, 11.70 mmol, 1.2 eq) were added, and the reaction solution reacted overnight at 120 °C under a nitrogen atmosphere. After the reaction was completed, as detected by TLC, the reaction solution was filtered. The filter cake was washed with ethyl acetate, and the filtrate was concentrated under reduced pressure and added with ethyl acetate (100.0 mL) for extraction. The organic phase was washed with water (4.0 mL × 4), dried and concentrated. The crude product was purified by silica gel column chromatography (DCM: MeOH = 150:1 ∼ 25:1) to obtain a product (694.0 mg, yield: 22.4%).

### Step 2: synthesis of 6-((6,7-dimethoxyquinolin-4-yl)oxy)pyridazin-3-amine

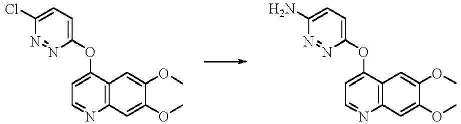

4-((6-chloropyridazin-3-yl)oxy)-6,7-dimethoxyquinoline (1.0 g, 3.147 mmol, 1.0 eq) was dissolved in aqueous ammonia (15.0 mL), and the reaction solution was sealed in a tube and reacted overnight at 130 °C. After the reaction was completed, as detected by TLC, the reaction solution was concentrated under reduced pressure. The crude product was first purified by silica gel column chromatography (DCM: MeOH = 150: 1 ∼ 30: 1), and then purified by preparative thin-layer chromatography (DCM : MeOH = 18: 1) to obtain a product (50.0 mg, yield: 5.3%).

### Step 3: synthesis of N-(6-((6,7-dimethoxyquinolin-4-yl)oxy)pyridazin-3-yl)-4-oxo-1-((tetrahydro-2H-pyran-4-yl)methyl)-5-(p-tolyl)-1,4-dihydropyridin-3-carboxamide

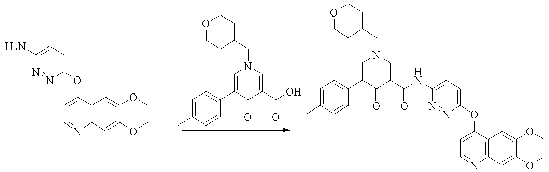

4-oxo-1-((tetrahydro-2*H*-pyran-4-yl)methyl)-5-(*p*-tolyl)-1,4-dihydropyridin-3-carboxylic acid (66.0 mg, 0.203 mmol, 1.1 eq) was dissolved in DMF (2.0 mL), then triethylamine (55.9 mg, 0.552 mmol, 3.0 eq) and HATU (105.0 mg, 0.276 mmol, 1.5 eq) were added at 0 °C under a nitrogen atmosphere, and the mixture was stirred at 0 °C for 1 hr. 6-((6,7-dimethoxyquinolin-4-yl)oxy) pyridazin-3-amine (55.0 mg, 0.184 mmol, 1.0 eq) was added, and the reaction solution reacted overnight at room temperature. After the reaction was completed, as detected by TLC, the reaction solution was concentrated under reduced pressure. Ethyl acetate (20 mL) was added, and the resulting mixture was washed with water (2.0 mL × 4), dried over anhydrous sodium sulfate, and concentrated under reduced pressure. The crude product was separated by preparative thin-layer chromatography (DCM: MeOH = 10:1) to obtain a product (33.0 mg, yield: 26.7%).

¹H NMR(400 MHz, DMSO-*d*₆) δ(ppm): 13.86 (s, 1H), 8.77-8.76 (m, 2H), 8.71-8.62 (d, 1H), 8.20 (d, 1H), 7.83-7.80 (d, 1H), 7.60-7.59 (m, 2H), 7.46-7.42 (m, 2H), 7.27-7.25 (m, 2H), 7.02-7.00 (d, 1H), 4.13-4.12 (m, 2H), 3.96-3.85 (m, 6H), 3.27-3.25 (m, 3H), 3.36-2.33 (m, 3H), 2.20-2.10 (m, 2H), 1.48-1.24 (m, 4H).

Molecular formula: C₃₄H₃₃N₅O₆ Molecular weight: 607.67 LC-MS(Pos, *m*/*z*)=608.31 [M+H]⁺.

### Example 19: synthesis of N-(5-((6,7-dimethoxyquinolin-4-yl)oxy)pyridin -2-yl)-4-oxo-1-((tetrahydro-2H-pyran-4-yl)methyl)-1,4-dihydroquinolin-3-carboxamide (compound 25)

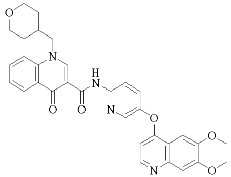

### Steps

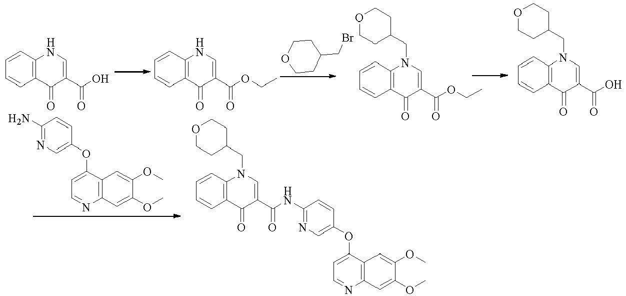

### Step 1: synthesis of ethyl 4-oxo-1,4-dihydroquinolin-3-carboxylate

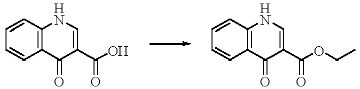

4-oxo-1,4-dihydroquinolin-3-carboxylic acid (1.08 g, 5.7 mmol, 1.0 eq) was dissolved in ethanol (15 mL), then thionyl chloride (6.8 g, 57 mmol, 10.0 eq.) was added dropwise with stirring, and the reaction solution reacted overnight at 85 °C. After the reaction was completed, as monitored by TLC, the reaction solution was concentrated under reduced pressure. The crude product was purified by silica gel column chromatography (dichloromethane : methanol = 60:1 ∼ 40:1) to obtain a product (901.3 mg, yield: 72.8%).

### Step 2: synthesis of ethyl 4-oxo-1-((tetrahydro-2H-pyran-4-yl)methyl) -1,4-dihydroquinolin-3 -carboxylate

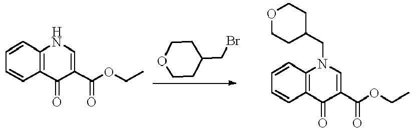

Ethyl 4-oxo-1,4-dihydroquinolin-3-carboxylate (0.7 g, 3.22 mmol, 1.0 eq) was dissolved in DMF (4.5 mL), then 4-(bromomethyl)tetrahydro-2*H*-pyran (1.73 g, 9.66 mmol, 3.0 eq) and potassium carbonate (1.34 g, 9.66 mmol, 3.0 eq) were added, and the reaction solution was stirred overnight at 80 °C under a nitrogen atmosphere. After the reaction was completed, as monitored by TLC, the reaction solution was concentrated under reduced pressure. Ethyl acetate (15 mL) and water (20 mL) were added, and the liquid was separated. The organic phase was concentrated, and the crude product was purified by silica gel column chromatography (dichloromethane: methanol = 50:1 ∼ 30:1) to obtain a product (550.3 mg, yield: 54.2%).

### Step 3: synthesis of 4-oxo-1-((tetrahydro-2H-pyran-4-yl)methyl)-1,4-dihydroquinolin - 3-carboxylic acid

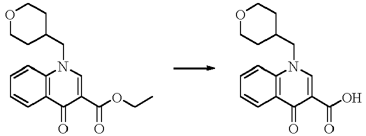

Ethyl 4-oxo-1-((tetrahydro-2*H*-pyran-4-yl)methyl)-1,4-dihydroquinolin-3-carboxylate (550.3 mg, 1.74 mmol, 1.0 eq) was dissolved in tetrahydrofuran (5.0 mL), then a lithium hydroxide aqueous solution (1 mol/L, 5.0 mL) was added, and the reaction solution was stirred at room temperature for 1 hr. After the reaction was completed, as monitored by TLC, the reaction solution was concentrated under reduced pressure, and pH was adjusted to 4 with hydrochloric acid (1 mol/L, 2.0 mL). The concentrate was stirred for 20 min and filtered under vacuum, and the filter cake was dried to obtain a product (380.1 mg, yield: 76.0%).

### Step 4: synthesis of N-(5-((6,7-dimethoxyquinolin-4-yl)oxy)pyridin-2-yl) -4-oxo-1-((tetrahydro-2H-pyran-4-yl)methyl)-1,4-dihydroquinolin-3-carboxamide

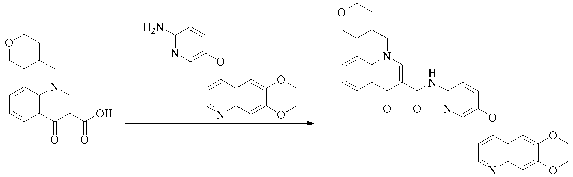

4-oxo-1-((tetrahydro-2*H*-pyran-4-yl)methyl)-1,4-dihydroquinolin-3 -carboxylic acid (380.3 mg, 1.32 mmol, 1.0 eq.) was dissolved in a mixture solution of tetrahydrofuran (4 mL), DMF (4 mL) and acetonitrile (4 mL), and the resulting solution was cooled to 0 °C under an ice bath. DIPEA (513.1 mg, 3.97 mmol, 3.0 eq) and HATU (553.3 mg, 1.45 mmol, 1.1 eq) were added, and the reaction solution was stirred at 0 °C for 1 hr. 5-((6,7- dimethoxyquinolin-4-yl)oxy)pyridin-2-amine (432.5 mg, 1.45 mmol, 1.1 eq) was added, and the resulting reaction solution was stirred overnight under a nitrogen atmosphere. After the reaction was completed, as monitored by TLC, the reaction solution was concentrated under reduced pressure. Dichloromethane (20 mL) and water (15 mL) were added, and the liquid was separated. The organic phase was dried over anhydrous sodium sulfate and concentrated. The crude product was purified by silica gel column chromatography (dichloromethane: methanol = 80:1 ∼ 50:1) to obtain a product (160.0 mg, yield: 21.4%).

¹H NMR (400 MHz, DMSO-*d*₆) δ(ppm): 12.92 (s, 1H), 9.02 (s, 1H), 8.39-8.51 (m, 4H), 8.03-8.06 (m, 1H), 7.85-7.93 (m, 1H), 7.60-7.64 (m, 1H), 7.55 (s, 1H), 7.41 (s, 1H), 6.56-6.57 (s, 1H), 4.47-4.49 (m, 2H), 3.95 (s, 6H), 3.82-3.85 (m, 2H), 3.18-3.24 (m, 3H), 2.13 (s, 1H), 1.36-1.48 (m, 4H).

Molecular formula: C₃₂H₃₀N₄O₆ Molecular weight: 566.61 LC-MS(Pos, *m*/*z*)=567.4[M+H]⁺.

### Example 20: synthesis of N-(5-((6,7-dimethoxyquinolin-4-yl)oxy)pyrimidin -2-yl)-5-(4-fluorophenyl)-4-oxo-1-(tetrahydro-2H-pyran-4-yl)-1,4-dihydropyridin-3 -carboxamide (compound 31)

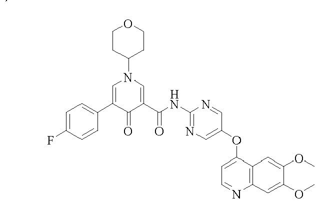

### Step 1: synthesis of N-(5-((6,7-dimethoxyquinolin-4-yl)oxy)pyrimidin- 2-yl)-5-(4-fluorophenyl)-4-oxo-1-(tetrahydro-2H-pyran-4-yl)-1,4-dihydropyridin-3-carboxamide

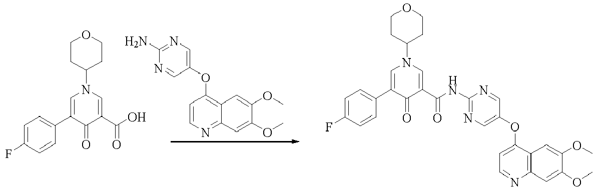

5-(4-fluorophenyl)-4-oxo-1-(tetrahydro-2*H*-pyran-4-yl)-1,4-dihydropyridin-3-carboxylic acid (300.0 mg, 0.945 mmol, 1.0 eq) and 5-((6,7- dimethoxyquinolin-4-yl)oxy)pyrimidin-2-amine (282.0 mg, 0.945 mmol, 1.0 eq) were dissolved in pyridine (2.0 mL), and the resulting solution was cooled to 0 °C. Phosphorus oxychloride (0.5 mL) was added dropwise under a nitrogen atmosphere, and the reaction solution was slowly warmed to room temperature. 1 hr later, the reaction was completed, as detected by TLC. A saturated sodium bicarbonate aqueous solution was added, and ethyl acetate was added for extraction. The organic phase was washed with 1 mol/L hydrochloric acid (2.0 mL × 2), dried over anhydrous sodium sulfate, and concentrated under reduced pressure. The crude product was first purified by preparative thin-layer chromatography (DCM: MeOH = 20:1), and then purified by silica gel column chromatography (DCM: MeOH = 80:1 ∼ 20:1) to obtain a product (190.0 mg, yield: 33.6%).

¹H NMR (400 MHz, DMSO-*d*₆) δ(ppm): 13.63(s, 1H), 8.81(s, 2H), 8.77-8.76(d, 1H), 8.52-8.51(d, 1H), 8.31-8.30(d, 1H), 7.79-7.75(m, 2H), 7.50(s, 1H), 7.43(s, 1H), 7.31-7.26(m, 2H), 6.71-6.70(d, 1H), 4.60-4.55(m, 1H), 4.05-4.01(m, 2H), 3.96-3.95(m, 6H), 3.45-3.42(m, 2H), 2.16-2.00(m, 4H).

Molecular formula: C₃₂H₂₈FN₅O₆ Molecular weight: 597.60 LC-MS(Pos, *m*/*z*)=598.38 [M+H]⁺.

### Example 21: synthesis of N-(5-((6,7-dimethoxyquinolin-4-yl)oxy)pyridin-2-yl)-1-(4-fluorophenyl)-2-oxo-5-((tetrahydro-2H-pyran-4-yl)methyl)-1,2-dihydropyridin-3-carboxamide (compound 32)

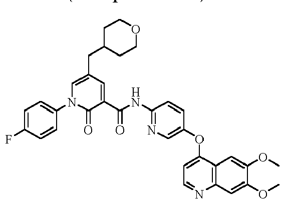

### Step 1: Synthesis of 1-(4-fluorophenyl)-2-oxo-5-((tetrahydro-2H-pyran-4-yl)methyl)-1,2-dihydropyridin-3 -carboxylic acid

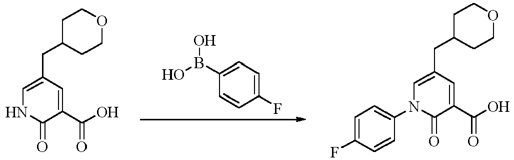

2-oxo-5-((tetrahydro-2*H*-pyran-4-yl)methyl)-1,2-dihydropyridin-3 -carboxylic acid (350.0 mg, 1.48 mmol, 1.0 eq), (4-fluorophenyl)boronic acid (619.3 mg, 4.43 mmol, 3.0 eq), pyridine (933.4 mg, 11.80 mmol, 8.0 eq) and triethylamine (746.3 mg, 7.38 mmol, 5.0 eq) were dissolved in dichloromethane (20.0 mL), then copper acetate (589.0 mg, 2.95 mmol, 2.0 eq) was added, and the reaction solution was stirred overnight at room temperature. After the reaction was completed, as detected by TLC, the reaction solution was filtered, and the filtrate was concentrated under reduced pressure. The concentrate was dissolved in ethyl acetate (20.0 mL), washed with water (2.0 mL), and then washed with a saturated ammonium chloride aqueous solution (2.0 mL). The resulting solution was dried over anhydrous sodium sulfate and concentrated under reduced pressure. The crude product was purified by silica gel column chromatography (DCM: MeOH = 100:1 ∼ 15:1) to obtain a product (200.0 mg, yield: 40.9%).

### Step 2: synthesis of N-(5-((6,7-dimethoxyquinolin-4-yl)oxy)pyridine-2-yl)-1-(4-fluorophenyl)-2-oxo-5-((tetrahydro-2H-pyran-4-yl)methyl)-1,2-dihydropyridin-3-carboxamide

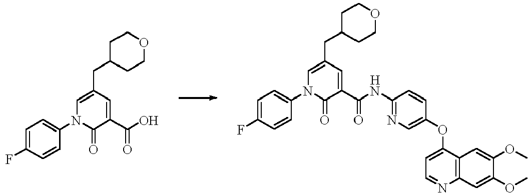

1-(4-fluorophenyl)-2-oxo-5-((tetrahydro-2*H*-pyran-4-yl)methyl)-1,2-dihydropyridin-3-carboxylic acid (200.0 mg, 0.604 mmol, 1.0 eq) and *N,N*-diisopropylethylamine (234.0 mg, 1.81 mmol, 3.0 eq) were dissolved in *N, N*-dimethylformamide (2.0 mL), and the resulting solution was cooled to 0 °C. HATU (344.0 mg, 0.906 mmol, 1.5 eq) was added under a nitrogen atmosphere, and the reaction solution was stirred for 0.5 hr. 5-((6,7-dimethoxyquinolin-4-yl)oxy)pyridin-2-amine (179.0 mg, 0.604 mmol, 1.0 eq) was added, and the resulting solution reacted for 4 hrs. After the reaction was completed, as detected by TLC, Ethyl acetate (30.0 mL) was added, and the resulting mixture was washed with a saturated sodium bicarbonate aqueous solution (2.0 mL), then with a saturated ammonium chloride aqueous solution (2.0 mL), and then with water (2.0 mL × 4). The resulting solution was dried and concentrated under reduced pressure. The crude product was purified by preparative thin-layer chromatography (DCM: MeOH = 20: 1) to obtain a product (250.0 mg, yield: 67.9%).

¹H NMR (400 MHz, DMSO-*d*₆) δ(ppm): 12.54 (s, 1H), 8.51-8.50 (m, 2H), 8.49-8.36 (m, 2H), 7.99-7.98 (d, 1H), 7.87-7.85 (m, 1H), 7.95-7.94 (d, 1H), 7.63-7.62 (m, 2H), 7.61-7.61 (m, 1H), 7.53-7.41 (m, 3H), 6.57-6.55 (d, 1H), 3.96-3.94 (d, 6H), 3.86-3.83 (m, 2H), 3.30-3.27 (m, 2H), 2.49-2.47 (m, 2H), 1.80-1.70 (m, 1H), 1.60-1.56 (m, 2H), 1.24-1.23 (m, 2H).

Molecular formula: C₃₄H₃₁FN₄O₆ Molecular weight: 610.64 LC-MS(Pos, *m*/*z*)=611.40[M+H]⁺.

### Example 22: synthesis of N-(5-((6,7-dimethoxyquinolin-4-yl)oxy) pyrimidin-2-yl)-5-(4-fluorophenyl)-4-oxo-1-(tetrahydro-2H-pyran-4-yl)-1,4-dihydropyridazin-3-carboxamide (compound 33)

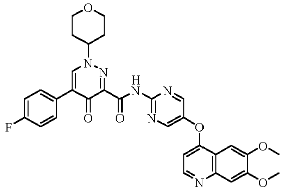

### Steps

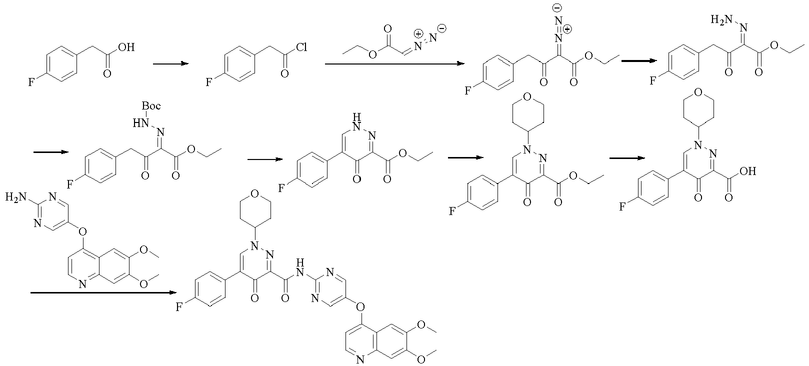

### Step 1: synthesis of 2-(4-fluorophenyl)acetyl chloride

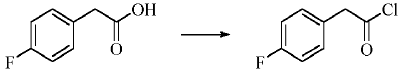

2-(4-fluorophenyl)acetic acid (100.0 g, 648 mmol, 1.0 eq) was dissolved in dichloromethane (200 mL), then thionyl chloride (193.0 g, 1,622 mmol, 2.5 eq) was added, and the reaction solution was stirred at 50 °C for 2 hrs. After the reaction was completed, as monitored by TLC, the reaction solution was concentrated under reduced pressure to obtain a product (116.0 g crude).

### Step 2: synthesis of ethyl 2-diazo-4-(4-fluorophenyl)-3-oxobutyrate

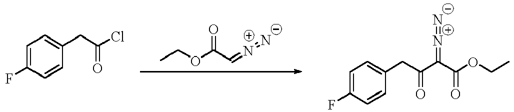

2-(4-fluorophenyl)acetyl chloride (116.0 g crude) was dissolved in ethyl acetate (50 mL), then ethyl 2-diazoacetate (153.3 g, 1,344 mmol) was added under an ice bath, and the reaction solution was gradually warmed to room temperature and stirred overnight. After the reaction was completed, as monitored by TLC, a saturated potassium carbonate aqueous solution (100 mL) was added dropwise under an ice bath. The resulting solution was stirred for 20 min, and the liquid was separated. The organic phase was dried over anhydrous sodium sulfate, filtered and concentrated. The crude product was purified by silica gel column chromatography (petroleum ether : ethyl acetate = 100:1 ∼ 60:1) to obtain a product (60.3 g, two-step yield: 35.8%).

### Step 3: synthesis of ethyl 4-(4-fluorophenyl)-2-hydrazono-3-oxobutyrate

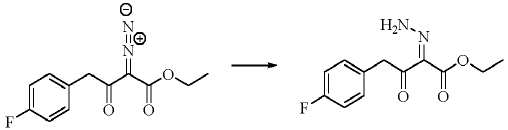

Ethyl 2-diazo-4-(4-fluorophenyl)-3-oxobutyrate (57.1 g, 228.2 mmol) was dissolved in methyl *tert-butyl* ether (270.0 mL), then tributylphosphine (55.4 g, 273 mmol, 1.2 eq) was added under an ice bath, and the reaction solution was gradually warmed to room temperature and stirred overnight. After the reaction was completed, as monitored by TLC, ethyl acetate (150 mL) and water (200 mL) were added, and the liquid was separated. The organic phase was washed with saturated brine (100 mL), dried over anhydrous sodium sulfate, and filtered under vacuum. The filtrate was concentrated under reduced pressure to obtain a product (125.3 g crude).

### Step 4: synthesis of tert-butyl 2-(1-ethoxy-4-(4-fluorophenyl)-1,3- dioxobutan-2-ylene)hydrazin-1-carboxylate

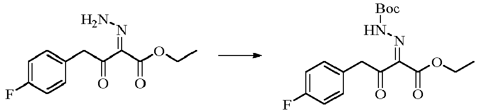

Ethyl 4-(4-fluorophenyl)-2-hydrazono-3-oxobutyrate (93.0 g crude) was dissolved in tetrahydrofuran (150 mL), and the resulting solution was cooled to 0 °C under an ice bath. Triethylamine (37.2 g, 368 mmol) and di-*tert*-butyl dicarbonate (96.5 g, 442.0 mmol) were added, and DMAP (13.4 g, 110.0 mmol) was added in bathes. The reaction solution was gradually warmed to room temperature and stirred overnight. After the reaction was completed, as monitored by TLC, the reaction solution was concentrated under reduced pressure. Ethyl acetate (300 mL) and water (500 mL) were added, and the liquid was separated. The organic phase was dried over anhydrous sodium sulfate and filtered under vacuum, and the filtrate was concentrated under reduced pressure to obtain a product (52.0 g crude).

### Step 5: synthesis of ethyl 5-(4-fluorophenyl)-4-oxo-1,4-dihydropyridazin-3-carboxylate

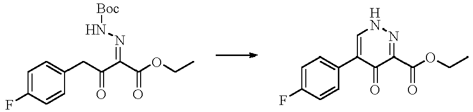

*Tert*-butyl 2-(1-ethoxy-4-(4-fluorophenyl)-1,3-dioxobutan-2-ylene)hydrazine-1-carboxylate (52.0 g crude) was dissolved in toluene (100 mL), then *N,N*-dimethylformamide dimethyl acetal (35.1 g, 295.0 mmol) was added, and the reaction solution was stirred overnight at 60 °C. After the reaction was completed, as monitored by LC-MS, the system was cooled to room temperature and filtered under vacuum, and the filter cake was dried to obtain a product (22.1 g, 3-step yield: 49.8%).

### Step 6: synthesis of ethyl 5-(4-fluorophenyl)-4-oxo-1-(tetrahydro-2H-pyran-4-yl)-1,4-dihydropyridazin-3 -carboxylate

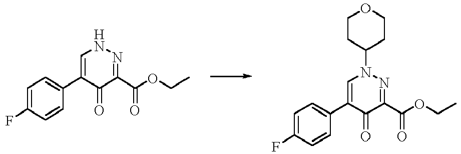

Ethyl 5-(4-fluorophenyl)-4-oxo-1,4-dihydropyridazin-3-carboxylate (500.0 mg, 1.9 mmol, 1.0 eq) was dissolved in DMF (5 mL), then cesium carbonate (1.85 g, 5.70 mmol, 3.0 eq) and 4-bromotetrahydro-2*H*-pyran (628.7 mg, 3.81 mmol, 2.0 eq) were added, and the reaction solution was stirred at 120 °C for 5 hrs. After the reaction was completed, as monitored by TLC, the reaction solution was concentrated under reduced pressure. Ethyl acetate (15 mL) and water (20 mL) were added, and the liquid was separated. The organic phase was dried over anhydrous sodium sulfate, filtered and concentrated. The crude product was purified by silica gel column chromatography (petroleum ether: ethyl acetate = 8:1 ∼ 2:1) to obtain a product (342.0 mg, yield: 51.9%).

### Step 7: synthesis of 5-(4-fluorophenyl)-4-oxo-1-(tetrahydro-2H-pyran-4-yl)-1,4-dihydropyridazin-3-carboxylic acid

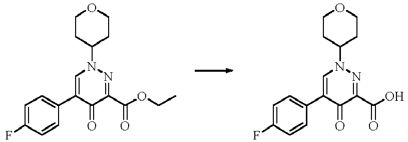

Ethyl 5-(4-fluorophenyl)-4-oxo-1-(tetrahydro-2*H*-pyran-4-yl)-1,4-dihydropyridazin-3-carboxylate (342.0 mg, 0.98 mmol, 1.0 eq) was dissolved in methanol (4.0 mL), then the aqueous solution (2.0 mL) of lithium hydroxide monohydrate (124.2 mg, 2.96 mmol, 3.0 eq.) was added, and the reaction solution was stirred at room temperature for 0.5 hr. After the reaction was completed, as monitored by TLC, the reaction solution was concentrated under reduced pressure, and pH was adjusted to 4 with hydrochloric acid (1 mol/L, 2.0 mL). The concentrate was stirred for 20 min and filtered under vacuum, and the filter cake was dried to obtain a product (235.0 mg, yield: 74.8%).

### Step 8: synthesis of N-(5-((6,7-dimethoxyquinolin-4-yl)oxy)pyrimidin-2-yl)-5-(4-fluorophenyl)-4-oxo-1-(tetrahydro-2H-pyran-4-yl)-1,4-dihydropyridazin-3-carboxamide

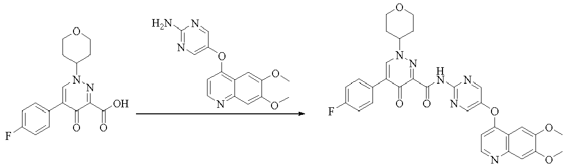

5-(4-fluorophenyl)-4-oxo-1-(tetrahydro-2*H*-pyran-4-yl)-1,4-dihydropyridazin-3-carboxylic acid (235.0 mg, 0.73 mmol, 1.0 eq) and 5-((6,7- dimethoxyquinolin-4-yl)oxy)pyrimidin-2-amine (220.2 mg, 0.73 mmol, 1.0 eq) were added to pyridine (3 mL), then phosphorus oxychloride (0.2 mL) was added dropwise, and the reaction solution was stirred for 30 min. After the reaction was completed, as monitored by TLC, the reaction solution was concentrated under reduced pressure. Dichloromethane (20 mL) and water (15 mL) were added, and the liquid was separated. The organic phase was dried over anhydrous sodium sulfate, filtered and concentrated, and the crude product was purified by silica gel column chromatography (dichloromethane: methanol = 80:1 ∼ 40:1) to obtain a product (186.3 mg, yield: 41.0%).

¹H NMR(400 MHz, DMSO-*d*₆) δ(ppm): 12.86 (s, 1H), 8.91 (s, 1H), 8.80 (s,2H), 8.50-8.51 (m, 1H), 7.94-7.97 (m, 2H), 7.55 (s, 1H), 7.43(s, 1H), 7.31-7.35(m, 2H), 6.68-6.69 (d, 1H),4.61-4.69 (m, 1H), 4.03-4.06 (m, 2H), 3.95-3.96 (m, 6H), 3.44-3.50 (m, 2H), 2.03-2.16 (m, 4H).

Molecular formula: C₃₁H₂₇FN₆O₆ Molecular weight: 598.59 LC-MS (Pos, m/z)=599.33 [M+H]⁺.

### Example 23: synthesis of N-(5-((6,7-dimethoxyquinolin-4-yl)oxy)pyridin-2 -yl)-5-(4-fluorophenyl)-4-oxo-1-(tetrahydro-2H-pyran-4-yl)-1,4-dihydropyridazin-3-carboxamide (compound 34)

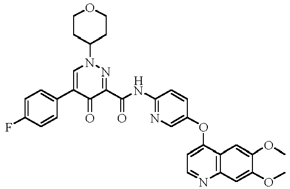

### Step 1: synthesis of N-(5-((6,7-dimethoxyquinolin-4-yl)oxy)pyridin-2-yl)-5-(4-fluorophenyl)-4-oxo-1-(tetrahydro-2H-pyran-4-yl)-1,4-dihydropyridazin-3-carboxamide

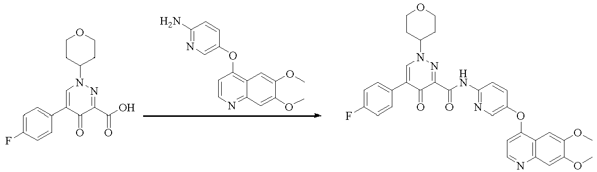

5-(4-fluorophenyl)-4-oxo-1-(tetrahydro-2*H*-pyran-4-yl)-1,4-dihydropyridazin-3-carboxylic acid (238.0 mg, 0.74 mmol, 1.0 eq) and 5-((6,7- dimethoxyquinolin-4-yl)oxy)pyridin-2-amine (222.3 mg, 0.74 mmol, 1.0 eq) were added to pyridine (3 mL), then phosphorus oxychloride (0.2 mL) was added dropwise, and the reaction solution was stirred for 30 min. After the reaction was completed, as monitored by TLC, the reaction solution was concentrated under reduced pressure. Ethyl acetate (20 mL) and water (15 mL) were added, and the liquid was separated. The organic phase was dried over anhydrous sodium sulfate, filtered and concentrated, and the crude product was purified by preparative thin-layer chromatography (dichloromethane: methanol = 15:1) to obtain a product (184.7 mg, yield: 41.6%).

¹H NMR (400 MHz, DMSO-*d*₆) δ(ppm): 13.02 (s, 1H), 8.95 (s, 1H), 8.50-8.51 (d, 1H), 8.41-8.44 (m, 2H), 7.95-7.99 (m, 2H), 7.88-7.91 (m, 1H), 7.55 (s, 1H), 7.42 (s, 1H), 7.32-7.37 (m, 2H), 6.58-6.59 (d, 1H), 4.67-4.70 (m, 1H), 4.04-4.08 (m, 2H). 3.95-3.96 (m, 6H), 3.45-3.51 (m, 2H), 2.07-2.19 (m, 4H).

Molecular formula: C₃₂H₂₈FN₅O₆ Molecular weight: 597.60 LC-MS(Pos, *m*/*z*)=598.32 [M+H]⁺.

### Example 24: synthesis of N-(4-(2-amino-5-(3,4-dimethoxyphenyl)pyridin -3-yl)phenyl)-2-oxo-1-((tetrahydro-2H-pyran-4-yl)methyl)-5-(p-tolyl)-1,2-dihydropyridin-3-carboxamide (compound 35)

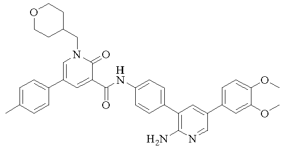

### Step 1: synthesis ofN-(4-(2-amino-5-(3,4-dimethoxyphenyl)pyridine-3-yl)phenyl)-2-oxo-1-((tetrahydro-2H-pyran-4-yl)methyl)-5-(p-tolyl)-1,2-dihydropyridin-3 -carboxamide

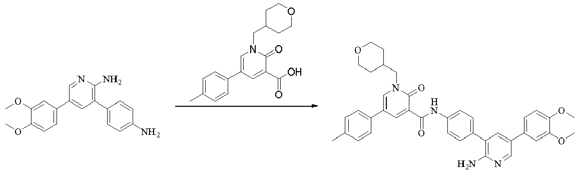

2-oxo-1-((tetrahydro-2*H*-pyran-4-yl)methyl)-5-(*p*-tolyl)-1,2-dihydropyridin-3-formic acid (50.94 mg, 0.1556 mmol, 1.0 eq), HOBT (21.03 mg, 0.1556 mmol, 1.0 eq), HATU (88.75 mg, 0.2334 mmol, 1.5 eq), DMAP (9.5 mg, 0.0778 mmol, 0.5 eq) and DIPEA (40.2 mg, 0.3112 mmol, 2.0 eq) were dissolved in DMF (1 mL), and the resulting solution was stirred for 3 hrs. Then 3-(4-aminophenyl)-5-(3,4-dimethoxyphenyl)pyridin-2-amine (50 mg, 0.1556 mmol, 1.0 eq) was added, and the reaction solution was stirred overnight. After the reaction was completed, as monitored by TLC, the reaction solution was poured into H₂O (10 mL). DCM (10 mL × 3) was added for extraction, and the organic phases were combined, washed with H₂O (10 mL × 3), and concentrated. The crude product was purified by preparative thin-layer chromatography (DCM MeOH = 20:1) to obtain *N*-(4-(2-amino -5-(3,4-dimethoxyphenyl)pyridin-3-yl)phenyl)-2-oxo-1-((tetrahydro-2*H*-pyran-4-yl)methyl)-5-(*p-*tolyl)-1,2-dihydropyridin-3-carboxamide (7.0 mg, yield: 7.1%).

¹HNMR(400MHz, DMSO-*d*₆) δ(ppm): 12.29 (s, 1H), 8.76 (s, 1H), 8.75 (s, 1H), 8.52 (s, 1H), 7.84-7.86 (m, 2H), 7.54-7.58 (m, 5H), 7.20-7.32 (m, 2H), 7.16-7.19 (m, 2H), 7.14-7.16 (m, 1H), 5.66 (s, 2H), 4.12 (m, 2H), 3.71-3.89 (m, 8H), 3.25-3.27 (m, 2H), 2.36 (s, 3H), 2.22 (m, 1H), 1.43-1.49 (m, 2H), 1.39-1.41 (m, 2H).

Molecular formula: C₃₈H₃₈N₄O₅ Molecular weight: 630.75 LC-MS(Pos, *m*/*z*)=631.26 [M+H]⁺.

### Example 25: synthesis of N-(4-(2-amino-5-(3,4-dimethoxyphenyl)pyridin -3-yl)phenyl)-6-oxo-1-((tetrahydro-2H-pyran-4-yl)methyl)-5-(p-tolyl)-1,6-dihydropyridin-3-carboxamide (compound 36)

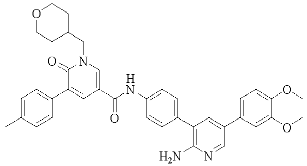

### Step 1: synthesis of N-(4-(2-amino-5-(3,4-dimethoxyphenyl)pyridin-3-yl) phenyl) -6-oxo-1-((tetrahydro-2H-pyran-4-yl)methyl)-5-(p-tolyl)-1,6- dihydropyridin-3-carboxamide

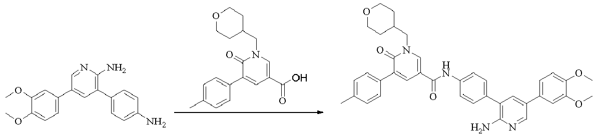

6-oxo-1-((tetrahydro-2*H*-pyran-4-yl)methyl)-5-(p-tolyl)-1,6-dihydropyridin-3-formic acid (61.22 mg, 0.187 mmol, 1.0 eq), HATU (106.66 mg, 0.2805 mmol, 1.5 eq) and triethylamine (56.77 mg, 0.561 mmol, 3.0 eq) were dissolved in DMF (1 mL), and the resulting solution was stirred for 1 hr. Then 3-(4-aminophenyl)-5-(3,4-dimethoxyphenyl)pyridin-2-amine (60 mg, 0.187 mmol, 1.0 eq) was added, and the reaction solution was further stirred for 2 hrs. After the reaction was completed, as monitored by LC-MS, the reaction solution was poured into H₂O (10 mL). EA was added for extraction (10 mL × 3), and the organic phases were combined, washed with H₂O (10 mL × 3), and concentrated. The crude product was purified by preparative thin-layer chromatography (DCM : MeOH = 20:1) to obtain *N*-(4-(2-amino-5- (3,4-dimethoxyphenyl)pyridin-3-yl)phenyl)-6-oxo-1-((tetrahydro-2*H*-pyran-4-yl)methyl)-5-(*p-*tolyl)-1,6-dihydropyridin-3-carboxamide (20.0 mg, yield: 16.9%).

¹HNMR(400MHz, DMSO-*d*₆)δ(ppm): 10.13 (s, 1H), 8.49 (s, 1H), 7.84-8.48 (m, 2H), 7.67-7.69 (m, 2H), 7.53-7.61 (m, 5H), 7.01-7.62 (m, 4H), 6.98-7.00 (m, 1H), 5.67-5.76 (s, 2H), 3.77-4.01 (m, 10H), 3.23-3.33 (m, 2H), 2.35 (s, 3H), 2.10-2.11 (m, 1H), 1.47-1.50 (m, 2H), 1.33-1.39 (m, 2H).

Molecular formula: C₃₈H₃₈N₄O₅ Molecular weight: 630.75 LC-MS(Pos, *m*/*z*)=631.24 [M+H]⁺.

### Example 26: synthesis of N-(4-(2-amino-5-(3,4-dimethoxyphenyl)pyridin -3-yl)phenyl)-2,4-dioxo-1-((tetrahydro-2H-pyran-4-yl)methyl)-3-(p-tolyl)-1,2,3,4-tetrahydropyrimidin-5-carboxamide (compound 37)

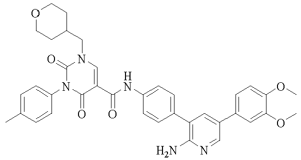

### Step 1: synthesis of N-(4-(2-amino-5-(3,4-dimethoxyphenyl)pyridin-3-yl) phenyl)-2,4-dioxo-1-((tetrahydro-2H-pyran-4-yl)methyl)-3-(p-tolyl)-1,2,3,4-tetrahydropyrimidin-5-carboxamide

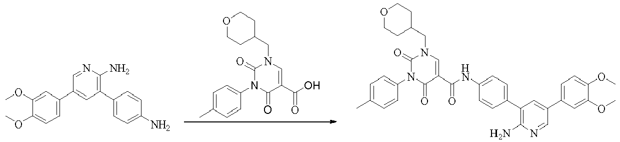

2,4-dioxo-1-((tetrahydro-2*H*-pyran-4-yl)methyl)-3-(*p*-tolyl)-1,2,3,4-tetrahydropyrimidin-5-formic acid (64.4 mg, 0.187 mmol, 1.0 eq), HATU (106.85 mg, 0.281 mmol, 1.5 eq) and Et₃N (57.17 mg, 0.561 mmol, 3.0 eq) were dissolved in DMF (1 mL), and the resulting solution was stirred for 2 hrs. Then 3-(4-aminophenyl)-5-(3,4-dimethoxyphenyl)pyridin-2-amine (60 mg, 0.187 mmol, 1.0 eq) was added, and the reaction solution was further stirred for 2 hrs. After the reaction was completed, as monitored by LC-MS, the reaction solution was poured into H₂O (10 mL). EA was added for extraction (10 mL × 3), and the organic phases were combined, washed with H₂O (10 mL × 3), and concentrated. The crude product was purified by preparative thin-layer chromatography (DCM: MeOH = 20:1) to obtain *N*-(4-(2-amino-5- (3,4-dimethoxyphenyl)pyridin-3-yl)phenyl)-2,4-dioxo-1-((tetrahydro-2*H*-pyran-4-yl)methyl)-3-(*p*-tolyl)-1,2,3,4-tetrahydropyrimidin-5-carboxamide (9.6 mg, yield: 7.9%).

¹HNMR(400MHz, DMSO-*d*₆) δ(ppm): 11.01 (s, 1H), 8.81 (s, 1H), 8.25 (s, 1H), 7.51-7.78 (m, 2H), 7.22-7.32 (m, 3H), 6.97-7.18 (m, 7H), 5.67-5.76 (s, 2H), 3.76-3.88 (m, 8H), 3.24-3.27 (m, 2H), 2.38-2.51 (m, 2H), 1.99 (s, 1H), 1.57-1.61 (m, 2H), 1.26-1.29 (m, 2H).

Molecular formula: C₃₇H₃₇N₅O₆ Molecular weight: 647.73 LC-MS(Pos, *m*/*z*)=648.23 [M+H]⁺.

### Example 27: synthesis of intermediate 2,4-dioxo-1-((tetrahydro-2H-pyran -4yl)ethyl)-(p-tolyl)-1,2,3,4-tetrahydropyrimidin-5-formic acid

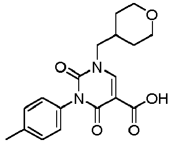

### Steps

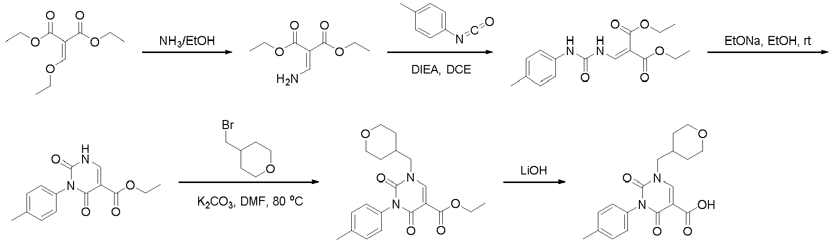

### Step 1: synthesis of diethyl 2-(aminomethylene)malonate

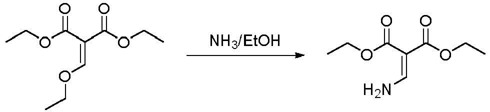

Ethanol (200 mL) was purged with ammonia gas for 30 min. Diethyl 2-(ethoxymethylene)malonate (21.6 g, 0.1 mol, 1.0 eq) was dissolved in the solution of ammonia in ethanol, and the reaction solution reacted at room temperature for 1 hr. After the reaction was completed, as detected by LC-MS and TLC, the reaction solution was concentrated, and the crude product was purified by silica gel column chromatography (100-200 mesh silica gel, PE/EA = 5:1) to obtain a white solid (18 g, yield: 96%).

### Step 2: synthesis of diethyl 2-((3-(p-tolyl)ureido)methylene)malonate

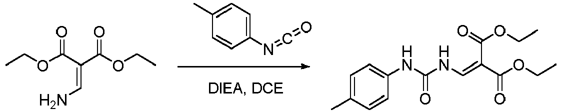

Diethyl 2-(aminomethylene)malonate (14 g, 74.86 mmol, 1.0 eq) was dissolved in 1,2-dichloroethane (200 mL), then 1-isocyanato-4-methylbenzene (10.95 g, 82.35 mmol, 1.1 eq) and DIEA (10.62 g, 82.35 mmol, 1.1 eq) were added, and the reaction solution was heated to reflux for 6 hrs. After the reaction was completed, as detected by LC-MS and TLC, the reaction solution was cooled to 0 °C, and a large number of white solids were precipitated. The mixture was filtered under vacuum, and the filter cake was washed with diethyl ether and dried to obtain a white solid (7.8 g, yield: 33%).

### Step 3: synthesis of ethyl 2,4-dioxo-3-(p-tolyl)-1,2,3,4-tetrahydropyrimidin-5-formate

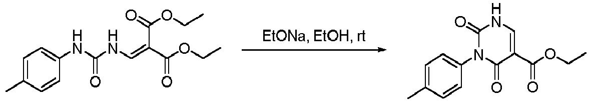

Diethyl 2-((3-(*p*-tolyl)ureido)methylene)malonate (8.0 g, 25 mmol, 1.0 eq) was dissolved in ethanol (40 mL), then a solution of 21% sodium ethoxide in ethanol (8.93 g, 27.5 mmol, 1.1 eq) was added, and the reaction solution was stirred at room temperature for 4 hrs. After the reaction was completed, as detected by LC-MS and TLC, the reaction solution was concentrated. EA (300 mL) and a solution of 1 mol/L citric acid in water (150 mL) were added to the residue, and the liquid was separated. EA (150 mL × 2) was added to the resulting aqueous phase for extraction, and the organic phases were combined, washed with brine, dried, filtered and concentrated. The crude product was purified by silica gel column chromatography (100-200 mesh silica gel, PE/EA = 1:1) to obtain a white solid (3.8 g, yield: 55%).

### Step 4: synthesis of ethyl 2,4-dioxo-1-((tetrahydro-2H-pyran-4-yl) methyl)-3-(p-tolyl)-1,2,3,4-tetrahydropyrimidin-5 -formate

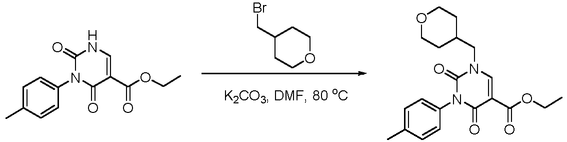

Ethyl 2,4-dioxo-3-(*p*-tolyl)-1,2,3,4-tetrahydropyrimidin-5-formate (3.76 g, 13.7 mmol, 1.0 eq) was dissolved in DMF (45 mL), and 4-(bromomethyl) tetrahydro-2*H*-pyran (4.88 g, 27.4 mmol, 2.0 eq) and potassium carbonate (5.67 g, 41.1 mmol, 3.0 eq) were added. The reaction solution was heated to 60 °C and reacted for 4 hrs. After the reaction was completed, as detected by LC-MS and TLC, the reaction solution was cooled to room temperature and filtered under vacuum. The filtrate was poured into water (50 mL), and EA (50 mL × 3) was added for extraction. The organic phases were combined, washed three times with brine, dried, filtered and concentrated. The crude product was purified by silica gel column chromatography (100-200 mesh silica gel, PE/EA = 0-50%) to obtain an off-white solid (1.9 g, yield: 37%).

### Step 5: synthesis of 2,4-dioxo-1-((tetrahydro-2H-pyran-4-yl)methyl)-3- (p-tolyl)-1,2,3,4-tetrahydropyrimidin-5-formic acid

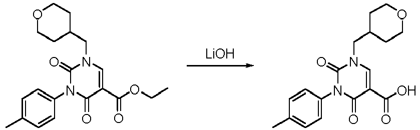

Ethyl 2,4-dioxo-1-((tetrahydro-2*H*-pyran-4-yl)methyl)-3-(*p*-tolyl)-1,2,3,4 - tetrahydropyrimidin-5-formate (1.86 g, 5 mmol, 1.0 eq) was dissolved in a mixture solution (20 mL) of THF/water/MeOH (1:1:1), and LiOH (0.36 g, 15 mmol, 3 eq) was added. The reaction solution reacted at room temperature for 3 hrs. After the reaction was completed, as detected by LC-MS, the reaction solution was concentrated to remove most of the solvents. Water (20 mL) was added, and the pH was adjusted to 2 with 2 mol/L hydrochloric acid. White solids were precipitated and filtered out under vacuum. The solid was dried to obtain 2,4-dioxo-1-((tetrahydro-2*H*-pyran-4-yl)methyl)-3-(p-tolyl)-1,2,3,4- tetrahydropyrimidin-5 -formic acid (white solid, 1.2 g, yield: 70%).

¹HNMR (300 MHz, DMSO-*d*₆) δ ppm: 12.70 (br, 1H), 8.76 (s, 1H), 7.28 (d, *J=* 6.9 Hz, 2H), 7.17 (m, 2H), 3.87-3.83 (m, 4H), 3.25 (t, *J=* 11.4 Hz, 2H), 2.36 (s, 3H), 1.99-1.95 (m, 1H), 1.57-1.54 (m, 2H), 1.30-1.18 (m, 2H).

LC-MS(*m*/*z*)= 345.0 [M+H]⁺.

### Example 28: synthesis of intermediate 6-oxo-1-((tetrahydro-2H-pyran-4-yl) methyl)-5-(p-tolyl)-1,6-dihydropyridin-3-formic acid

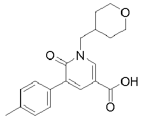

### Steps

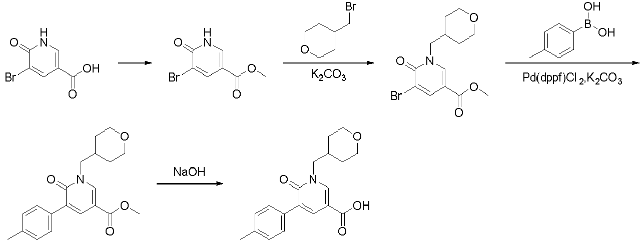

### Step 1: synthesis of methyl 5-bromo-6-oxo-1,6-dihydropyridin-3-carboxylate

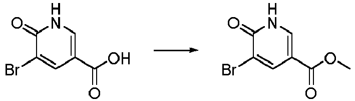

5-bromo-6-oxo-1,6-dihydropyridin-3-carboxylic acid (2.18 g, 10 mmol, 1.0 eq) was dissolved in MeOH (20 mL), and concentrated sulfuric acid (1 mL) was add dropwise. Then the reaction solution was heated to reflux for 24 hrs. After the reaction was completed, as detected by LC-MS and TLC, the reaction solution was cooled to 0 °C and white solids were precipitated. The mixture was filtered under vacuum, and the filter cake was washed with a small amount of cold methanol and dried to obtain a white solid (1.4 g, yield: 61%).

### Step 2: synthesis of methyl 5-bromo-6-oxo-1-((tetrahydro-2H-pyran-4-yl) methyl)-1,6-dihydropyridin-3 -formate

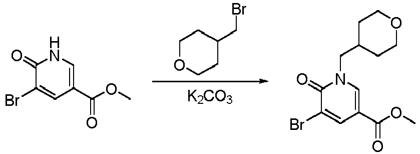

Methyl 5-bromo-6-oxo-1,6-dihydropyridin-3-carboxylate (2.3 g, 10 mmol, 1.0 eq) was dissolved in DMF (30 mL), and 4-(bromomethyl)tetrahydro -2*H*-pyran (2.15 g, 12 mmol, 1.2 eq) and potassium carbonate (4.15 g, 30 mmol, 3.0 eq) were added. The reaction solution was heated to 60 °C and stirred for 4 hrs. After the reaction was completed, as detected by LC-MS and TLC, the reaction solution was cooled to room temperature and filtered under vacuum. The mother liquor was poured into water (50 mL), and EA (50 mL × 3) was added for extraction. The organic phases were combined, washed 3 times with saturated brine, dried, filtered and concentrated. The crude product was purified by silica gel column chromatography (100-200 mesh silica gel, PE/EA = 0-50%) to obtain an off-white solid (1.8 g, yield: 55%).

### Step 3: synthesis of methyl 6-oxo-1-((tetrabydro-2H-pyran-4-yl)methyl)-5-(p-tolyl)-1,6-dihydropyridin-3 -formate

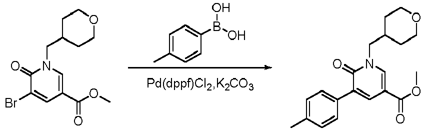

Methyl 5-bromo-6-oxo-1-((tetrahydro-2*H*-pyran-4-yl)methyl)-1,6-dihydropyridin-3-formate (3.3 g, 10 mmol, 1.0 eq), *p*-tolylboronic acid (1.63 g, 12 mmol, 1.2 eq), Pd(dppf)Cl₂.CH₂Cl₂ (816 mg, 1 mmol, 0.1 eq) and potassium carbonate (4.15 g, 30 mmol, 3.0 eq) were dissolved in a mixture of 1,4-dioxane and water (5:1, 50 mL), and nitrogen was charged to replace three times by evacuation. The reaction solution was heated to 85 °C and reacted for 2 hrs. After the reaction was completed, as detected by LC-MS and TLC, the reaction solution was cooled to room temperature and filtered under vacuum. The filtrate was added with EA (100 mL) and water (100 mL), and the liquid was separated. The organic phase was washed with brine, dried, filtered and concentrated. The crude product was purified by silica gel column chromatography (100-200 mesh silica gel, PE/EA = 20-100%) to obtain a white solid (1.7 g, yield: 50%).

### Step 4: synthesis of 6-oxo-1-((tetrahydro-2H-pyran-4-yl)methyl)-5-(p-tolyl)-1,6-dihydropyridin-3-formic acid

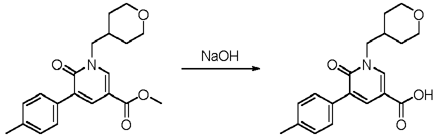

Methyl 6-oxo-1-((tetrahydro-2*H*-pyran-4-yl)methyl)-5-(*p*-tolyl)-1,6-dihydropyridin-3-formate (1.7 g, 5 mmol, 1 eq) was dissolved in a mixture (20 mL) of THF/water/MeOH (1:1:1), then NaOH (2.0 g, 50 mmol, 10 eq) was added, and the reaction solution reacted at room temperature for 3 hrs. After the reaction was completed, as detected by LC-MS, the reaction solution was concentrated to remove most of the solvents. Water (20 mL) was added, and the pH was adjusted to 2 with 2 mol/L hydrochloric acid. White solids were precipitated, filtered out under vacuum, and dried to obtain 6-oxo-1-((tetrahydro-2*H*-pyran-4-yl)methyl)-5-(*p-*tolyl)-1,6-dihydropyridin-3-formic acid (white solid, 1.2 g, yield: 73%).

¹HNMR (300 MHz, DMSO-*d*₆) δ ppm: 8.42 (d, *J* = 2.1 Hz, 1 H), 7.87 (d, *J* = 2.1 Hz, 1 H), 7.57 (d, *J* = 8.4 Hz, 2 H), 7.21 (d, *J* = 8.4 Hz, 2 H), 3.97 (d, *J* = 6.9 Hz, 2 H), 3.85-3.81 (m, 2H), 3.26-3.19 (m, 2 H), 2.33 (s, 3 H), 2.04 (m, 1 H), 1.46-1.24 (m, 4 H).

LC-MS(*m*/*z*)= 328.0 [M+H]⁺.

### Example 29: synthesis of intermediate 2-oxo-1-((tetrahydro-2H-pyran-4-yl)methyl)-5-(p-tolyl)-1,2-dihydropyridin-3 -formic acid

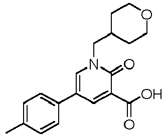

### Steps

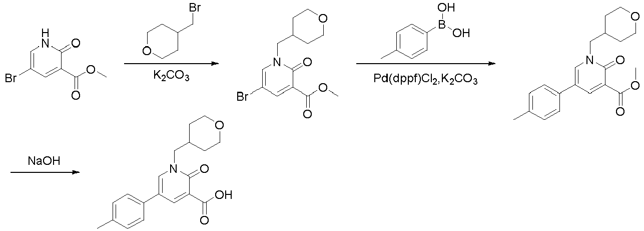

### Step 1: synthesis of methyl 5-bromo-2-oxo-1-((tetrahydro-2H-pyran-4-yl) methyl)-1,2-dihydropyridin-3 -formate

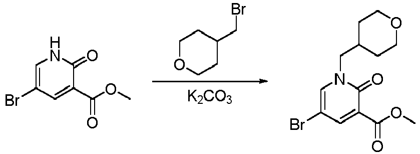

Methyl 5-bromo-2-oxo-1,2-dihydropyridin-3-carboxylate (2.3 g, 10 mmol, 1.0 eq) was dissolved in DMF (30 mL), and 4-(bromomethyl)tetrahydro -2*H*-pyran (2.15 g, 12 mmol, 1.2 eq) and potassium carbonate (4.15 g, 30 mmol, 3.0 eq) were added. The reaction solution was heated to 60 °C and stirred for 4 hrs. After the reaction was completed, as detected by LC-MS and TLC, the reaction solution was cooled to room temperature and filtered under vacuum. The mother liquor was poured into water (50 mL), and EA (50 mL × 3) was added for extraction. The organic phases were combined, washed 3 times with saturated brine, dried, filtered and concentrated. The crude product was purified by silica gel column chromatography (100-200 mesh silica gel, PE/EA = 0-50%) to obtain an off-white solid (1.8 g, yield: 55%).

### Step 2: synthesis of methyl 2-oxo-1-((tetrahydro-2H-pyran-4-yl)methyl)-5-(p-tolyl)-1,2-dihydropyridin-3 -formate

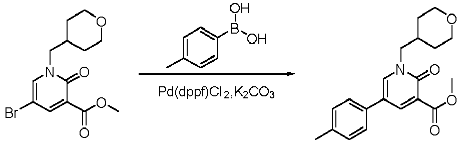

Methyl 5-bromo-2-oxo-1-((tetrahydro-2*H*-pyran-4-yl)methyl)-1,2-dihydropyridin-3-formate (3.3 g, 10 mmol, 1.0 eq), *p*-tolylboronic acid (1.63 g, 12 mmol, 1.2 eq), Pd(dppf)Cl₂.CH₂Cl₂ (816 mg, 1 mmol, 0.1 eq) and potassium carbonate (4.15 g, 30 mmol, 3.0 eq) were dissolved in a mixture of 1,4-dioxane and water (5:1, 50 mL), and nitrogen was charged to replace three times by evacuation. The reaction solution was heated to 85 °C and reacted for 2 hrs. After the reaction was completed, as detected by LC-MS and TLC, the reaction solution was cooled to room temperature and filtered under vacuum. The filtrate was added with EA (100 mL) and water (100 mL), and the liquid was separated. The organic phase was washed with brine, dried, filtered and concentrated. The crude product was purified by silica gel column chromatography (100-200 mesh silica gel, PE/EA = 20-100%) to obtain a white solid (1.7 g, yield: 50%).

### Step 3: synthesis of 2-oxo-1-((tetrahydro-2H-pyran-4-yl)methyl)-5-(p-tolyl)-1,2-dihydropyridin-3-formic acid

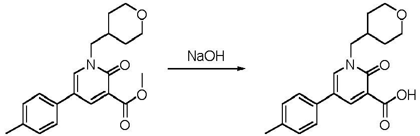

Methyl 2-oxo-1-((tetrahydro-2*H*-pyran-4-yl)methyl)-5-(*p*-tolyl)-1,2-dihydropyridin-3-formate (1.7 g, 5 mmol, 1 eq) was dissolved in a mixture (20 mL) of THF/water/MeOH (1:1:1), then NaOH (2.0 g, 50 mmol, 10 eq) was added, and the reaction solution reacted at room temperature for 3 hrs. After the reaction was completed, as detected by LC-MS, the reaction solution was concentrated to remove most of the solvents. Water (20 mL) was added, and the pH was adjusted to 2 with 2 mol/L hydrochloric acid. White solids were precipitated, filtered out under vacuum, and dried to obtain 2-oxo-1-((tetrahydro-2*H*-pyran-4-yl)methyl)-5-(*p-*tolyl)-1,2-dihydropyridin-3-formic acid (white solid, 1.2 g, yield: 73%).

¹HNMR (300 MHz, DMSO-*d*₆) δ ppm: 14.68 (br, 1H), 8.61 (s, 2H), 7.57 (d, *J* = 7.8 Hz, 2 H), 7.30 (d, *J* = 7.8 Hz, 2H), 4.09 (d, *J* = 7.2 Hz, 2H), 3.86-3.83 (m, 2H), 3.24 (t, *J* = 11.4 Hz, 2 H), 2.34 (s, 3 H), 2.16 (m, 1H), 1.49-1.32 (m, 4H).

LC-MS(*m*/*z*)= 328.0 [M+H]⁺.

### Example 30: synthesis of intermediate 3-(4-aminophenyl)-5-(3,4-dimethoxyphenyl)pyridin-2-amine

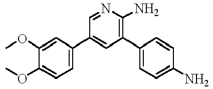

### Steps

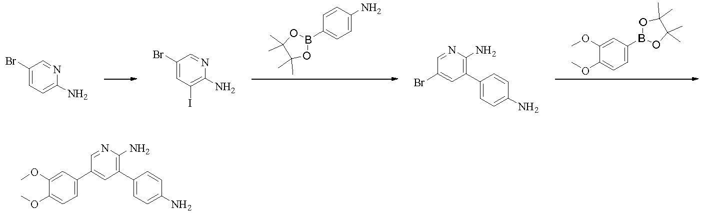

### Step 1: synthesis of 5-bromo-3-iodopyridin-2-amine

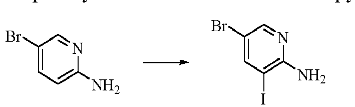

5-bromopyridin-2-amine (3.0 g, 17.34 mmol, 1.0 eq) was dissolved in MeCN, and NaIO₄ (1.484 g, 6.94 mmol, 0.4 eq) and I₂ (2.86 g, 11.27 mmol, 0.65 eq) were added. Then trifluoroacetic acid (1.285 g, 11.27 mmol, 0.65 eq) was slowly added dropwise with stirring, and the resulting solution was stirred for 5 min. Then the reaction solution was heated to 80 °C and reacted overnight. After the reaction was completed, as monitored by TLC, H₂O (200 mL) and EA (300 mL) were added, and the resulting solution was stirred and cooled to 0 °C. Sodium thiosulfate was slowly added until the system became colorless, and the liquid was separated. EA (200 mL) was added to the aqueous phase for extraction, and the organic phases were combined, dried, filtered and concentrated. The crude product was purified by silica gel column chromatography (PE : EA = 20:1 ∼ 15:1) to obtain a product (4.442 g, yield: 85.4%).

### Step 2: synthesis of 3-(4-aminophenyl)-5-bromopyridin-2-amine

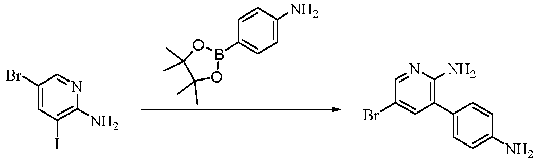

The intermediate 5-bromo-3-iodopyridin-2-amine (2.0 g, 6.691 mmol, 1.0 eq), Pd(PPh₃)₄ (773.2 mg, 0.669 mmol, 0.1 eq), 4-(4,4,5,5-tetramethyl-1,3,2-dioxaborolan-2-yl)aniline (1.466 g, 6.691 mmol, 1.0 eq) and potassium carbonate (2.774 g, 20.07 mmol, 3.0 eq) were dissolved in a mixture of dioxane (30 mL) and H₂O (7.5 mL), and the reaction solution reacted overnight at 95 °C. After the reaction was completed, as detected by TLC, H₂O (200 mL) and EA (200 mL) were added. The resulting solution was stirred and filtered under vacuum, and the filtrate was separated. EA (200 mL) was added to the aqueous phase for extraction, and the organic phases were combined, dried, filtered and concentrated. The crude product was purified by silica gel column chromatography (PE : EA = 10:1 ∼ 1:1) to obtain a product of amine (1.316 g, yield: 67.8%).

### Step 3: synthesis of 3-(4-aminophenyl)-5-(3,4-dimethoxyphenyl)pyridin- 2-amine

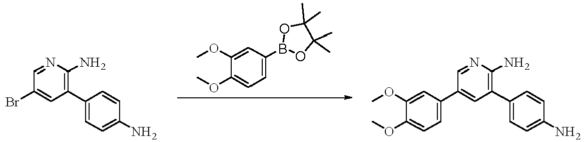

3-(4-aminophenyl)-5-bromopyridin-2-amine (1.3 g, 4.481 mmol, 1.0 eq), Pd(PPh₃)₄ (517.8 mg, 0.448 mmol, 0.1 eq), 2-(3,4-dimethoxyphenyl)-4,4,5,5- tetramethyl-1,3,2-dioxaborolane (1.18 g, 4.481 mmol, 1.0 eq) and potassium carbonate (1.86 g, 13.44 mmol, 3.0 eq) were dissolved in a mixture of dioxane (20 mL) and H₂O (5 mL), and the reaction solution reacted overnight at 90 °C. After the reaction was completed, as detected by TLC, H₂O (200 mL) and EA (200 mL) were added. The reaction solution was stirred and filtered under vacuum, and the filtrate was separated. EA (200 mL) was added to the aqueous phase for extraction, and the organic phases were combined, dried, filtered and concentrated. The crude product was purified by silica gel column chromatography (PE: EA = 3:1 ∼ 0:1) to obtain 3-(4-aminophenyl)-5-(3,4- dimethoxyphenyl)pyridin-2-amine (840 mg, yield: 58.3%).

### Example 31: synthesis of N-(5-((6,7-dimethoxyquinolin-4-yl)oxy)thiophen-2-yl)-4-oxo-1-((tetrahydro-2H-pyran-4-yl)methyl)-5-(p-tolyl)-1,4-dihydropyndin-3-carboxamide (compound 6)

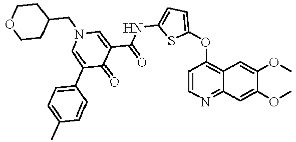

### Step 1: synthesis of 6,7-dimethoxyquinolin-4-((5-nitrothiophen-2-yl)oxy) quinoline

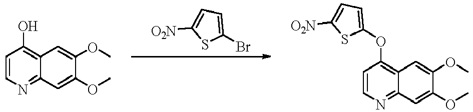

6,7-dimethoxyquinolin-4-ol (800.0 mg, 3.9 mmol), 2-bromo-5- nitrothiophene (892.4 mg, 4.29 mmol) and K₂CO₃ (1.62 g, 11.7 mmol) were added to DMF (6 mL), and the reaction solution was stirred overnight at 75 °C under a nitrogen atmosphere. After the reaction was completed, as monitored by TLC, the reaction solution was concentrated under reduced pressure. The crude product was purified by silica gel column chromatography (DCM: MeOH = 200:1 ∼ 80:1) to obtain a product (380.0 mg, yield: 29.2%).

### Step 2: synthesis of 6,7-dimethoxyquinolin-4-((5-aminothiophen-2-yl) oxy)quinoline

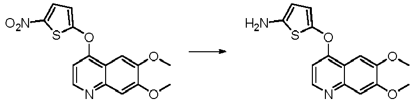

6,7-dimethoxyquinolin-4-((5-nitrothiophen-2-yl)oxy)quinoline (360.0 mg, 1.05 mmol) was added to a mixture of a saturated ammonium chloride aqueous solution (2 mL) and ethanol (8 mL), then reduced iron powder (607.2 mg, 10.48 mmol, 6.0 eq) was added, and the reaction solution was stirred overnight at 75 °C. After the reaction was completed, as monitored by TLC, the reaction solution was filtered through celite under vacuum, and the filtrate was concentrated under reduced pressure. The crude product was purified by silica gel column chromatography (DCM : MeOH = 60:1 ∼ 40:1) to obtain a product (80.0 mg, yield: 24.2%).

### Step 3: synthesis of N-(5-((6,7-dimethoxyquinolin-4-yl)oxy)thiophen-2-yl)-4-oxo-1-((tetrahydro-2H-pyran-4-yl)methyl)-5-(p-tolyl)-1,4-dihydropyridin-3 -carboxamide

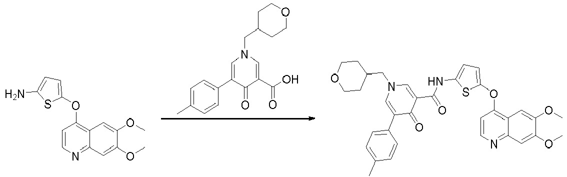

4-oxo-1-((tetrahydro-2*H*-pyran-4-yl)methyl)-5-(*p*-tolyl)-1,4-dihydropyridin-3-carboxylic acid (80.3 mg, 0.27 mmol) was dissolved in DMF (2 mL), and the resulting solution was cooled to 0 °C under an ice bath. HATU (156.0 mg, 0.41 mmol) and triethylamine (82.0 mg, 0.81 mmol) were added, and the reaction solution was stirred for 1 hr. 6,7-dimethoxyquinolin-4-((5- aminothiophen-2-yl)oxy)quinoline (81.6 mg, 0.27 mmol) was added, and the reaction solution was stirred overnight at room temperature. After the reaction was completed, as detected by TLC, the reaction solution was concentrated under reduced pressure. The concentrate was dissolved in ethyl acetate (10 mL), washed successively with a saturated sodium bicarbonate aqueous solution (5 mL), a saturated ammonium chloride aqueous solution (5 mL), water (5 mL × 4) and saturated brine (5 mL), dried over anhydrous sodium sulfate, and filtered under vacuum. The filtrate was concentrated under reduced pressure, and the crude product was purified by silica gel column chromatography (DCM : MeOH = 100:1 ∼ 40:1) to obtain a product (41.0 mg, yield 24.8%).

¹H NMR (400 MHz, DMSO-*d*₆) δ(ppm): 13.99 (s, 1H), 8.74-8.73 (d, 1H), 8.22-8.21 (d, 1H), 7.94-7.92 (d, 2H), 7.62-7.60 (d, 1H), 7.56 (s, 1H), 7.28-7.24 (m, 2H), 6.98-6.97 (d, 1H), 6.63 (s, 1H), 6.11-6.09 (d, 1H), 4.12-4.10 (d, 2H), 3.87-3.85 (d, 6H), 3.68 (s, 3H), 3.28-3.23 (t, 2H), 2.36 (s, 3H), 2.13-2.08 (m, 1H), 1.50-1.43 (m, 2H), 1.35-1.24 (m, 2H).

Molecular formula: C₃₄H₃₃N₃O₆S Molecular weight: 611.71 LC-MS(Pos, *m*/*z)=* 612.35[M+H]⁺.

### Example 32: synthesis of N-(5-((6,7-dimethoxyquinolin-4-yl)oxy)pyridin-2-yl)-1-((1,1-dioxotetrahydro-2H-thiopyran-4-yl)methyl)-4-oxo-5-(p-tolyl)-1,4-dihydropyridin-3-carboxamide (compound 7)

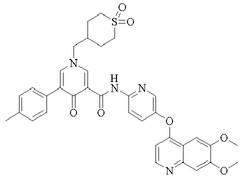

### Step 1: synthesis of methyl 1-((1,1-dioxotetrahydro-2H-thiopyran-4-yl) methyl)-4-oxo-5 -(p-tolyl)-1,4-dihydropyridin-3 -carboxylate

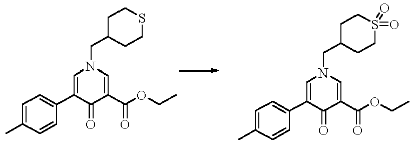

Methyl 4-oxo-1-((tetrahydro-2*H*-thiopyran-4-yl)methyl)-5-(*p*-tolyl)-1,4- dihydropyridin-3-carboxylate (150.0 mg, 0.404 mmol, 1.0 eq) was dissolved in dichloromethane (5.0 mL), then m-chloroperoxybenzoic acid (139.4 mg, 0.808 mmol, 2.0 eq) was added, and the reaction solution was stirred at room temperature for 2 hrs. After the reaction was completed, as detected by TLC, a saturated sodium thiosulfate aqueous solution was added to quench the reaction, and dichloromethane (10.0 mL) was added for extraction. The organic phase was dried and concentrated, and the crude product was purified by silica gel column chromatography (DCM : MeOH = 100:1 ∼ 40:1) to obtain a product (130.0 mg, yield: 79.8%).

### Step 2: synthesis of 1-((1,1-dioxotetrahydro-2H-thiopyran-4-yl)methyl)-4-oxo-5-(p-tolyl)-1,4-dihydropyridin-3-carboxylic acid

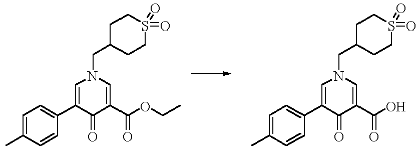

Methyl 1-((1,1-dioxotetrahydro-2*H*-thiopyran-4-yl)methyl)-4-oxo-5-(*p*-tolyl)-1,4-dihydropyridin-3-carboxylate (130.0 mg, 0.322 mmol, 1.0 eq) was dissolved in tetrahydrofuran (3.0 mL), then a lithium hydroxide aqueous solution (1 mol/L, 3.0 mL) was added, and the reaction solution was stirred at room temperature for 1 hr. After the reaction was completed, as detected by TLC, the reaction solution was concentrated under reduced pressure. Water (1.5 mL) was added, and the pH was adjusted to 3-4 with hydrochloric acid (1 mol/L). The resulting solution was filtered under vacuum, and the filter cake was dried at 40 °C to obtain a product (110.0 mg, yield: 90.9%).

### Step 3: synthesis of N-(5-((6,7-dimethoxyquinolin-4-yl)oxy)pyridin-2-yl)-1-((1,1-dioxotetrahydro-2H-thiopyran-4-yl)methyl)-4-oxo-5-(p-tolyl)-1,4-dihydropyridin-3-carboxamide

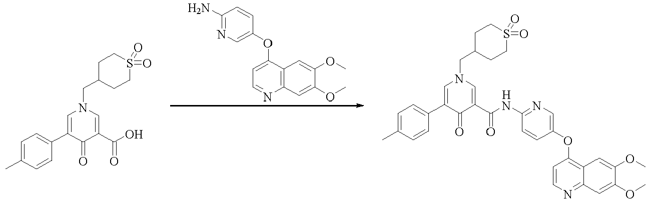

1-((1,1-dioxotetrahydro-2*H*-thiopyran-4-yl)methyl)-4-oxo-5-(*p*-tolyl)-1,4-dihydropyridin-3-carboxylic acid (110.0 mg, 0.293 mmol, 1.0 eq) and triethylamine (89.0 mg, 0.879 mmol, 3.0 eq) were dissolved in DMF, then HATU (167.0 mg, 0.439 mmol, 1.5 eq) was added at 0 °C under a nitrogen atmosphere, and the reaction solution was stirred for 1 hr. 5-((6,7-dimethoxyquinolin-4-yl)oxy)pyridin-2-amine (95.8 mg, 0.322 mol, 1.1 eq) was added, and the resulting reaction solution was slowly warmed to room temperature and reacted overnight. After the reaction was completed, as detected by TLC, the reaction solution was concentrated under reduced pressure. The crude product was dissolved in ethyl acetate (20.0 mL), washed with a saturated sodium bicarbonate aqueous solution (2.0 mL × 2) and then with water (2.0 mL × 4), dried over anhydrous sodium sulfate, and concentrated. The concentrate was purified by preparative thin-layer chromatography (DCM: MeOH = 10:1) to obtain a product (100.0 mg, yield: 52.1%).

¹H NMR(400 MHz, DMSO-*d*₆) δ(ppm): 13.47 (s, 1H), 8.84 (s, 1H), 8.50-8.49 (d, 1H), 8.45-8.43 (d, 1H), 8.39-8.38 (d, 1H), 8.23 (s, 1H), 7.88-7.85 (m, 1H), 7.62-7.59 (m, 2H), 7.55 (s, 1H), 7.42 (s, 1H), 7.27-7.25 (m, 2H), 6.57-6.55 (d, 1H), 4.23-4.20 (m, 2H), 3.96-3.95 (d, 6H), 3.22-3.06 (m, 3H), 2.93-2.90 (m, 1H), 2.06 (s, 3H), 2.32-2.28 (m, 1H), 2.05-2.01 (m, 2H), 1.82-1.79 (m, 2H).

Molecular formula: C₃₅H₃₄N₄O₇S Molecular weight: 654.74 LC-MS(Pos, *m*/*z*)=655.36[M+H]⁺.

### Example 33: synthesis of N-(5-((6,7-dimethoxyquinolin-4-yl)oxy)pyridin -2-yl)-1-((1-oxotetrahydro-2H-thiopyran-4-yl)methyl)-4-oxo-5-(p-tolyl)-1,4-dihydropyridin-3-carboxamide (compound 8)

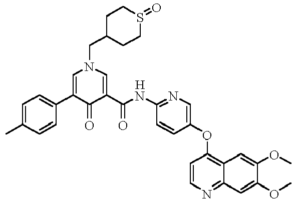

### Step 1: synthesis of N-(5-((6,7-dimethoxyquinolin-4-yl)oxy)pyridin-2-yl)-1-((1-oxotetrahydro-2H-thiopyran-4-yl)methyl)-4-oxo-5-(p-tolyl)-1,4-dihydropyridin-3-carboxamide

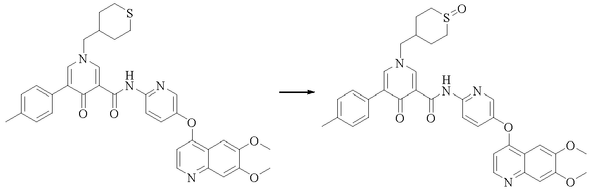

*N*-(5-((6,7-dimethoxyquinolin-4-yl)oxy)pyridin-2-yl)-4-oxo-1-((tetrahydro-2*H-*thiopyran-4-yl)methyl)-5-(*p*-tolyl)-1,4-dihydropyridin-3-carboxamide (65.0 mg, 0.104 mmol, 1.0 eq) was dissolved in dichloromethane (2.5 mL), then m-chloroperoxybenzoic acid (20.6 mg, 0.0835 mmol, 0.8 eq) was added at 0 °C, and the reaction solution was stirred for 0.5 hr. After the reaction was completed, as detected by TLC, a saturated sodium thiosulfate aqueous solution was added to quench the reaction, and dichloromethane (10.0 mL × 3) was added to the aqueous phase for extraction. The organic phases were combined, dried over anhydrous sodium sulfate, filtered and concentrated. The crude product was purified by silica gel column chromatography (DCM: MeOH = 80:1 ∼ 20:1) to obtain a product (40.0 mg, yield: 60%).

¹H NMR(400 MHz, DMSO-*d*₆) δ(ppm): 13.48 (s, 1H), 8.84 (s, 1H), 8.50-8.38 (m, 3H), 8.25 (s, 1H), 7.88-7.86 (m, 1H), 7.62-7.60 (m, 2H), 7.55 (s, 1H), 7.42 (s, 1H), 7.27-7.25 (m, 2H), 6.57-6.55 (d, 1H), 4.27-4.25 (m, 2H), 3.96 (s, 6H), 2.98-2.60 (m, 4H), 2.36 (s, 3H), 2.15-2.13 (m, 2H), 2.01-1.99 (m, 2H), 1.67-1.57 (m, 1H).

Molecular formula: C₃₅H₃₄N₄O₆S Molecular weight: 638.74 LC-MS(Pos, *m*/*z*)=639.0[M+H]⁺.

### Example 34: synthesis of N-(5-((6,7-dimethoxyquinolin-4-yl)oxy)pyridin -2-yl)-4-oxo-1-((tetrahydro-2H-thiopyran-4-yl)methyl)-5-(p-tolyl)-1,4-dihydropyridin-3-carboxamide (compound 9)

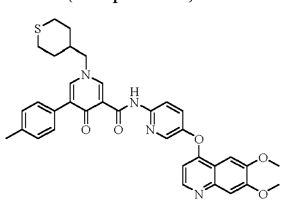

### Step 1: synthesis of 4-(bromomethyl)tetrahydro-2H-thiopyran

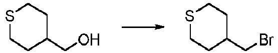

(Tetrahydro-2*H*-thiopyran-4-yl)methanol (1.0 g, 7.56 mmoL) and carbon tetrabromide (3.78 g, 11.34 mmol) were dissolved in dichloromethane (60 mL), and the resulting solution was cooled to 0 °C under an ice bath. Triphenylphosphine (2.99 g, 11.34 mmol) was added, and the reaction solution was slowly warmed to room temperature and reacted overnight. After the reaction was completed, as monitored by TLC, the reaction solution was concentrated. The crude product was purified by silica gel column chromatography (PE: EA = 100:1 ∼ 20:1) to obtain a product (350.0 mg, yield: 23.9%).

### Step 2: synthesis of ethyl 4-oxo-1-((tetrahydro-2H-thlopyran-4-yl) methyl)-5-(p-tolyl)-1,4-dihydropyridin-3 -carboxylate

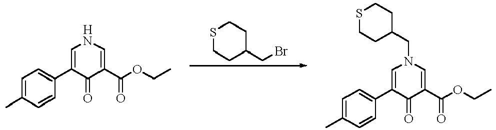

Ethyl 4-(bromomethyl)tetrahydro-2*H*-thiopyran (300.0 mg, 1.55 mmol), 4-oxo-5-(*p-*tolyl)-1,4-dihydropyridin-3-carboxylate (398.5 mg, 1.55 mmol) and potassium carbonate (642.7 mg, 4.65 mmol) were added to DMF (25 mL), and the reaction solution was stirred overnight at room temperature. After the reaction was completed, as detected by TLC, the reaction solution was concentrated under reduced pressure. Ethyl acetate and water were added, and the liquid was separated. The organic phase was dried over anhydrous sodium sulfate, filtered and concentrated. The crude product was purified by silica gel column chromatography (DCM: MeOH = 100:1 ∼ 20:1) to obtain a product (350.0 mg, yield: 60.8 %).

### Step 3: synthesis of 4-oxo-1-((tetrahydro-2H-thiopyran-4-yl)methyl)-5-(p-tolyl)-1,4-dihydropyridin-3-carboxylic acid

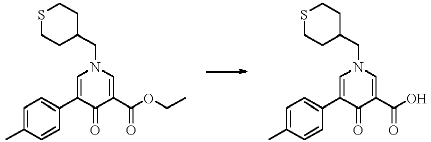

Ethyl 4-oxo-1-((tetrahydro-2*H*-thiopyran-4-yl)methyl)-5-(*p*-tolyl)-1,4- dihydropyridin-3-carboxylate (160.0 mg, 0.43 mmol) was added to a flask, then lithium hydroxide (1 mol/L, 2 mL) and tetrahydrofuran (2 mL) were added, and the reaction solution was stirred overnight at room temperature. After the reaction was completed, as detected by TLC, the reaction solution was concentrated under reduced pressure, and the pH was adjusted to 6-7 with hydrochloric acid (1 mol/L). Solids were precipitated, and filtered out under vacuum to obtain a product (151.0 mg, yield: 100%).

### Step 4: synthesis of N-(5-((6,7-dimethoxyquinolin-4-yl)oxy)pyridine-2-yl) -4-oxo-1-((tetrahydro-2H-thiopyran-4-yl)methyl)-5-(p-tolyl)-1,4-dihydropyridin-3-carboxamide

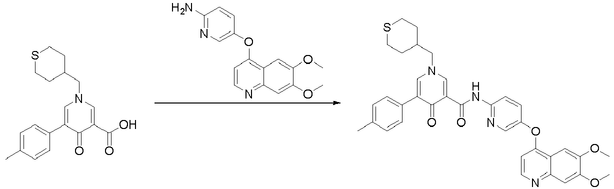

4-oxo-1-((tetrahydro-2*H*-thiopyran-4-yl)methyl)-5-(*p*-tolyl)-1,4-dihydropyridin-3-carboxylic acid (140.7 mg, 0.41 mmol) was added to a flask, then DMF (3 mL) was added, and the resulting solution was cooled to 0 °C under an ice bath. Triethylamine (124.6 mg, 1.23 mmol) and HATU (235.8 mg, 0.62 mmol) were added, and the reaction solution was stirred for 30 min. 5-((6,7-dimethoxyquinolin-4-yl)oxy)pyridin-2-amine (127.8 mg, 0.43 mmol) was added, and the reaction solution was slowly warmed to room temperature and stirred overnight. After the reaction was completed, as detected by TLC, the reaction solution was concentrated under reduced pressure. The concentrate was dissolved in ethyl acetate (10 mL), washed successively with a saturated sodium bicarbonate aqueous solution (5 mL), a saturated ammonium chloride aqueous solution (5 mL), water (5 mL × 4) and saturated brine (5 mL), dried over anhydrous sodium sulfate, and filtered under vacuum. The filtrate was concentrated under reduced pressure, and the crude product was purified by silica gel column chromatography (DCM: MeOH = 100:1 ∼ 20:1) to obtain a product (40.0 mg, yield: 15.7%).

¹H NMR (400 MHz, DMSO-*d*₆) δ(ppm): 13.46 (s, 1H), 8.82 (s, 1H), 8.51-8.50 (d, 1H), 8.45-8.43 (d, 1H), 8.39-8.38 (d, 1H), 8.23 (s, 1H), 7.88-7.85 (m, 1H), 7.62-7.60 (t, 2H), 7.55 (s, 1H), 7.42 (s, 1H), 7.28-7.26 (d, 2H), 6.57-6.56 (d, 1H), 4.28-4.24 (t, 2H), 3.96-3.95 (d, 6H), 2.92-2.89 (t, 1H), 2.83-2.79 (m, 2H), 2.62-2.57 (t, 2H), 2.37 (s, 3H), 2.13-2.11 (t, 2H), 1.96-1.94 (d, 2H).

Molecular formula: C₃₅H₃₄N₄O₅S Molecular weight: 622.74 LC-MS (Pos, *m*/*z)=* 623.45[M+H]⁺.

### Example 35: synthesis of N-(5-((2-amino-6,7-dimethoxyquinolin-4-yl) oxy)pyridin-2-yl)-4-oxo-1-((tetrahydro-2H-pyran-4-yl)methyl)-5-(p-tolyl)-1,4-dihydropyridin-3-carboxamide (compound 10)

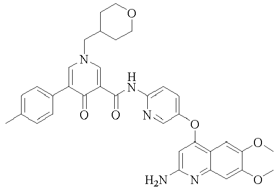

### Step 1: synthesis of 4-chloro-6,7-dimethoxyquinoline 1-oxide

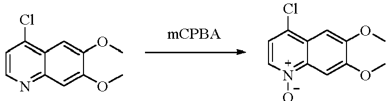

4-chloro-6,7-dimethoxyquinoline (10.0 g, 44.71 mmol) was added to tetrahydrofuran (200 mL), and the resulting solution was cooled to 0 °C. mCPBA (17.29 g, 70.25 mmol) was added, and the reaction solution was slowly warmed from 0 °C to room temperature and stirred overnight. Solids were precipitated, and the mixture was filtered under vacuum. The filter cake was collected, and the filtrate was concentrated under reduced pressure, slurried with ethyl acetate, and filtered under vacuum. The filter cakes were combined to obtain a product (5.0 g, yield: 46.6%).

### Step 2: synthesis of 2-bromo-4-chloro-6,7-dimethoxyquinoline

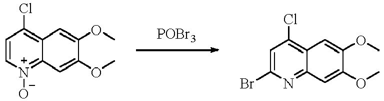

4-chloro-6,7-dimethoxyquinoline 1-oxide (5.0 g, 20.86 mmol) and phosphoryl bromide (17.94 g, 62.59 mmol) were dissolved in tetrahydrofuran (40 mL), and the reaction solution was stirred overnight at 60 °C. After the reaction was completed, as detected by TLC, the reaction solution was concentrated. The crude product was purified by silica gel column chromatography (PE: EA = 30:1 ∼ 15:1) to obtain a product (7.0 g, yield: 93.3%).

### Step 3: synthesis of 4-chloro-6,7-dimethoxy-N-(4-methoxybenzyl) quinolin-2-amine

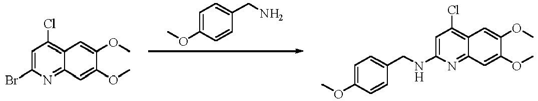

2-bromo-4-chloro-6,7-dimethoxyquinoline (4.4 g, 14.6 mmol), *p*-methoxybenzylamine (2.0 g, 14.6 mmol), potassium *tert*-butoxide (1.64 g, 14.6 mmol), dppf (161.03 mg, 0.292 mmol) and tetrakis(triphenylphosphine) alladium (168.9 mg, 0.146 mmol) were added to a mixture of tetrahydrofuran (45 mL) and toluene (45 mL), and the reaction solution was heated to 110 °C and reacted at reflux overnight under a nitrogen atmosphere. After the reaction was completed, as monitored by TLC, the reaction solution was concentrated under reduced pressure. Water and ethyl acetate were added, and the liquid was separated. Ethyl acetate was added to the aqueous phase for extraction, and the organic phases were combined, washed with water and then with saturated brine, dried over anhydrous sodium sulfate, filtered and concentrated. The crude product was purified by silica gel column chromatography (PE: EA = 15:1 ∼ 5:1, DCM: MeOH = 200:1 ∼ 100:1) to obtain a product (2.5 g crude).

### Step 4: synthesis of 4-((6-bromopyridin-3-yl)oxy)-6,7-dimethoxy-N-(4-ethoxybenzyl)uinolin-2-amine

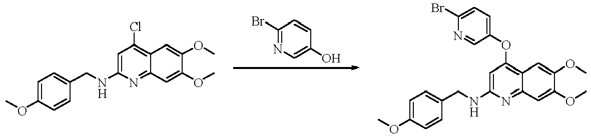

4-chloro-6,7-dimethoxy-*N*-(4-methoxybenzyl)quinolin-2-amine (2.5 g), 6-bromopyridin-3-ol (1.34 g, 7.68 mmol) and DMAP (1.28 g, 10.5 mmol) were added to diphenyl ether (10 mL), and the reaction solution reacted overnight at 140 °C. After the reaction was completed, as detected by TLC, the reaction solution was concentrated under reduced pressure. The crude product was purified by silica gel column chromatography (PE: EA = 15:1 ∼ 5:1, DCM: MeOH = 200:1 ∼ 20:1) to obtain a product (650.0 mg, two-step yield: 11.2%).

### Step 5: synthesis of 4-((6-aminopyridin-3-yl)oxy)-6,7-dimethoxy--(4-ethoxybenzyl)quinolin-2-amine

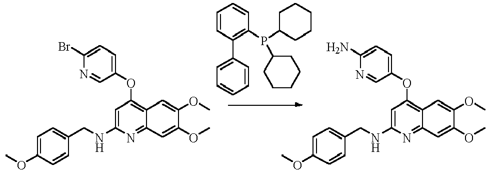

4-((6-bromopyridin-3-yl)oxy)-6,7-dimethoxy-*N*-(4-methoxybenzyl)quinolin-2-amine (300.0 mg, 0.605 mmol), 2-(dicyclohexylphosphino)biphenyl (21.4 mg, 0.061 mmol) and LiHMDS (6 mL, 10.5 mmol) were added to a microwave tube, and the reaction solution reacted at 100 °C for 2 hrs under microwaves and a nitrogen atmosphere. After the reaction was completed, as detected by TLC, the pH was adjusted to 6-7 with a saturated ammonium chloride aqueous solution. Ethyl acetate was added for extraction, and the organic phases were combined, washed with water and then with saturated brine, dried over anhydrous sodium sulfate, filtered and concentrated. The crude product was purified by silica gel column chromatography (DCM: MeOH = 150:1 ∼ 20:1) to obtain a product (245.0 mg, yield: 93.9%).

### Step 6: synthesis of N-(5-((6,7-dimethoxy-2-((4-methoxybenzyl)amino) uinolin-yl)oxy)pyridin-2-yl)-4-oxo-1-((tetrahydro-2H-pyran-4-yl)methyl)-5-(p-tolyl)-1,4-dihydropyridin-3 -carboxamide

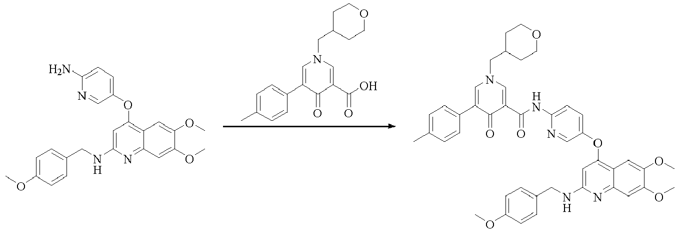

4-oxo-1-((tetrahydro-2*H*-pyran-4-yl)methyl)-5-(*p*-tolyl)-1,4-dihydropyridin-3-carboxylic acid (149.4 mg, 0.457 mmol) was dissolved in DMF (3 mL), and the resulting solution was cooled to 0 °C under an ice bath. HATU (237.0 mg, 0.623 mmol) and triethylamine (139.8 mg, 1.25 mmol) were added, and the reaction solution was stirred at 0 °C for 30 min. 4-((6-aminopyridin 3-yl)oxy)-6,7-dimethoxy-*N*-(4-methoxybenzyl)quinolin-2-amine (180.0 mg, 0.415 mmol) was added, and the reaction solution was stirred overnight at room temperature. After the reaction was completed, as detected by TLC, the reaction solution was concentrated under reduced pressure. The concentrate was dissolved in ethyl acetate (10 mL), washed successively with a saturated sodium bicarbonate aqueous solution (5 mL), a saturated ammonium chloride aqueous solution (5 mL), water (5 mL × 4) and saturated brine (5 mL), dried over anhydrous sodium sulfate, and filtered under vacuum. The filtrate was concentrated under reduced pressure, and the crude product was purified by silica gel column chromatography (DCM: MeOH = 80:1 ∼ 40:1) to obtain a product (300.0 mg, yield 88.6%).

### Step 7: synthesis of N-(5-((2-amino-6,7-dimethoxyquinolin-4-yl)oxy) yridin-2-yl)-4-oxo-1-((tetrahydro-2H-pyran-4-yl)methyl)-5-(p-tolyl)-1,4-dihydropyridin-3 -carboxamide

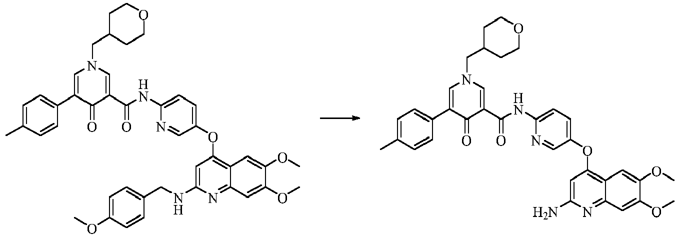

*N*-(5-((6,7-dimethoxy-2-((4-methoxybenzyl)amino)quinolin-4-yl)oxy)pyridin-2-yl)-4-oxo-1-((tetrahydro-2*H*-pyran-4-yl)methyl)-5-(*p*-tolyl)-1,4-dihydropyridin-3 -carboxamide (180.0 mg, 0.243 mmol) was added to a mixture of TFA (5 mL) and DCM (1 mL), and the reaction solution was stirred overnight at room temperature. After the reaction was completed, as detected by TLC, the pH was adjusted to 7-8 with a saturated sodium bicarbonate aqueous solution, and dichloromethane was added for extraction. The organic phases were combined, washed successively with water (5 mL) and saturated brine (5 mL), dried over anhydrous sodium sulfate, and filtered under vacuum. The filtrate was concentrated under reduced pressure, and the crude product was purified by silica gel column chromatography (DCM : MeOH = 80:1 ∼ 20:1) to obtain a product (80.0 mg, yield: 52.9%).

¹H NMR (400 MHz, DMSO-*d*₆) δ(ppm): 13.48 (s, 1H), 8.75-8.74 (d, 1H), 8.46-8.44 (d, 1H), 8.39-8.38 (d, 1H), 8.18 (s, 1H), 7.89-7.87 (d, 1H), 7.61-7.59 (d, 2H), 7.39 (s, 1H), 7.28-7.26 (d, 2H), 7.00 (s, 1H), 6.58 (s, 2H), 5.85 (s, 1H), 4.13-4.11 (d, 2H), 3.88-3.86 (d, 6H), 3.28-3.25 (d, 2H), 2.36-3.32 (d, 3H), 2.11 (s, 1H), 1.48-1.46 (d, 2H), 1.36-1.30 (m, 2H), 1.28-1.11 (m, 2H).

Molecular formula: C₃₅H₃₅N₅O₆ Molecular weight: 621.69 LC-MS(Pos, *m*/*z*)=622.4[M+H]⁺

### Example 36: synthesis of N-(5-((6,7-dimethoxyquinolin-4-yl)oxy)pyridin 2yl)-2-oxo-1-((tetrahydro-2H-pyran-4-yl)methyl)-5-(p-tolyl)-1,2-dihydropyridin-3-carboxamide (compound 13)

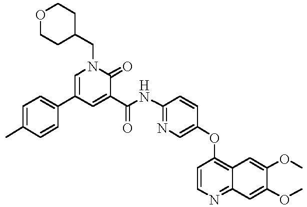

### Step 1: synthesis of N-(5-((6,7-dimethoxyquinolin-4-yl)oxy)pyridin--yl) 2-oxo-1-((tetrahydro-2H-pyran-4-yl)methyl)-5-(p-tolyl)-1,2-dihydropyridin-3-carboxamide

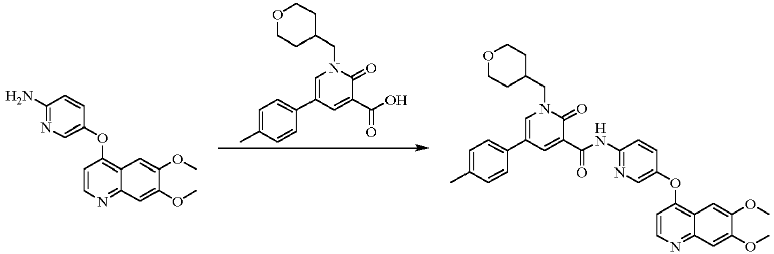

The intermediate 2-oxo-1-((tetrahydro-2*H*-pyran-4-yl)methyl)-5-(*p*-tolyl) 12-ihydropyridin-3-carboxylic acid (100 mg, 0.305 mmol, 1.0 eq) was dissolved in *N, N-*dimethylformamide (3 mL), then triethylamine (93 mg, 0.915 mmol, 3.0 eq) and HATU (174 mg, 0.457 mmol, 1.5 eq) were added, and the reaction solution was stirred at room temperature for 1 hr. Then the intermediate 5-((6,7-dimethoxyquinolin-4-yl)oxy)pyridin-2-amine (91 mg, 0.305 mmol, 1.0 eq) was added, and the reaction solution was further stirred at room temperature for 1 hr. After the reaction was completed, as detected by LC-MS, the reaction solution was poured into water (30 mL), and ethyl acetate (30 mL × 3) was added for extraction. The organic phases were combined, dried and filtered. The filtrate was concentrated, and the crude product was purified by silica gel column chromatography (DCM: MeOH = 50:1 ∼ 10:1) to obtain a product (40 mg, yield: 21%).

¹HNMR (400 MHz, DMSO-*d*₆) δ(ppm): 12.72 (s, 1H), 8.77 (s, 1H), 8.41-8.55 (m, 4H), 8.29-8.30 (m, 1H), 7.86-7.88 (m, 3H), 7.42 (s, 1H), 7.31-7.33 (m, 2H), 6.58 (s, 1H), 4.12 (s, 2H), 3.96 (s, 6H), 3.87 (s, 2H), 3.24-3.27 (m, 2H), 2.36 (s, 3H), 2.20 (s, 1H), 1.36-1.52 (m, 5H).

Molecular formula: C₃₅H₃₄N₄O₆ Molecular weight: 606.68 LC-MS(Pos, *m*/*z*)=607.24[M+H]⁺

### Example 37: synthesis of N-(5-((6,7-dimethoxyquinolin-4-yl)oxy)pyridin -2-yl)--oxo-5-((tetrahydro-2H-pyran-4-yl)methyl)-1-(p-tolyl)-1,2-dihydropyridin-3-carboxamide (compound 26)

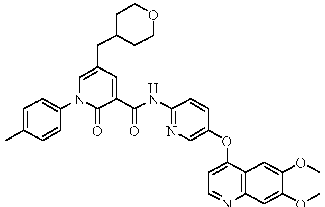

### Steps

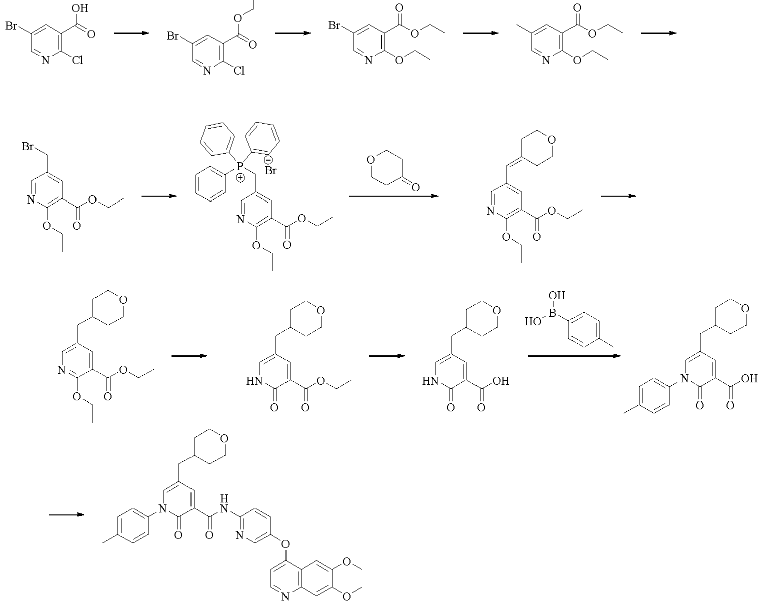

### Step 1: Synthesis of ethyl 5-bromo-2-chloronicotinate

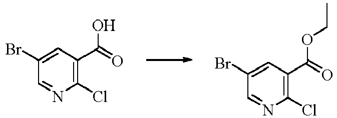

5-bromo-2-chloronicotinic acid (140 g, 0.592 mol) was added to a flask, and ethanol (1.4 L) was added. The resulting solution was cooled to 0 °C under an ice bath, and then thionyl chloride (70.91 g, 5.9 mol) was added. The reaction solution was heated to reflux for 6 hrs. After the reaction was completed, as detected by TLC, the reaction solution was poured into ice water, and EA (1,000 mL) was added for extraction. The organic phases were combined, dried over anhydrous sodium sulfate, and concentrated under reduced pressure to obtain a product (200.0 g crude).

### Step 2: synthesis of ethyl 5-bromo-2-ethoxynicotinate

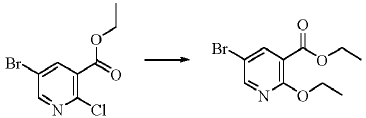

Ethanol (193 mL) was added to a flask and cooled to 0 °C under an ice bath. Sodium (20.27 g, 0.888 mol) was added, and the reaction solution was warmed to room temperature. After the reaction was completed, the reaction solution was cooled to 0 °C under an ice bath. Then ethyl 5-bromo-2-chloronicotinate (193.0 g crude) was added, and the reaction solution was slowly warmed to room temperature and reacted overnight. After the reaction was completed, as detected by TLC, the reaction solution was filtered under vacuum. The filter cake was washed with ethanol, and the filtrate was concentrated under reduced pressure, slurried with methyl *tert-butyl* ether, and filtered under vacuum. The filtrate was concentrated to obtain a product (68.0 g, yield: 43.4%).

### Step 3: synthesis of ethyl 2-ethoxy-5-methylnicotinate

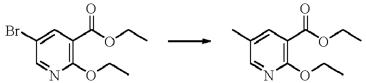

Ethyl 5-bromo-2-ethoxynicotinate (64.8 g, 0.236 mmol), trimethylboroxine (237.5 g, 0.946 mol, mass fraction: 50%), Pd(dppf)Cl₂ (8.78 g, 0.012 mol), sodium bicarbonate (118.96 g, 1.42 mol), 1,4-dioxane (70 mL) and water (25 mL) were added to a flask, and the reaction solution reacted at 110 °C for 4 hrs under a nitrogen atmosphere. After the reaction was completed, as detected by TLC, the reaction solution was cooled to room temperature, and water was added. Then ethyl acetate (300 mL) was added for extraction, and the organic phases were combined, washed with water (100 mL) and then with saturated brine (100 mL), dried over anhydrous sodium sulfate and concentrated. The crude product was purified by silica gel column chromatography (PE: EA = 120:1 ∼ 40:1) to obtain a product (31.0 g, yield: 63%).

### Step 4: synthesis of ethyl 5-(bromomethyl)-2-ethoxynicotinate

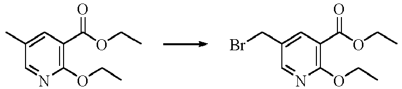

Ethyl 2-ethoxy-5-methylnicotinate (29.3 g, 0.139 mol), NBS (24.96 g, 0.139 mol), azobisisobutyronitrile (23.0 g, 0.139 mol) and carbon tetrachloride (108.5 mL) were added to a flask, and the reaction solution reacted overnight at 80 °C. After the reaction was completed, as detected by TLC, the reaction solution was concentrated. The crude product was purified by silica gel column chromatography (PE: EA = 120:1 ∼ 40:1) to obtain a product (35.1 g, yield: 87.4%).

### Step 5: synthesis of ((6-ethoxy-5-(ethoxycarbonyl)pyridin-3-yl)methyl) triphenylphosphonium bromide

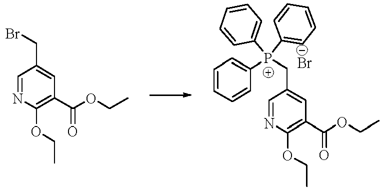

Ethyl 5-(bromomethyl)-2-ethoxynicotinate (28.48 g, 99.25 mmol), triphenylphosphine (52.12 g, 198.5 mmol) and toluene (99.68 mL) were added to a flask, and the reaction solution reacted at 110 °C for 4 hrs. After the reaction was completed, as detected by TLC, the reaction solution was filtered under vacuum to obtain a product (38.5 g, yield: 70.6%).

### Step 6: synthesis of ethyl 2-ethoxy-5-((tetrahydro-4H-pyran-4-ylene) methyl)nicotinate

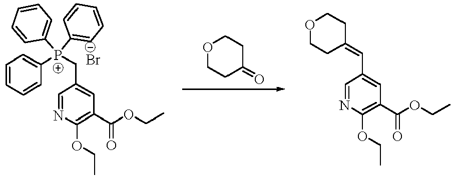

((6-ethoxy-5-(ethoxycarbonyl)pyridin-3-yl)methyl)triphenylphosphonium bromide (34.9 g, 0.063 mol) was added to a flask, then tetrahydrofuran (110 mL) was added, and the resulting solution was cooled to -65 °C under a dry ice/ethanol bath. LiHMDS (95 mL, 1 mol/L) was added, and the reaction solution was stirred at -65 °C for 1 hr. Then tetrahydro-4*H*-pyran-4-one (9.5 g, 0.095 mol) was added, and the reaction solution was slowly warmed from -65 °C to room temperature and reacted overnight. After the reaction was completed, as detected by TLC, the reaction solution was added dropwise to a saturated ammonium chloride aqueous solution (200 mL). The liquid was separated, and ethyl acetate (400 mL) was added to the aqueous phase for extraction. The organic phases were combined, dried over anhydrous sodium sulfate, filtered and concentrated. The crude product was purified by silica gel column chromatography (DCM: MeOH = 60:1 ∼ 10:1) to obtain a product (7.0 g, yield: 38.2%).

### Step 7: synthesis of ethyl 2-ethoxy-5-((tetrahydro-2H-pyran-4-yl)methyl) nicotinate

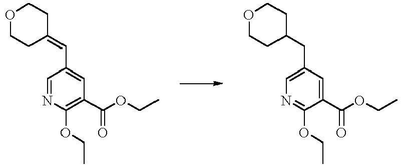

Ethyl 2-ethoxy-5-((tetrahydro-4*H*-pyran-4-ylene)methyl)nicotinate (2.0 g, 6.86 mmol, 1.0 eq) was dissolved in methanol (6.0 mL), and Pd/C (150.0 mg) was added. Then hydrogen was charged to replace by evacuation, and the reaction solution reacted overnight at room temperature. After the reaction was completed, as detected by TLC, the reaction solution was filtered, and the filtrate was concentrated under reduced pressure to obtain a product (2.0 g, yield: 99.5%).

### Step 8: synthesis of ethyl 2-oxo-5-((tetrahydro-2H-pyran-4-yl)methyl)-1, 2-dihydropyridin-3 -carboxylate

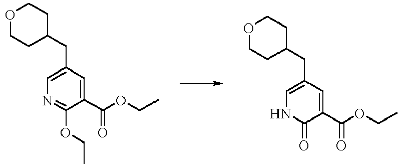

Ethyl 2-ethoxy-5-((tetrahydro-2*H*-pyran-4-yl)methyl)nicotinate (2.0 g, 6.82 mmol) was dissolved in toluene (15.0 mL), then *p*-toluenesulfonic acid (5.835 g, 30.68 mmol) was added, and the reaction solution was heated to 110 °C and reacted overnight. After the reaction was completed, as detected by TLC, the reaction solution was concentrated under reduced pressure. The crude product was purified by silica gel column chromatography (DCM : MeOH = 100:1 ∼ 20:1) to obtain a product (1.5 g, yield: 82.9%).

### Step 9: synthesis of 2-oxo-5-((tetrahydro-2H-pyran-4-yl)methyl)-1,2- dihydropyridin-3-carboxylic acid

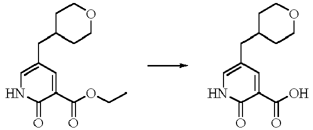

Ethyl 2-oxo-5-((tetrahydro-2*H*-pyran-4-yl)methyl)-1,2-dihydropyridin-3- carboxylate (1.5 g, 5.65 mmol) was dissolved in tetrahydrofuran (6.0 mL), then a 1 mol/L lithium hydroxide aqueous solution (3.0 mL) was added, and the reaction solution was heated to 70 °C and reacted for 3 hrs. After the reaction was completed, as detected by TLC, the reaction solution was concentrated under reduced pressure to remove the solvent. Water (10.0 mL) was added, and the pH was adjusted to 3-4. The resulting solution was filtered under vacuum, and the filter cake was dried at 50 °C to obtain a product (980.0 mg, yield: 73.1%).

### Step 10: synthesis of 2-oxo-5-((tetrahydro-2H-pyran-4-yl)methyl)-1-(p-tolyl)-1,2-dihydropyridin-3-carboxylic acid

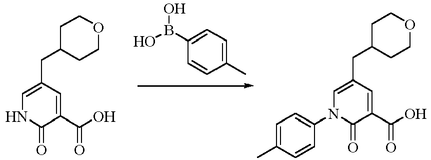

2-oxo-5-((tetrahydro-2*H*-pyran-4-yl)methyl)-1,2-dihydropyridin-3 -carboxylic acid (350.0 mg, 1.48 mmol),*p*-tolylboronic acid (602.0 mg, 4.43 mmol), pyridine (933.4 mg, 11.80 mmol) and triethylamine (746.3 mg, 7.38 mmol) were dissolved in dichloromethane (20.0 mL), then copper acetate (589.0 mg, 2.95 mmol) was added, and the reaction solution was stirred overnight at room temperature. After the reaction was completed, as detected by TLC, the reaction solution was filtered, and the filtrate was concentrated under reduced pressure. The crude product was purified by silica gel column chromatography (DCM : MeOH = 200:1 ∼ 40:1) to obtain a product (325.0 mg, yield: 67.3%).

### Step 11: synthesis of N-(5-((6,7-dimethoxyquinolin-4-yl)oxy)pyridin-2-yl)-2-oxo-5-((tetrahydro-2H-pyran-4-yl)methyl)-1-(p-tolyl)-1,2-dihydropyridin-3 -carboxamide

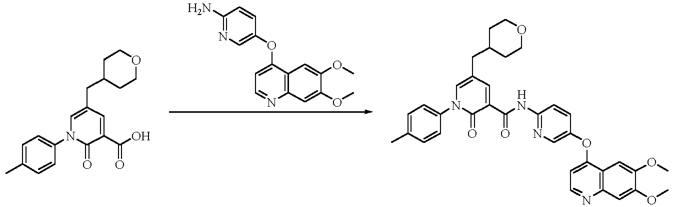

2-oxo-5-((tetrahydro-2*H*-pyran-4-yl)methyl)-1-(*p*-tolyl)-1,2-dihydropyridin-3 -carboxylic acid (325.0 mg, 0.993 mmol) and *N, N*-diisopropylethylamine (385.0 mg, 2.98 mmol) were dissolved in *N, N*-dimethylformamide (2.0 mL), and the resulting solution was cooled to 0 °C. Under a nitrogen atmosphere, HATU (566.2 mg, 1.49 mmol, 1.5 eq) was added, and the reaction solution was stirred at 0 °C for 0.5 hr. 5-((6,7-dimethoxyquinolin-4-yl)oxy)pyridine - 2-amine (295.0 mg, 0.993 mmol, 1.0 eq) was added, and 4 hrs later, the reaction was completed, as detected by TLC. Ethyl acetate (30.0 mL) was added, and the organic phase was washed with a saturated sodium bicarbonate aqueous solution (2.0 mL), a saturated ammonium chloride solution (2.0 mL) and water (2.0 mL × 4), dried, and concentrated under reduced pressure. The crude product was purified by preparative thin-layer chromatography (DCM:MeOH = 20:1) to obtain a product (320.0 mg, yield: 53.1%).

¹H NMR(400 MHz, DMSO-*d*₆) δ(ppm): 12.58 (s, 1H), 8.50-8.49 (m, 2H), 8.44-8.42 (d, 1H), 8.37-8.36 (m, 1H), 7.95-7.94 (m, 1H), 7.87-7.84 (m, 1H), 7.53 (s, 1H), 7.42-7.36 (m, 5H), 6.57-6.55 (d, 1H), 3.96-3.94 (d, 6H), 3.86-3.82 (m, 2H), 3.30-3.24 (m, 2H), 2.52-2.47 (m, 2H), 2.41 (m, 3H), 1.85-1.70 (m, 1H), 1.59-1.55 (m, 2H), 1.24-1.20 (m, 2H).

Molecular formula: C₃₅H₃₄N₄O₆ Molecular weight: 606.68 LC-MS(Pos, *m*/*z*)=607.44[M+H]⁺.

### Example 38: synthesis of N-(5-((6,7-dimethoxyquinolin-4-yl)oxy)pyridin-2-yl)-5-(4-fluorophenyl)-4-oxo-1-(tetrahydro-2H-pyran-4-yl)-1,4-dihydropyridin-3 -carboxamide (compound 27)

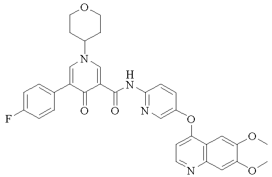

### Step 1: synthesis of ethyl 5-(4-fluorophenyl)-4-oxo-1-(tetrahydro-2H-pyran-4-yl)-1,4-dihydropyridin-3 -carboxylate

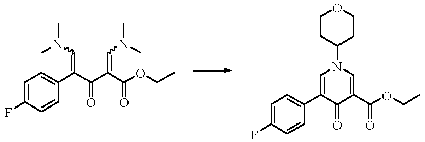

Ethyl 5-(dimethylamino)-2-((dimethylamino)methylene)-4-(4-fluorophenyl) -3-oxopentan-4-enoate (5.96 g, 17.84 mmol, 1.0 eq) was dissolved in ethanol (40.0 mL), then tetrahydro-2*H-*pyran-4-amine (2.71 g, 26.76 mmol, 1.5 eq) was added, and the reaction solution was heated to 10 °C and reacted at reflux for 3 hrs. After the reaction was completed, as detected by TLC, the reaction solution was concentrated under reduced pressure. The crude product was purified by silica gel column chromatography (PE: EA = 50:1 ∼ 1:1) to obtain a product (2.0 g, yield: 32.5%).

### Step 2: synthesis of 5-(4-fluorophenyl)-4-oxo-1-(tetrahydro-2H-pyran-4-yl)-1,4-dihydropyridin-3-carboxylic acid

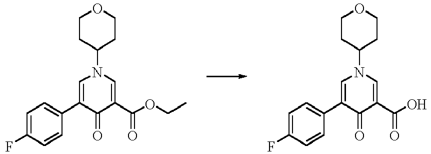

Ethyl 5-(4-fluorophenyl)-4-oxo-1-(tetrahydro-2*H*-pyran-4-yl)-1,4-dihydropyridin-3-carboxylate (2.0 g, 5.79 mmol, 1.0 eq) was dissolved in tetrahydrofuran (10.0 mL), then a 1 mol/L lithium hydroxide aqueous solution (10.0 mL) was added, and the reaction solution was stirred at room temperature for 0.5 hr. After the reaction was completed, as detected by TLC, the reaction solution was concentrated under reduced pressure, and pH was adjusted to 5-6. A large number of solids were precipitated, filtered out under vacuum, and dried to obtain a product (1.6 g, yield: 87.1%).

### Step 3: synthesis of N-(5-((6,7-dimethoxyquinolin-4-yl)oxy)pyridin-2-yl)-5-(4-fluorophenyl)-4-oxo-1-(tetrahydro-2H-pyran-4-yl)-1,4-dihydropyridin-3-carboxamide

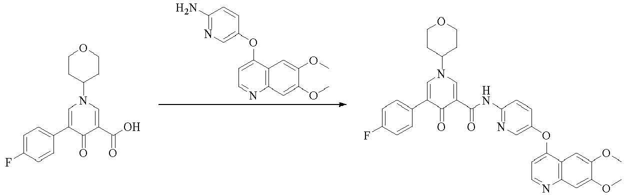

5-(4-fluorophenyl)-4-oxo-1-(tetrahydro-2*H*-pyran-4-yl)-1,4-dihydropyridin-3-carboxylic acid (150.0 mg, 0.47 mmol, 1.0 eq) andN, *N*-diisopropylethylamine (183.0 mg, 1.42 mmol, 3.0 eq) were dissolved in *N, N*-dimethylformamide (1.5 mL), and the resulting solution was cooled to 0 °C. Under a nitrogen atmosphere, HATU (270.0 mg, 0.709 mmol, 1.5 eq) was added, and the reaction solution was stirred at 0 °C for 0.5 hr. 5-((6,7-dimethoxyquinolin-4-yl)oxy)pyridin-2-amine (140.0 mg, 0.473 mmol, 1.0 eq) was added, and the reaction solution was slowly warmed to room temperature. 1 hr later, the reaction was completed, as detected by TLC. The reaction solution was concentrated under reduced pressure, and ethyl acetate (20.0 mL) was added. The organic phase was washed with a saturated sodium bicarbonate aqueous solution (2.0 mL) and then with water (2.0 mL), dried over anhydrous sodium sulfate, and concentrated under reduced pressure. The crude product was first purified by preparative thin-layer chromatography (DCM: MeOH = 18:1), and then purified by silica gel column chromatography (DCM : MeOH = 100:1 ∼ 50:1) to obtain a product (160.0 mg, yield: 56.7%).

¹HNMR (400 MHz, DMSO-*d*₆) δ(ppm): 13.37 (s, 1H), 8.80-8.79 (m, 1H), 8.51-8.49 (d, 1H), 8.45-8.37 (m, 2H), 8.30-8.29 (d, 1H), 7.87-7.76 (m, 3H), 7.55 (s, 1H), 7.42 (s, 1H), 7.30-7.26 (m, 2H), 6.57-6.56 (d, 1H), 4.66-4.56 (m, 1H), 4.05-4.02 (m, 2H), 3.96-3.95 (m, 6H), 3.44-3.42 (m, 2H), 2.13-2.00 (m, 4H).

Molecular formula: C₃₃H₂₉FN₄O₆ Molecular weight: 596.62 LC-MS(Pos, *m*/*z*)=597.42[M+H]⁺.

### Example 39: Synthesis of N-(5-((6,7-dimethoxyquinolin-4-yl)oxy)-3-fluorophenyl)-4-oxo-1-(tetrahydro-2H-pyran-4-yl)-5-p-tolyl-1,4-dihydropyridazin-3-carboxamide (compound 28)

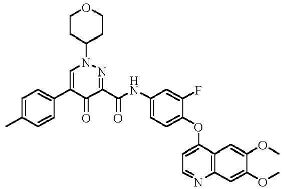

### Step 1: Synthesis of 4-(2-fluoro-4-nitrophenoxy)-6,7-dimethoxyquinoline

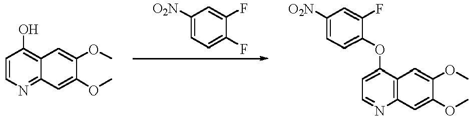

6,7-dimethoxyquinolin-4-ol (25.00 g, 121.83 mmol, 1.0 eq), 1,2-difluoro-4-nitrobenzene (29.10 g, 182.74 mmol, 1.5 eq) and K₂CO₃ (33.70 g, 243.65 mmol, 2.0 eq.) were added to DMF (250 mL), and the reaction solution was stirred overnight at 80 °C under a nitrogen atmosphere. After the reaction was completed, as detected by TLC, the reaction solution was filtered. The filter cake was rinsed with dichloromethane, and the filtrate was concentrated under reduced pressure and added with dichloromethane (100 mL). The organic phase was washed with water (40 mL × 4) and saturated brine (40 mL), dried over anhydrous sodium sulfate, and filtered under vacuum. The filtrate was concentrated under reduced pressure, and the crude product was purified by silica gel column chromatography (PE: EA = 1:4 ∼ 1:9) to obtain a product (9.00 g, yield: 21.4 %).

### Step 2: synthesis of 4-((6,7-dimethoxyquinolin-4-yl)oxy)-3-fluoroaniline

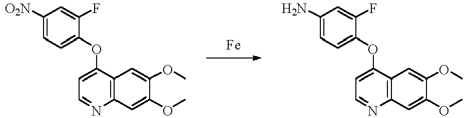

4-(2-fluoro-4-nitrophenoxy)-6,7-dimethoxyquinoline (5.00 g, 14.52 mmol, 1.0 eq), reduced iron powder (4.70 g, 84.21 mmol, 5.8 eq) and NH₄Cl (9.32 g, 174.24 mmol, 12.0 eq) were added to a mixed solvent of ethanol (100 mL) and water (40 mL), and the reaction solution was stirred at 80 °C for 2 hrs. After the reaction was completed, as detected by TLC, the reaction solution was immediately filtered. The filter cake was rinsed with dichloromethane, and the filtrate was concentrated under reduced pressure. Dichloromethane (20 mL × 3) was added to the aqueous phase for extraction, and the organic phases were combined, washed with saturated brine (20 mL), dried over anhydrous sodium sulfate, filtered under vacuum, and concentrated under reduced pressure to obtain a product (4.50 g, yield: 98.6 %).

### Step 3: Synthesis of N-(5-((6,7-dimethoxyquinolin-4-yl)oxy)-3-fluorophenyl) -4-oxo-1-(tetrahydro-2H-pyran-4-yl)-5-p-tolyl-1,4-dihydropyridazin-3 -carboxamide

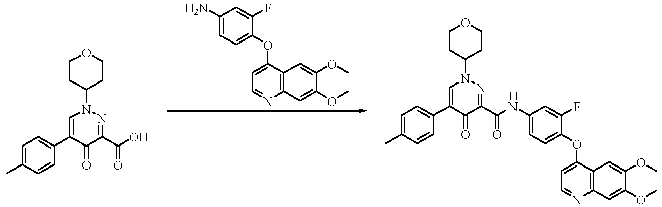

4-oxo-1-(tetrahydro-2*H*-pyran-4-yl)-5-*p*-tolyl-1,4-dihydropyridazin-3 -carboxylic acid (138.2 mg, 0.44 mmol, 1.0 eq) was dissolved in DMF (2 mL), then HATU (250.8 mg, 0.66 mmol, 1.5 eq) and DIPEA (170.5 mg, 1.32 mmol, 3.0 eq) were added, and the resulting solution was stirred for 10 min. 4-((6,7-dimethoxyquinolin-4-yl)oxy)-3-fluoroaniline (165.7 mg, 0.53 mmol, 1.2 eq) was added, and the reaction solution was stirred at room temperature for 1 hr. After the reaction was completed, as detected by TLC, the reaction solution was concentrated under reduced pressure. The concentrate was dissolved in ethyl acetate (20 mL), washed successively with a saturated sodium bicarbonate aqueous solution (10 mL), a saturated ammonium chloride aqueous solution (10 mL), distilled water (10 mL × 3) and saturated brine (10 mL), dried over anhydrous sodium sulfate, and filtered under vacuum. The filtrate was concentrated under reduced pressure, and the crude product was purified by silica gel column chromatography (PE: EA = 2:1) to obtain a product (122 mg, yield: 45.5 %).

¹H NMR (400 MHz, DMSO-*d*₆) δ(ppm): 12.40 (s, 1H), 8.89 (s, 1H), 8.50-8.49 (d, 1H), 8.07-8.03 (q, 1H), 7.83-7.81 (d, 2H), 7.58-7.57 (d, 2H), 7.55-7.47 (q, 1H), 7.42 (s, 1H), 7.32-7.30 (d, 2H), 6.52-6.51 (d, 1H), 4.71-4.63 (m, 1H), 4.07-4.04 (q, 2H), 3.96 (s, 6H), 3.50-3.45 (q, 2H), 2.37 (s, 3H), 2.14-2.06 (m, 4H).

Molecular formula: C₃₄H₃₁FN₄O₆ Molecular weight: 610.64 LC-MS(Pos, *m*/*z)=* 611.42[M+H]⁺.

### Example 40: synthesis of N-(5-([1,3]dioxolo[4,5-g]quinolin-8-yloxy) pyridin-2-yl)-4-oxo-1-(tetrahydro-2H-pyran-4-yl)-5-(p-tolyl)-1,4-dihydropyridazin-3 -carboxamide (compound 29)

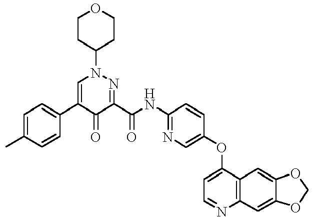

### Steps

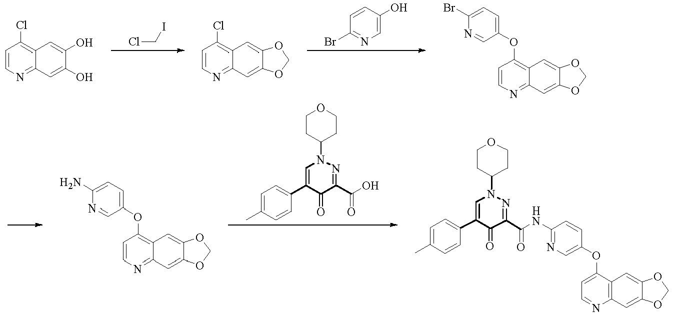

### Step 1: synthesis of 8-chloro-[1,3]dioxolo[4,5-g]quinoline

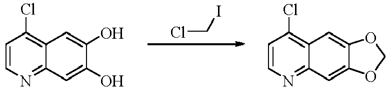

4-chloroquinolin-6,7-diol (2.67 g, 13.0 mmol, 1.0 eq) was dissolved in DMF (10 mL), then cesium carbonate (6.67 g, 20.0 mmol, 1.5 eq) and chloroiodomethane (3.61 g, 20.0 mmol, 1.5 eq) were added, and the reaction solution reacted overnight at 110 °C. After the reaction was completed, as detected by TLC, the reaction solution was concentrated under reduced pressure. The crude product was purified by silica gel column chromatography (dichloromethane: methanol = 100:1) to obtain a product (746.6 mg, yield: 28.6%).

### Step 2: synthesis of 8-((6-bromopyridin-3-yl)oxy)-[1,3]dioxolo[4,5-g] quinoline

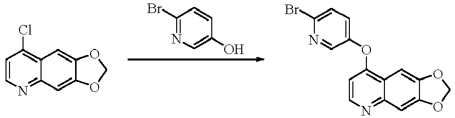

8-chloro-[1,3]dioxolo[4,5-g]quinoline (746.6 mg, 3.59 mmol, 1.0 eq) was dissolved in toluene (7 mL), then 6-bromopyridin-3-ol (750.8 mg, 4.31 mmol, 1.2 eq) and 4-dimethylaminopyridine (878.6 mg, 7.19 mmol, 2.0 eq) were added, and the reaction solution reacted overnight at 120 °C under a nitrogen atmosphere. After the reaction was completed, as monitored by TLC, the reaction solution was concentrated under reduced pressure. Ethyl acetate (15 mL) and water (20 mL) were added, and the liquid was separated. The organic phase was concentrated, and the crude product was purified by silica gel column chromatography (dichloromethane: methanol = 200:1) to obtain a product (577.3 mg, yield: 48.1%).

### Step 3: synthesis of 5-([1,3]dioxolo[4,5-g]quinolin-8-yloxy)pyridine-2-amine

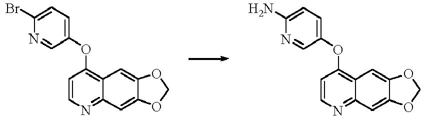

8-((6-bromopyridin-3-yl)oxy)-[1,3]dioxolo[4,5-g]quinoline (577.3 mg, 1.67 mmol, 1.0 eq.) was dissolved in Li-HMDS (4.0 mL), then [1,1'-biphenyl]-2-dicyclohexylphosphine (58.5 mg, 0.16 mmol, 0.1 eq.) and Pd₂(dba)₃ (76.5 mg, 0.08 mmol, 0.05 eq.) were added, and the reaction solution was heated to 100 °C and stirred for 2 hrs under microwaves. After the reaction was completed, as monitored by TLC, ethyl acetate (15 mL) and a saturated ammonium chloride aqueous solution (20 mL) were added, and the reaction system were separated. The organic phase was dried over anhydrous sodium sulfate, and purified by column chromatography (dichloromethane: methanol = 100:1 ∼ 80:1 ∼ 60:1 ∼ 50:1) to obtain 5-([1,3]dioxolo[4,5-g] quinolin-8-yloxy)pyridin-2-amine (184.5 mg, yield: 39.3%).

### Step 4: synthesis of N-(5-([1,3]dioxolo[4,5-g]quinolin-8-yloxy)pyridine-2-yl)-4-oxo-1-(tetrahydro-2H-pyran-4-yl)-5-(p-tolyl)-1,4-dihydropyridazin-3 -carboxamide

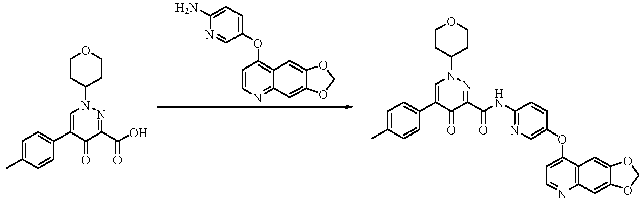

4-oxo-1-(tetrahydro-2*H*-pyran-4-yl)-5-(*p*-tolyl)-1,4-dihydropyridazin-3- carboxylic acid (206.1 mg, 0.65 mmol, 1.0 eq.) was added to DMF (2.5 mL), then HATU (373.5 mg, 0.98 mmol, 1.5 eq.) and DIPEA (253.7 mg, 1.96 mmol, 3.0 eq.) were added, and the reaction solution was stirred for 1.5 hrs under an ice bath. 5-([1,3]dioxolo[4,5-g]quinolin-8-yloxy)pyridin-2-amine (184.5 mg, 0.65 mmol, 1.0 eq.) was added, and the reaction solution was gradually warmed to room temperature and stirred overnight. A large number of raw materials were left, as detected by TLC, and the system was concentrated to dryness. Ethyl acetate (15 mL) and a saturated sodium bicarbonate aqueous solution (20 mL) were added, and the resulting solution was separated. The organic phase was washed with a saturated ammonium chloride aqueous solution (20 mL) and then with a saturated sodium chloride aqueous solution (20 mL), dried over anhydrous sodium sulfate, and concentrated to dryness. The concentrate was added to pyridine (2.0 mL), and cooled to 0 °C under an ice bath. Phosphorus oxychloride (0.2 mL) was added, and the resulting reaction solution was stirred at 0 °C for 0.5 hr. After the reaction was completed, as detected by TLC, the reaction solution was concentrated under reduced pressure. Ethyl acetate (20 mL) and water (15 mL) were added, and the liquid was separated. The organic phase was dried over anhydrous sodium sulfate, and purified by column chromatography (dichloromethane : methanol = 100:1 ∼ 80:1 ∼ 60:1 ∼ 50:1) to obtain *N*-(5-([1,3]dioxolo[4,5-g]quinolin-8-yloxy)pyridin-2-yl)-4-oxo-1-(tetrahydro-2*H*-pyran-4-yl)-5-(*p*-tolyl)-1,4-dihydropyridazin-3-carboxamide (114.0 mg, yield: 30.1%).

¹H NMR (400 MHz, DMSO-*d*₆) δ(ppm): 13.11 (s, 1H), 8.91 (s, 1H), 8.39-8.50 (m, 3H), 7.80-7.87 (m, 3H), 7.58 (s, 1H), 7.40 (s, 1H), 7.30-7.32 (m, 2H), 6.63-6.64 (m, 1H), 6.25 (s, 2H), 4.65-4.73 (m, 1H), 4.04-4.07 (m, 2H), 3.45-3.50 (m, 2H), 2.37 (s, 3H), 2.04-2.15 (m, 4H).

Molecular formula: C₃₂H₂₇N₅O₆ Molecular weight: 577.60 LC-MS (Pos, *m*/*z*)=578.34[M+H]⁺.

### Example 41: synthesis of N-(5-((2,3-dihydro-[1,4]dioxino[2,3-g]quinolin -9-yl)oxy)pyridin-2-yl)-4-oxo-1-(tetrahydro-2H-pyran-4-yl)-5-(p-tolyl)-1,4-dihydropyridazin-3-carboxamide (compound 30)

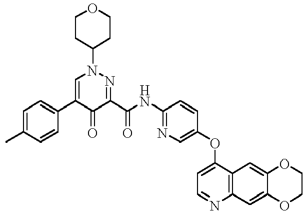

### Step 1: synthesis of 4-chloroquinolin-6,7-diol

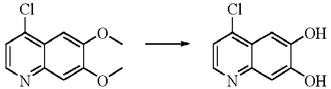

4-chloro-6,7-dimethoxyquinoline (5.0 g, 22.0 mmol, 1.0 eq.) was dissolved in dichloromethane (20 mL), then boron tribromide (56.0 g, 220 mmol, 10.0 eq) was added dropwise at -40 °C, and the reaction solution was gradually warmed to room temperature and reacted overnight. After the reaction was completed, as detected by TLC, the pH was adjusted to 8 with a saturated sodium bicarbonate aqueous solution, and the liquid was separated. n-butanol (40 mL) was added to the aqueous phase for extraction, and the organic phase was dried over anhydrous sodium sulfate, and concentrated under reduced pressure to obtain a product (4.01 g, yield: 95.3%).

### Step 2: synthesis of 9-chloro-2,3-dihydro-[1,4]dioxino[2,3-g]quinoline

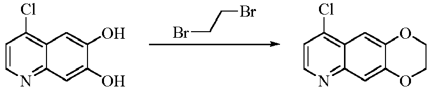

4-chloroquinolin-6,7-diol (2.5 g, 12.7 mmol, 1.0 eq) was dissolved in DMA (15 mL), then cesium carbonate (12.6 g, 38.3 mmol, 3 eq) and 1,2-dibromoethane (7.2 g, 38.3 mmol, 3.0 eq.) were added, and the reaction solution was stirred overnight at 100 °C under a nitrogen atmosphere. After the reaction was completed, as detected by TLC, the reaction solution was concentrated under reduced pressure. Ethyl acetate (100 mL) and water (60 mL) were added, and the liquid was separated. The organic phase was dried over anhydrous sodium sulfate and filtered. The filtrate was concentrated under reduced pressure, and the crude product was purified by silica gel column chromatography (dichloromethane: methanol = 200:1) to obtain a product (670.3 mg, yield: 23.9%).

### Step 3: synthesis of 9-((6-bromopyridin-3-yl)oxy)-2,3-dihydro[1,4] dioxino[2,3-g]quinoline

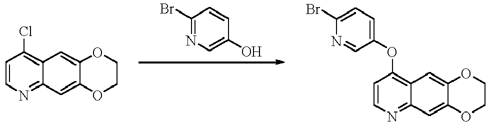

9-chloro-2,3-dihydro-[1,4]dioxino[2,3-g]quinoline (638.0 mg, 2.87 mmol, 1.0 eq) was dissolved in toluene (8.0 mL ), then DMAP (703.4 mg, 5.75 mmol, 2.0 eq) and 6-bromopyridin-3-ol (601.0 mg, 3.45 mmol, 1.2 eq) were added, and the reaction solution was stirred overnight at 110 °C. After the reaction was completed, as detected by TLC, the reaction solution was concentrated under reduced pressure. Ethyl acetate (12 mL) and a saturated sodium chloride aqueous solution (20 mL) were added, and the liquid was separated. The organic phase was dried over anhydrous sodium sulfate, filtered and concentrated. The crude product was purified by silica gel column chromatography (dichloromethane: methanol = 200:1 ∼ 150:1) to obtain a product (460.3 mg, yield: 46.0%).

### Step 4: synthesis of 5-((2,3-dihydro-[1,4]dioxino[2,3-g]quinolin-9-yl) oxy)pyridin-2-amine

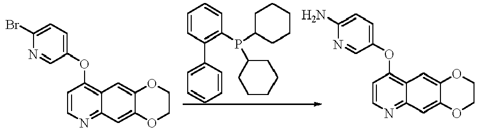

9-((6-bromopyridin-3-yl)oxy)-2,3-dihydro[1,4]dioxino[2,3-g]quinoline (390.0 mg, 1.08 mmol, 1.0 eq) was dissolved in a solution of LiHMDS in tetrahydrofuran (3.0 mL), then 2-(dicyclohexylphosphino)biphenyl (38.0 mg, 0.10 mmol, 0.1 eq) and Pd₂(dba)₃ (49.0 mg, 0.05 mmol, 0.05 eq) were added, and the reaction solution was stirred at 100 °C for 2 hrs under microwaves. After the reaction was completed, as detected by TLC, ethyl acetate (15 mL) and a saturated ammonium chloride aqueous solution (20 mL) were added, and the liquid was separated. The organic phase was dried over anhydrous sodium sulfate, filtered and concentrated. The crude product was purified by silica gel column chromatography (dichloromethane: methanol = 200:1 ∼ 100:1) to obtain a product (234.6 mg, yield: 73.1%).

### Step 5: synthesis of N-(5-((2,3-dihydro-[1,4]dioxino[2,3-g]quinolin-9-yl) oxy)pyridin-2-yl)-4-oxo-1-(tetrahydro-2H-pyran-4-yl)-5-(p-tolyl)-1,4-dihydropyridazin-3-carboxamide

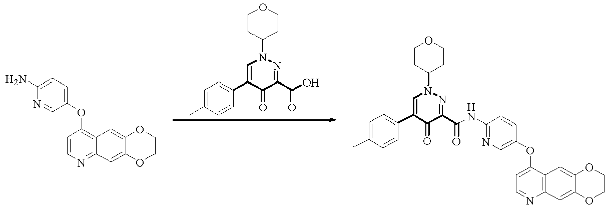

4-oxo-1-(tetrahydro-2*H*-pyran-4-yl)-5-(*p*-tolyl)-1,4-dihydropyridazin-3-carboxylic acid (128.1 mg, 0.40 mmol, 1.0 eq) was added to DMF (3.0 mL), then HATU (232.1 mg, 0.61 mmol, 1.5 eq.) and DIPEA (157.6 mg, 1.22 mmol, 3.0 eq.) were added, and the reaction solution was stirred for 1.5 hrs under an ice bath. 5-((2,3-dihydro-[1,4]dioxino[2,3-*g*]quinolin-9-yl)oxy) pyridine-2-amine (120.4 mg, 0.40 mmol, 1.0 eq) was added, and the reaction solution was warmed to room temperature and stirred overnight. After the reaction was completed, as detected by TLC, the reaction solution was concentrated under reduced pressure. Ethyl acetate (15 mL) and water (20 mL) were added, the liquid was separated. The organic phase was dried over anhydrous sodium sulfate, filtered and concentrated. The crude product was purified by silica gel column chromatography (dichloromethane: methanol = 100:1 ∼ 60:1) to obtain a product (82.0 mg, yield: 34.1%).

¹H NMR (400 MHz, DMSO-*d*₆) δ(ppm): 13.11 (s, 1H), 8.91 (s, 1H), 8.50-8.52 (m, 1H), 8.39-8.43 (m, 2H), 7.81-7.88 (m, 3H), 7.62 (s, 1H), 7.43 (s, 1H), 7.30-7.32 (m, 2H), 6.56-6.57 (m, 1H), 4.67-4.72 (m, 1H), 4.42 (s, 4H), 4.05-4.07 (m, 2H), 3.45-3.50 (m, 2H), 2.12-2.15 (s, 3H), 2.04-2.07 (m, 4H).

Molecular formula: C₃₃H₂₉N₅O₆ Molecular weight: 591.62 LC-MS (Pos, *m*/*z*)=592.35[M+H]⁺.

### Example 42: N-((cis)-4-((6,7-dimethoxyquinolin-4-yl)oxy)cyclohexyl)-4-oxo-1-((tetrahydro-2H-pyran-4-yl)methyl)-5-(p-tolyl)-1,4-dihydropyridin-3 -carboxamide (compound 38)

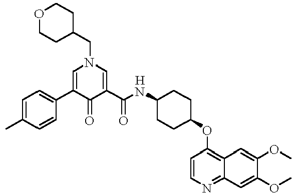

¹HNMR(400MHz, DMSO-*d*₆) δ(ppm): 10.65-10.63 (d, 1H), 8.54-8.50 (m, 2H), 8.08-8.07 (d, 1H), 7.55-7.53 (d, 2H), 7.36 (s, 1H), 7.31 (s, 1H), 7.24-7.22 (d, 2H), 6.98-6.97 (d, 1H), 4.75 (s, 1H), 4.06-4.04 (d, 2H), 3.91-3.90 (d, 6H), 3.86-3.83 (t, 2H), 3.28-3.23(t, 2H), 2.36 (s, 3H) 2.16-2.04 (m, 5H), 1.75-1.72 (d, 2H), 1.55-1.50 (t, 2H), 1.45-1.42 (d, 2H), 1.33-1.24 (m, 3H).

Molecular formula: C₃₆H₄₁N₃O₆ Molecular weight: 611.74 LC-MS(Pos, *m*/*z*)=612.0 [M+H]⁺.

### Example 43: N-((trans)-4-((6,7-dimethoxyquinolin-4-yl)oxy)cyclohexyl)-4-oxol-((tetrahydro-2H-pyran-4-yl)methyl)-5-(p-tolyl)-1,4-dihydropyridin-3-carboxamide (compound 39)

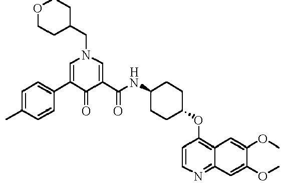

¹HNMR(400MHz, DMSO-*d*₆) δ(ppm): 10.76-10.74 (d, 1H), 8.54-8.50 (m, 2H), 8.07-8.06 (d, 1H), 7.54-7.52 (d, 2H), 7.41 (s, 1H), 7.31 (s, 1H), 7.25-7.23 (d, 2H), 6.98-6.97 (d, 1H), 4.75 (s, 1H), 4.06-4.04 (d, 2H), 3.91-3.90 (d, 6H), 3.88-3.83 (m, 2H), 3.33-3.22(m, 2H), 2.36-2.34 (d, 3H), 1.96-1.74 (m, 9H), 1.44-1.41 (d, 2H), 1.30-1.24 (m, 3H).

Molecular formula: C₃₆H₄₁N₃O₆ Molecular weight: 611.74 LC-MS(Pos, *m*/*z*)=612.0 [M+H]⁺.

### Step 1: synthesis of 4-((1,4-dioxaspiro[4.5]decan-8-yl)oxy)-6,7-dimethoxyquinoline

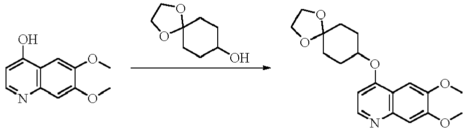

6,7-dimethoxyquinolin-4-ol (1.0 g, 4.8 mmol) and 1,4-dioxaspiro[4.5] decan-8-ol (760.0 mg, 4.8 mmol) were added to a flask, and the resulting solution was cooled to 0 °C under an ice bath. Triphenylphosphine (1.89 g, 7.2 mmol) was added, then DEAD (1.25 g, 7.2 mmol) was added, and the reaction solution was slowly warmed to room temperature and reacted overnight. After the reaction was completed, as detected by TLC, the reaction solution was concentrated under reduced pressure. Ethyl acetate was added, and the organic phase was washed with water and then with a saturated sodium chloride aqueous solution, dried over anhydrous sodium sulfate, filtered and concentrated. The crude product was purified by silica gel column chromatography (DCM : MeOH = 150:1 ∼ 80:1) to obtain a product (1.2 g, yield: 72.3%).

### Step 2: synthesis of 4-((6,7-dimethoxyquinolin-4-yl)oxy)cyclohexan-1-one

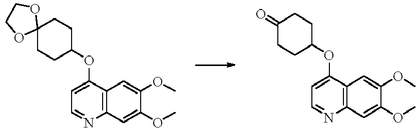

4-((1,4-dioxaspiro[4.5]decan-8-yl)oxy)-6,7-dimethoxyquinoline (700.0 mg, 2.02 mmol) was added to a flask, then methanol (5 mL) and hydrochloric acid (1 mol/L, 2.5 mL) were added, and the reaction solution was stirred at room temperature for 5 hrs. After the reaction was completed, as detected by TLC, the pH was adjusted to 7-8 with a sodium carbonate aqueous solution. Ethyl acetate (20 mL) was added, and the liquid was separated. Ethyl acetate (3 × 10 mL) was added to the aqueous phase for extraction, and the organic phases were combined, dried over anhydrous sodium sulfate, and filtered. The filtrate was concentrated under reduced pressure to obtain a product (400.0 mg, yield: 65.39%).

### Step 3: synthesis of 4-((6,7-dimethoxyquinolin-4-yl)oxy)cyclohexan-1-amine

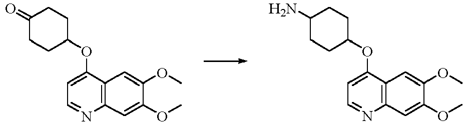

4-((6,7-dimethoxyquinolin-4-yl)oxy)cyclohexan-1-one (400.0 mg, 1.33 mmol) was added to methanol (16 mL) and cooled to 0 °C under an ice bath, and ammonium formate (826.56 mg, 13.12 mmol) was added. Then sodium cyanoborohydride (166.88 mg, 2.66 mmol) was added, and the reaction solution was slowly warmed to room temperature and stirred for 4 hrs. After the reaction was completed, as detected by TLC, the reaction solution was concentrated under reduced pressure. Dichloromethane (20 mL) and a sodium hydroxide aqueous solution (1 mol/L, 5 mL) were added, and the liquid was separated. The organic phase was washed with a sodium hydroxide aqueous solution (1 mol/L), a saturated sodium bicarbonate aqueous solution, water and saturated brine, dried over anhydrous sodium sulfate, and filtered. The filtrate was concentrated under reduced pressure to obtain a product (300 mg, yield: 74.6%).

### Step 4: synthesis of N-((cis)-4-((6,7-dimethoxyquinolin-4-yl)oxy)cyclohexyl)-4-oxo-1-((tetrahydro-2H-pyran-4-yl)methyl)-5-(p-tolyl)-1,4-dihydropyridin-3 -carboxamide and N-((trans)-4-((6,7-dimethoxyquinolin-4-yl)oxy)cyclohexyl)-4-oxo-1-((tetrahydro-2H-pyran-4-yl)methyl)-5-(p-tolyl)-1,4-dihydropyridin-3 -carboxamide

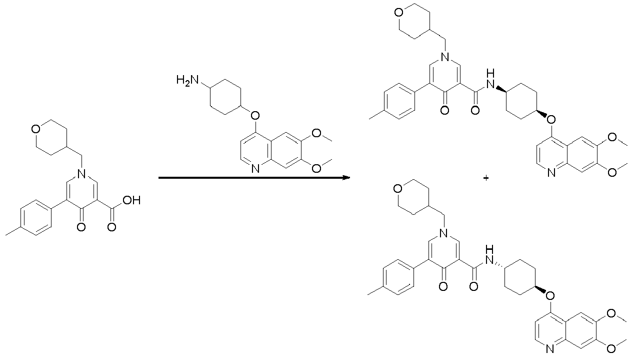

4-oxo-1-((tetrahydro-2*H*-pyran-4-yl)methyl)-5-(*p*-tolyl)-1,4-dihydropyridin-3-carboxylic acid (137.4 mg, 0.42 mmol) was added to DMF (3 mL), and the resulting solution was cooled to 0 °C under an ice bath. Triethylamine (127.5 mg, 1.26 mmol) was added, then HATU (226.6 mg, 0.596 mmol) was added, and the reaction solution was stirred for 30 min. 4-((6,7-dimethoxyquinolin-4-yl)oxy)cyclohexan-1-amine (120.0 mg, 0.397 mmol) was added, and the reaction solution was slowly warmed to room temperature and stirred overnight. After the reaction was completed, as detected by TLC, the reaction solution was concentrated under reduced pressure. The crude product was dissolved in ethyl acetate (10 mL), washed successively with a saturated sodium bicarbonate aqueous solution (5 mL), a saturated ammonium chloride aqueous solution (5 mL), water (5 mL × 4) and saturated brine (5 mL), dried over anhydrous sodium sulfate, and filtered under vacuum. The filtrate was concentrated under reduced pressure, and the crude product was purified by silica gel column chromatography (DCM : MeOH = 100:1 ∼ 20:1) to obtain *N*-((*cis*)-4-((6,7-dimethoxyquinolin-4-yl)oxy) cyclohexyl)-4-oxo-1-((tetrahydro-2*H*-pyran-4-yl)methyl)-5-(*p*-tolyl)-1,4-dihydropyridin-3-carboxamide (21.0 mg, yield: 8.7%) and *N*-((*trans*)-4-((6,7-dimethoxyquinolin-4-yl)oxy)cyclohexyl)-4-oxo-1-((tetrahydro-2*H*-pyran-4-yl)methyl)-5-(*p-*tolyl)-1,4-dihydropyridin-3-carboxamide (38.0 mg, yield: 15.66%).

The present invention can be better understood according to the following experimental examples. However, it is easily understood by those skilled in the art that the content described in the examples are only used to illustrate the present invention, and should not and will not limit the present invention described in detail in the claims.

### Experimental example 1: enzymatic activity assay on compounds of the present invention

### Test samples: the compounds of the present invention, having structures and preparation methods shown above.

### Test method:

### (1) Preparation of compound stock solutions

Compounds were each dissolved in 100% DMSO to prepare stock solutions with a maximum concentration of 500 µM.

### (2) Preparation of compound working solutions

Compound working solutions (50 ×) were prepared by diluting a compound stock solution to a final concentration of 500, 150, 50, 15, 5, 1.5, 0.5, 0.15 and 0.05 µM.

### (3) Preparation of different enzymatic reaction solutions

### a) Axl (h)

An enzyme solution with a final concentration of 1.7 nM was prepared by dissolving Axl (h) enzyme in a mixture of 8 mM MOPS (pH 7.0), 0.2 mM EDTA and 250 µM KKSRGDYMTMQIG. A Mg/ATP mixture of 10 mM magnesium acetate and 10 µM [γ-³³P]-ATP was used to activate the enzymatic reaction.

### b) Mer (h)

An enzyme solution with a final concentration of 3.1 nM was prepared by dissolving Mer (h) enzyme in a mixture of 8 mM MOPS (pH 7.0), 0.2 mM EDTA, 30 mM NaCl and 250 µM GGMEDIYFEFMGGKKK. A Mg/ATP mixture of 10 mM magnesium acetate and 10 µM [γ-³³P]-ATP was used to activate the enzymatic reaction.

### c) Tyro-3 (h)

An enzyme solution with a final concentration of 38 nM was prepared by dissolving Tyro3 (h) enzyme in a mixture of 8 mM MOPS (pH 7.0), 0.2 mM EDTA, 1 mM MnCl₂ and 250 µM KVEKIGEGTYGVVYK. A Mg/ATP mixture of 10 mM magnesium acetate and 10 µM [γ-³³P]-ATP was used to activate the enzymatic reaction.

### d) CSF1R (h)

An enzyme solution with a final concentration of 64 nM was prepared by dissolving CSF1R enzyme in a mixture of 8 mM MOPS (pH 7.0), 0.2 mM EDTA and 250 µM KKSRGDYMTMQIG. A Mg/ATP mixture of 10 mM magnesium acetate and 10 µM [γ-³³P]-ATP was used to activate the enzymatic reaction.

### (4) Enzymatic reaction

A compound working solution was added to a 384-well plate, with final concentrations of 10,000, 3,000, 1,000, 300, 100, 30, 10, 3 and 1 nM, and then different enzymatic reaction solutions prepared according to the above conditions were added. The reaction solutions were incubated at room temperature for 40 min, and then 0.5% phosphoric acid solutions were added to terminate the reaction. 10 µL of the reaction solution was added dropwise to P30 filter paper, washed 4 times with a 0.425% phosphoric acid solution and once with methanol, and then placed in a scintillation counter for detection. Gray analysis was performed on the results with Image J software, and IC₅₀ values were calculated with GraphPad 5.0 software.

The test results are shown in Table 1 below.

**Table 1 Inhibitory activity (-IC₅₀ (nM)) of compounds of the present invention on different kinases**

| Sample | Axl(h) | Mer(h) | Tyro-3(h) | CSF1R(h) |
|---|---|---|---|---|
| Compound 1 | 5 | 2 | 37 | - |
| Compound 2 | 7 | < 1 | 13 | < 200 |
| Compound 4 | 9 | 5 | 178 | < 200 |
| Compound 7 | 20 | 50 | 192 | - |
| Compound 8 | 6 | 73 | 117 | - |
| Compound 9 | 35 | 5 | 28 | - |
| Compound 13 | 23 | 8 | 94 | - |
| Compound 16 | 4 | 3 | 23 | < 200 |
| Compound 17 | 3 | 1 | 14 | < 200 |
| Compound 18 | 2 | 5 | 58 | < 200 |
| Compound 19 | 12 | 4 | 34 | - |
| Compound 20 | 4 | 14 | 84 | < 200 |
| Compound 21 | 19 | 1 | 29 | - |
| Compound 22 | 16 | 5 | 103 | - |
| Compound 23 | 9 | 8 | 33 | - |
| Compound 24 | 9 | 34 | 212 | - |
| Compound 25 | 11 | 15 | 205 | - |
| Compound 26 | 20 | 8 | 48 | < 200 |
| Compound 27 | 13 | 2 | 23 | < 200 |
| Compound 28 | 23 | 1 | 37 | < 200 |
| Compound 29 | 28 | 48 | 321 | - |
| Compound 30 | 39 | 20 | 318 | - |
| Compound 31 | 15 | 8 | 78 | < 200 |
| Compound 32 | 31 | 12 | 35 | < 200 |
| Compound 33 | 27 | 6 | 23 | < 200 |
| Compound 34 | 29 | 2 | 19 | < 200 |
| Compound 35 | 44 | - | - | - |
| Compound 37 | 57 | - | - | - |
| Compound 38 | 22 | - | - | - |

| | | | | |
|---|---|---|---|---|
| - means that it is not determined. | | | | |

It can be seen from the experimental results in Table 1 that the compounds of the present invention have excellent inhibitory activities on Ax1 (h), Mer (h), Tyro3 (h) and CSF1R (h). Therefore, the compounds of the present invention can be used to prevent and/or treat diseases mediated by Axl (h), Mer (h), Tyro-3 (h) and CSF1R (h).

### Experimental example 2: assay of inhibitory activities of compounds of the present invention on cell pAxl

MDA-MB-231 is a breast cancer cell.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

### Test method:

MDA-MB-231 cells were inoculated to a 6-well plate (containing 10% FBS DMEM medium, 5 base, ⁵ cells per well) and adherently cultivated at 37 °C and 5% CO₂ for 18 hrs. The cells were starved overnight, and then compound solutions with different concentrations were added. Compounds 1 and 2 had final concentrations of 1.1, 3.3 and 10 nM, and the remaining compounds had final concentrations of 1.1, 3.3, 10 and 30 nM. DMSO had a final concentration of 1‰. There was a medium containing 1‰ DMSO in the negative control well. The cells were incubated at 37 °C and 5% CO₂ for 60 min, and then hGAS6 (R&D, final concentration: 200 ng/mL) was added to each well. The cells were further incubated for 60 min, and then the total protein of the cells was extracted for Western Blot experiment. Gray analysis was performed on the results with Image J software, and IC₅₀ values were calculated with GraphPad 5.0 software. The inhibitory activities of the compounds on cell pAxl were detected.

The test results are shown in Table 2 below.

**Table 2 Inhibitory activities of compounds of the present invention on cell pAxl**

| Compound | IC₅₀ (nM) |
|---|---|
| Compound 1 | <1.1 |
| Compound 2 | <1.1 |
| Compound 4 | <1.1 |
| Compound 7 | ≤1.1 |
| Compound 8 | ≤1. |
| Compound 9 | <10 |
| Compound 13 | ≤3.1 |
| Compound 16 | <1.1 |
| Compound 17 | <1.1 |
| Compound 18 | <1.1 |
| Compound 19 | <1.1 |
| Compound 20 | <10 |
| Compound 24 | <10 |
| Compound 25 | ≤503 |
| Compound 26 | <3.3 |

It can be seen from the experimental results in Table 2 that the compounds of the present invention have significant inhibitory effects on MDA-MB-231 cell pAxl. It demonstrates that the compounds of the present invention can effectively inhibit the activity of Ax1 at the cellular level and are prominent Axl inhibitors.

### Experimental example 3: assay of inhibitory activities of compounds of the present invention on cell pMer

H1299 is a non-small cell lung cancer cell.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

### Test method:

H1299 cells were inoculated to a 6-well plate (containing 10% FBS 1640 medium, 5 × 10⁵ cells per well) and adherently cultivated at 37 °C and 5% CO₂ for 18 hrs. The cells were starved overnight, and then compound solutions with different concentrations were added. The compounds had final concentrations of 0.35, 1.1, 3.3 and 10 nM. DMSO had a final concentration of 1‰. There was a medium containing 1‰ DMSO in the negative control well. The cells were incubated at 37 °C and 5% CO₂ for 60 min, and then Human Mer Mab (R&D, final concentration: 200 ng/mL) was added to each well. The cells were further incubated for 60 min, and then the total protein of the cells was extracted for Western Blot experiment. Gray analysis was performed on the results with Image J software, and IC₅₀ values were calculated with GraphPad 5.0 software. The inhibitory activities of the compounds on cell pMer were detected.

The test results are shown in Table 3 below.

**Table 3 Inhibitory activities of compounds of the present invention on cell pMer**

| Compound | IC₅₀ (nM) |
|---|---|
| Compound 1 | 1.1-3.3 |
| Compound 2 | 1.1 |
| Compound 4 | 1.1 |
| Compound 9 | 1.1-3.3 |
| Compound 16 | 1.1 |
| Compound 17 | <0.35 |
| Compound 18 | <1.1 |
| Compound 19 | <0.35 |
| Compound 20 | 3.3-10 |
| Compound 27 | <1.1 |
| Compound 28 | 1.1 |
| Compound 31 | 3.3-10 |
| Compound 32 | 1.1 |

It can be seen from the experimental results in Table 3 that the compounds of the present invention have significant inhibitory effects on H1299 cell pMer. It demonstrates that the compounds of the present invention can effectively inhibit the activity of Mer at the cellular level and are prominent Mer inhibitors.

### Experimental example 4: assay of inhibitory activities of compounds of the present invention on cell pAxl

H1299 is a non-small cell lung cancer cell.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

### Test method:

H1299 cells were inoculated to a 6-well plate (containing 10% FBS 1640 medium, 5 × 10⁵ cells per well) and adherently cultivated at 37 °C and 5% CO₂ for 18 hrs. The cells were starved overnight, and then compound solutions with different concentrations were added. The compounds had final concentrations of 1.1, 3.3, 10 and 30 nM. DMSO had a final concentration of 1‰. There was a medium containing 1‰ DMSO in the negative control well. The cells were incubated at 37 °C and 5% CO₂ for 60 min, and then hGAS6 (R&D, final concentration: 200 ng/mL) was added to each well. The cells were further incubated for 60 min, and then the total protein of the cells was extracted for Western Blot experiment. IC₅₀ values were calculated with GraphPad 5.0 software, and the inhibitory activities of the compounds on cell pAx1 were detected.

The test results are shown in Table 4 below.

**Table 4 Inhibitory activities of compounds of the present invention on cell pAxl**

| Compound | IC₅₀ (nM) |
|---|---|
| Compound 27 | <1.1 |
| Compound 28 | 1.1 |
| Compound 29 | 30 |
| Compound 30 | >10 |
| Compound 31 | <3.3 |
| Compound 32 | <1.1 |
| Compound 33 | 3.3 |
| Compound 34 | <1.1 |

It can be seen from the experimental results in Table 4 that the compounds of the present invention have significant inhibitory effects on H1299 cell pAxl. It demonstrates that the compounds of the present invention can effectively inhibit the activity of Ax1 at the cellular level and are prominent Axl inhibitors.

### Experimental example 5: PK assay of compounds of the present invention in rat

### Administration and sampling:

The experimental compounds were each dissolved in a mixture of 5% DMSO + 20% (30% solutol) + 75% saline to prepare compound solutions. The compound solutions were intragastrically administered to SD rats at a dosage of 5.0 mg/kg. The blood was sampled at time points of 15 min, 30 min, 1 hr, 2 hrs, 4 hrs, 6 hrs, 8 hrs and 24 hrs after administration.

The experimental compounds were each dissolved in a mixture of 5% DMSO + 20% (30% solutol) + 75% saline to prepare compound solutions. The compound solutions were intravenously administered to SD rats at a dosage of 1 mg/kg. The blood was sampled at time points of 5 min, 15 min, 30 min, 1 hr, 2 hrs, 4 hrs, 6 hrs, 8 hrs and 24 hrs after administration.

The animals were fixed, and the tails were heated under a water bath 10 min before each sampling time. About 100 µL of blood was collected via the tail vein, and the collected blood was placed in an anticoagulant tube containing EDTA-K₂. The blood samples were centrifuged at 8,000 rpm for 6 min at 4 °C to obtain plasma samples, and the plasma samples were prepared within 30 min after the blood collection. The plasma samples were stored in a refrigerator at - 80 °C before test.

### Sample analysis method:

The sample to be tested was taken out from the -80 °C refrigerator, naturally thawed at room temperature, and vortexed for 5 min. 20 µL of plasma sample was pipetted to a 1.5 mL centrifuge tube, and 200 µL of internal standard working solution (a solution of tolbutamide in methanol, 100 ng/mL) was added. The resulting mixture was thoroughly mixed, vortexed for 5 min, and centrifuged at 12,000 rpm for 5 min. 50 µL of supernatant was precisely pipetted to a 96-well plate with 150 µL of water per well, and the resulting mixture was vortexed for 5 min and then used for LC-MS/MS analysis.

The test results are shown in Table 5 below.

**Table 5 PK parameters of the compounds in SD rats (IV: 1 mg/kg, PO: 5 mg/kg, n = 3)**

| Compound | t_{z1/2} iv/po (h) | V_{z_obs}iv (L/kg) | Cl_{_obs} iv (L/h/kg) | Tₘₐₓ po (h) | AUCₗₐₛₜ iv/po (h*ng/mL) | F% |
|---|---|---|---|---|---|---|
| Compound 2 | 3.47/3.14 | 1.19 | 0.24 | 4.00 | 4068/6624 | 31.3 |
| Compound 13 | 9.08/8.12 | 2.65 | 0.20 | 4.00 | 4220/9792 | 46.4 |
| Compound 17 | 3.98/3.50 | 1.18 | 0.21 | 6.00 | 4864/17236 | 70.7 |
| Compound 18 | 0.96/1.17 | 0.92 | 0.66 | 0.50 | 1503/2592 | 34.6 |
| Compound 19 | 2.44/2.56 | 1.30 | 0.37 | 1.00 | 2444/5449 | 40.5 |
| Compound 34 | 5.18/4.56 | 0.84 | 0.11 | 6.00 | 8440/28046 | 65.7 |

| | | | | | | |
|---|---|---|---|---|---|---|
| Notes: t_{z1/2}: terminal half-life, Cl_{_obs}: clearance rate, V_{z_obs}: apparent volume of distribution, Tₘₐₓ: time of maximum observed plasma concentration, AUCₗₐₛₜ: area under plasma concentration-time curve, 0-24 h; F%: absolute bioavailability | | | | | | |

It can be seen from Table 5 that the compounds of the present invention exhibit low clearance rate in SD rats, and all have an absolute bioavailability greater than 30%.

### Experimental example 6: evaluation on the liver microsomal stability of compounds of the present invention

### Composition of the incubation system:

| Substances to be added | Initial concentration | Proportion (%) | Final concentration |
|---|---|---|---|
| Phosphate buffer | 100 mM | 50 | 50 mM |
| MgCl₂ | 20 mM | 5 | 1 mM |
| Liver microsome | 20 mg protein/mL | 2.5 | 0.5 mg protein/mL |
| Water to be supplemented | - | 22.5 | - |
| Compound | 10 µM | 10 | 1 µM |
| β-NADPH | 10 mM | 10 | 1 mM |

### Preparation of test samples:

An appropriate amount of the compound was precisely weighed and dissolved in DMSO to prepare a 5.0 mM stock solution. The 5.0 mM stock solution was diluted with DMSO to 1.0 mM, and finally diluted with water to a 10 µM working solution of the test sample for use (the DMSO content in the reaction system was 0.1%).

### Test steps:

(1) The liver microsomes (20 mg protein/mL) were taken from a -80 °C refrigerator, pre-incubated on a 37 °C water bath thermostatic oscillator for 3 min, and thawed for use.
(2) An incubation system (without compound and β-NADPH) was prepared according to "composition of the incubation system" described above, and pre-incubated on a 37 °C water bath thermostatic oscillator for 2 min.
(3) Control group (without β-NADPH): 30 µL of water and 30 µL of compound working solution (10 µM) were added to 240 µL of the incubation system in step (2), and the reaction system was vortexed for 30 s and thoroughly mixed. The total volume of the reaction system was 300 µL. The sample was duplicated. The reaction solution was placed on a 37 °C water bath thermostatic oscillator for incubation, and timing was started. The sampling was conducted at 0 min and 60 min.
(4) Sample group: 70 µL of β-NADPH solution (10 mM) and 70 µL of compound working solution (10 µM) were added to 560 µL of the incubation system in step (2), and the total volume of the reaction system was 700 µL. The reaction solution was vortexed for 30 s and thoroughly mixed. The sample was duplicated. The reaction solution was placed on a 37 °C water bath thermostatic oscillator for incubation, and timing was started. The sampling was conducted at 0 min, 5 min, 10 min, 20 min, 30 min and 60 min.
(5) The reaction solution was vortexed for 3 min, and centrifuged at 12,000 rpm for 5 min.
(6) 50 µL of the supernatant was added to 150 µL of water, and the resulting solution was thoroughly vortexed and analyzed by LC/MS/MS.

The results are shown in Table 6 below.

**Table 6 Assay results of the liver microsomal stability of compounds of the present invention in mouse**

| Compound | Mice | |
|---|---|---|
| | CLᵢₙₜ (mL/min/mg) | t_{1/2} (min) |
| Compound 4 | 0.0072 | 193 |
| Compound 8 | 0.0072 | 193 |
| Compound 9 | 0.0282 | 49.1 |
| Compound 16 | 0.0066 | 210 |
| Compound 17 | 0.0124 | 112 |
| Compound 18 | 0.0120 | 116 |
| Compound 19 | 0.0102 | 136 |
| Compound 20 | 0.0062 | 224 |
| Compound 26 | 0.0132 | 105 |
| Compound 27 | 0.0046 | 301 |
| Compound 28 | 0.0160 | 86.6 |
| Compound 29 | 0.0144 | 96.3 |
| Compound 30 | 0.0168 | 82.5 |
| Compound 31 | 0.0036 | 385 |
| Compound 32 | 0.0166 | 83.5 |
| Compound 33 | 0.0048 | 289 |
| Compound 34 | 0.0044 | 315 |

It can be seen from Table 6 that the compounds of the present invention exhibit a low clearance rate and a long half-life, and thereby have excellent druggability. It indicates that the compounds of the present invention have excellent metabolic stability *in vivo.*

### Experimental example 7: assay of inhibitory activities of compounds of the present invention on cell pCSF1R

HEK293T is a human renal epithelial cell.

CSF1R plasmid.

Test samples: the compounds of the present invention, having structures shown above.

Test instruments: protein electrophoresis apparatus (made by Bio Rad), membrane transfer apparatus (made by Bio Rad), exposure apparatus (made by Tanon), and CO₂ cell incubator (made by Thermo).

### Test method:

HEK293T cells were inoculated to a 6-well plate (containing 10% FBS DMEM medium, 4 × 10⁵ cells per well) and adherently cultivated at 37 °C and 5% CO₂ for 6 hrs. Cells in each well were transfected with 2 µg of CSF1R plasmid, and incubated overnight at 37 °C and 5% CO₂. 24 hrs after transfection, compound solutions with different concentrations were added to the administration groups. The compounds had final concentrations of 30, 100, 300 and 1000 nM. DMSO had a final concentration of 1‰. There was a medium containing 1‰ DMSO in the negative control well. The cells were incubated at 37 °C and 5% CO₂ for 120 min, and then the total protein of the cells was extracted for Western Blot experiment. Gray analysis was performed on the results with Image J software, and IC₅₀ values were calculated with GraphPad 5.0 software. The inhibitory activities of the compounds on cell pCSF1R were detected.

Test result: IC₅₀ values of 0-1000 nM were determined in the assay of inhibitory activities of compounds of the present invention on cell pCSF1R.

The results show that the compounds of the present invention have a significant inhibitory effect on pCSF1R of CSF1R-transfected HEK293T cells, and the inhibitory effect increases as the dosage increases. It demonstrates that the compounds of the present invention can effectively inhibit the activity of CSF1R at the cellular level and are prominent CSF1R inhibitors.

### Industrial applicability

The present invention provides a novel TAM family kinase inhibitor compound, which has an excellent kinase inhibitory activity and can be used to prevent and/or treat diseases mediated by abnormal expression of TAM family kinase receptors and/or ligands thereof. In addition, the compound of the present invention can also target CSF1R kinase, and thus can be used to prevent and/or treat diseases mediated by abnormal expression of TAM family kinase and/or CSF1R kinase receptors and/or ligands thereof. Furthermore, the compound of the present invention can inhibit the growth, migration and/or drug resistance of a tumor caused by TAM family kinase and/or CSF1R kinase. In addition, with a long half-life and excellent metabolic stability in the body, the compound of the present invention can improve the medicament efficacy, reduce the medication burden on a patient, and improve the compliance of a patient.

## Claims

1. A compound of general formula (I) or a pharmaceutically acceptable salt, ester, stereoisomer or tautomer thereof: 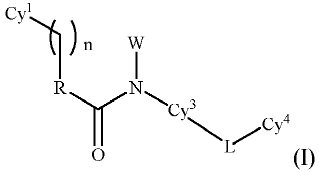
wherein, W is selected from hydrogen and optionally substituted C₁₋₆ alkyl;
R represents a group shown as the following general formula (a), (b) or (c), 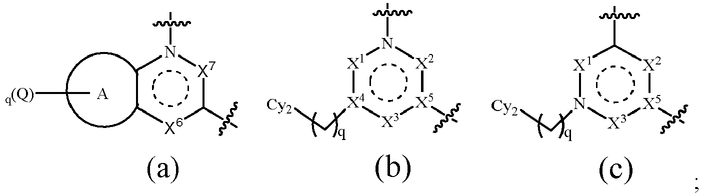
In formula (a), ring A represents 6-10 membered aromatic ring, 5-6 membered heteroaromatic ring having 1-3 heteroatoms selected from NR^{b}, O and S or 5-6 membered heterocyclic ring having 1-4 heteroatoms selected from NR^{b}, O and S; preferably, ring A represents benzene ring, furan ring, thiophene ring, pyrrole ring, oxazole ring, isoxazole ring, thiazole ring, isothiazole ring, pyrazole ring, imidazole ring, pyridine ring, pyridazine ring, pyrimidine ring, pyrazine ring, pyran ring, thiopyran ring, pyrrolidine ring, pyrroline ring, tetrahydrofuran ring, tetrahydrothiophene ring, piperidine ring or tetrahydropyran ring;
Q is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
q is an integer of 0 to 4; n is an integer of 0 to 4;
in formula (a), 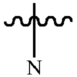 is linked to Cy¹ via a linking group;
in formula (a), X⁶ and X⁷ are each independently selected from CR^{a}R^{a}, C=O and NR^{b}, and preferably, at least one of X⁶ and X⁷ is C=O;
in formula (b), 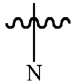 is linked to Cy¹ via a linking group;
in formula (c), 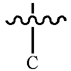 is linked to Cy¹ via a linking group;
X¹, X² and X³ are each independently selected from CR^{a}R^{a}, C=O, NR^{b} and O, and at least one of X¹, X² and X³ is C=O;
X⁴ and X⁵ are each independently selected from CR^{a} and N; preferably, X⁴ is selected from C and N, and X⁵ is selected from C;
Cy¹ is selected from 3-12 membered heterocyclyl optionally substituted with one or more R¹ and 3-12 membered cycloalkyl optionally substituted with one or more R¹, and R¹ is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C_{2- 8} alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy² is selected from 6-14 membered aryl optionally substituted with one or more R² and 5-10 membered heteroaryl optionally substituted with one or more R², and R² is each independently selected from the group consisting of hydrogen, cyano, hydroxyl, mercapto, halogen, carboxyl, nitro, -NR^{b}R^{c}, - C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, - NR^{b}SO₂R^{d}, optionally substituted C₁₋₆ alkyl, optionally substituted C₁₋₆ alkoxy, optionally substituted C_{2- 8} alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy³ is selected from the group consisting of 3-12 membered cycloalkyl optionally substituted with one or more R³, 3-14 membered heterocyclyl optionally substituted with one or more R³, 5-10 membered heteroaryl optionally substituted with one or more R³ and 6-14 membered aryl optionally substituted with one or more R³, and R³ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', - NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
Cy⁴ is selected from 3-12 membered cycloalkyl optionally substituted with one or more R⁴, 3-14 membered heterocyclyl optionally substituted with one or more R⁴, 5-14 membered heteroaryl optionally substituted with one or more R⁴ and 6-14 membered aryl optionally substituted with one or more R⁴, and R⁴ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl, or, two R⁴, together with the atoms attached thereto, can form 5-14 membered cyclic group;
L is selected from the group consisting of -NR^{b}-, -O-, -S-, (CR^{a}R^{a})ₘ optionally substituted 3-12 membered cycloalkyl, optionally substituted 3-14 membered heterocyclyl, optionally substituted 5-14 membered heteroaryl and optionally substituted 6-14 membered aryl, m is an integer of 0 to 3;
R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, - C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', -SO₂-R', optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
R' is selected from the group consisting of optionally substituted 3-12 membered cycloalkyl, optionally substituted 3-12 membered cycloalkenyl, optionally substituted 3-12 membered heterocyclyl, optionally substituted 6-14 membered aryl and optionally substituted 5-10 membered heteroaryl;
the substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-12 membered cycloalkyl, 6-14 membered aryl, 3-12 membered heterocyclyl, 5-10 membered heteroaryl and oxo; 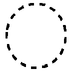 in formula (a), (b) and (c) represents a double bond optionally present in the ring structure;
with the proviso that when the ring in formula (b) carries two carbonyls, Cy¹ represents 3-12 membered heterocyclyl that is optionally substituted with one or more R¹ and contains 1-3 heteroatoms selected from O, S, S(O) and S(O)₂, and Cy³-L does not form aryloxy;
when, in group shown as formula (c), at least one of X¹ and X³ represents NR^{b}, Cy¹ represents 3-12 membered heterocyclyl that is optionally substituted with one or more R¹ and contains 1-3 heteroatoms selected from O, S, S(O) and S(O)₂;
when, in group shown as formula (b), X¹ and X² are CH, X³ is C=O, and X⁴ and X⁵ are C, L does not represent heteroaryl;
when, in group shown as formula (c), X¹ and X² are CH, X³ is C=O, and X⁵ is C, Cy¹ represents 3-12 membered heterocyclyl that is optionally substituted with one or more R¹ and contains 1-3 heteroatoms selected from O, S, S(O) and S(O)₂, and Cy² represents 6-14 membered aryl optionally substituted with one or more R²; and
the case where two carbonyls are directly bonded in the ring structure is impossible.

2. The compound or the pharmaceutically acceptable salt, ester, stereoisomer or tautomer thereof according to claim 1, wherein,
R represents a group shown as formula (b) or (c);
q represents 0, 1, 2 or 3;
X¹, X² and X³ are each independently selected from CR^{a}R^{a}, C=O, NR^{b} and O, and at least one of X¹, X² and X³ is C=O; and
X⁴ and X⁵ are each independently selected from CR^{a} and N.

3. The compound or the pharmaceutically acceptable salt, ester, stereoisomer or tautomer thereof according to any one of claims 1 to 2, having a structure of general formula (II), 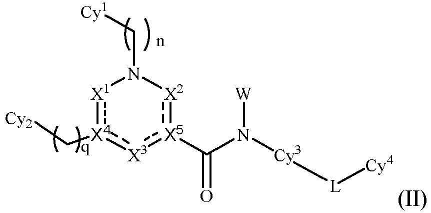
wherein, -̅-̅-̅-̅ represents a single bond or a double bond;
q represents 0, 1, 2 or 3;
X¹, X² and X³ are each independently selected from CR^{a}R^{a}, C=O and NR^{b}, and at least one of X¹, X² and X³ is C=O; and
X⁴ and X⁵ are each independently selected from CR^{a} and N.

4. The compound or the pharmaceutically acceptable salt, ester, stereoisomer or tautomer thereof according to any one of claims 1 to 2, having a structure of general formula (III) or (IV), 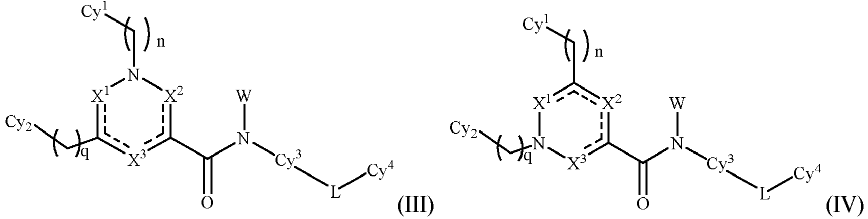 wherein,
-̅-̅-̅-̅ represents a single bond or a double bond;
q represents 0, 1, 2 or 3; and
X¹, X² and X³ are each independently selected from CR^{a}R^{a}, C=O and NR^{b}, and at least one of X¹, X² and X³ is C=O.

5. The compound or the pharmaceutically acceptable salt, ester, stereoisomer or tautomer thereof according to any one of claims 1 to 4, wherein, Cy¹ is selected from 4-10 membered heterocyclyl optionally substituted with one or more R¹ and 3-10 membered cycloalkyl optionally substituted with one or more R¹, preferably, Cy¹ is selected from 4-8 membered heterocyclyl optionally substituted with one or more R¹, and more preferably, Cy¹ is selected from 4-6 membered heterocyclyl that is optionally substituted with one or more R¹ and contains 1-3 heteroatoms selected from O, NR^{b}, S, S(O) and S(O)₂;
R¹ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl, preferably, R¹ is each independently selected from the group consisting of hydrogen, hydroxyl, mercapto, halogen, carboxyl, nitro, -NR^{b}R^{c}, -C(O)R^{d}, -C(O)OR^{d}, - C(O)NR^{b}R^{c}, -NR^{b}C(O)R^{d}, optionally substituted C₁₋₆ alkyl and optionally substituted C₁₋₆ alkoxy, more preferably, R¹ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy, and further preferably, R¹ is each independently selected from hydrogen, hydroxyl, fluorine, chlorine, bromine and C₁₋₄ alkyl;
Cy² is selected from 6-10 membered aryl optionally substituted with one or more R² and 5-6 membered heteroaryl optionally substituted with one or more R², and preferably, Cy² is selected from phenyl, naphthyl and pyridyl that are optionally substituted with one or more R²;
R² is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl, preferably, R² is each independently selected from the group consisting of hydrogen, hydroxyl, mercapto, halogen, carboxyl, nitro, -NR^{b}R^{c}, -C(O)R^{d}, -C(O)OR^{d}, - C(O)NR^{b}R^{c}, -NR^{b}C(O)R^{d}, optionally substituted C₁₋₆ alkyl and optionally substituted C₁₋₆ alkoxy, more preferably, R² is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy, and further preferably, R² is each independently selected from hydrogen, hydroxyl, fluorine, chlorine, bromine and C₁₋₄ alkyl;
Cy³ is selected from the group consisting of 3-8 membered cycloalkyl optionally substituted with one or more R³, 5-6 membered heteroaryl optionally substituted with one or more R³ and 6-10 membered aryl optionally substituted with one or more R³; preferably, Cy³ is selected from the group consisting of 5-6 membered heteroaryl optionally substituted with one or more R³, 3-6 membered cycloalkyl optionally substituted with one or more R³, phenyl or naphthyl optionally substituted with one or more R³, thienyl optionally substituted with one or more R³ and cyclohexyl optionally substituted with one or more R³; more preferably, Cy³ is a group shown as 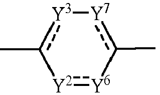 optionally substituted with one or more R³, and in the formula, -̅-̅-̅-̅ represents a single bond or a double bond, Y², Y³, Y⁶ and Y⁷ are each independently selected from CR^{a}R^{a} and NR^{b}, and preferably, at least one of Y², Y³, Y⁶ and Y⁷ is NR^{b}; and further more preferably, Cy³ is a group shown as 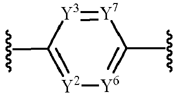 optionally substituted with one or more R³, and in the formula, Y², Y³, Y⁶ and Y⁷ are each independently selected from CH and N, and at least one of Y², Y³, Y⁶ and Y⁷ is N;
R³ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl, preferably, R³ is each independently selected from the group consisting of hydrogen, hydroxyl, mercapto, halogen, carboxyl, nitro, -NR^{b}R^{c}, -C(O)R^{d}, -C(O)OR^{d}, - C(O)NR^{b}R^{c}, -NR^{b}C(O)R^{d}, optionally substituted C₁₋₆ alkyl and optionally substituted C₁₋₆ alkoxy, more preferably, R³ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy and halogenated C₁₋₆ alkoxy, and further preferably, R³ is each independently selected from hydrogen, hydroxyl, fluorine, chlorine, bromine and C₁₋₄ alkyl; Cy
⁴ is selected from 5-10 membered heteroaryl optionally substituted with one or more R⁴ and 6-10 membered aryl optionally substituted with one or more R⁴; preferably, Cy⁴ is selected from 9-10 membered heteroaryl optionally substituted with one or more R⁴ and phenyl or naphthyl optionally substituted with one or more R⁴; more preferably, Cy⁴ is a group shown as 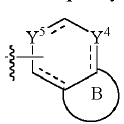 optionally substituted with one or more R⁴, and in the formula, -̅-̅-̅-̅ represents a single bond or a double bond, Y⁴ and Y⁵ are each independently selected from CR^{a}R^{a} and NR^{b}, and at least one of Y⁴ and Y⁵ is NR^{b}, and ring B is benzene ring, naphthalene ring or 5-10 membered heteroaromatic ring; and further more preferably, Cy⁴ is a group shown as 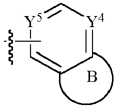 optionally substituted with one or more R⁴, and in the formula, Y⁴ and Y⁵ are each independently selected from C and N, and at least one of Y⁴ and Y⁵ is N, and preferably, ring B is benzene ring or 5-6 membered heteroaromatic ring having 1-3 heteroatoms selected from NR^{b}, O and S;
R⁴ is each independently selected from the group consisting of hydrogen, cyano, hydroxyl, mercapto, halogen, carboxyl, nitro, -NR^{b}R^{c}, -C(O)R^{d}, -C(O)OR^{d}, -C(O)NR^{b}R^{c}, -OC(O)NR^{b}R^{c}, -NR^{b}C(O)OR^{d}, - NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-12 membered heterocyclyl, optionally substituted 6-10 membered aryl and optionally substituted 5-10 membered heteroaryl, or, two R⁴, together with the atoms attached thereto, form 5-10 membered cyclic group; preferably, R⁴ is each independently selected from the group consisting of hydrogen, hydroxyl, mercapto, halogen, carboxyl, nitro, -NR^{b}R^{c}, -C(O)R^{d}, -C(O)OR^{d}, -C(O)NR^{b}R^{c}, - NR^{b}C(O)R^{d}, optionally substituted C₁₋₆ alkyl and optionally substituted C₁₋₆ alkoxy, or, two R⁴, together with the atoms attached thereto, form 5-6 membered cyclic group; more preferably, R⁴ is each independently selected from the group consisting of hydrogen, hydroxyl, mercapto, halogen, C₁₋₆ alkyl, hydroxy C₁₋₆ alkyl, amino C₁₋₆ alkyl, halogenated C₁₋₆ alkyl, C₁₋₆ alkoxy, halogenated C₁₋₆ alkoxy, C₁₋₆ alkoxy C₁₋₆ alkyl, C₁₋₆ alkyl C₁₋₆ alkoxy and C₁₋₆ alkoxy C₁₋₆ alkoxy, or, two R⁴, together with the atoms attached thereto, form 5-6 membered oxygen-containing cyclic group; and further more preferably, R⁴ is each selected from the group consisting of hydrogen, hydroxyl, mercapto, halogen, C₁₋₄ alkyl, halogenated C₁₋₄ alkyl, C₁₋₄ alkoxy, C₁₋₄ alkoxy C₁₋₄ alkyl, C₁₋₄ alkyl C₁₋₄ alkoxy, C₁₋₄ alkoxy C₁₋₄ alkoxy and halogenated C₁₋₄ alkoxy, or, two R⁴, together with the atoms attached thereto, form 5-6 membered oxygen-containing cyclic group;
L is selected from -NR^{b}-, -O-, -S- and optionally substituted 5-10 membered heteroaryl, and preferably, L is selected from -O- and optionally substituted pyridyl;
R^{a} is present or absent, and when present, R^{a} is each independently selected from the group consisting of hydrogen, cyano, hydroxyl, mercapto, halogen, carboxyl, nitro, -NR^{b}R^{c}, -C(O)R^{d}, - C(O)OR^{d}, -C(O)NR^{b}R^{c}, -OC(O)NR^{b}R^{c}, -NR^{b}C(O)OR^{d}, -NR^{b}C(O)R^{d}, -SO₂-NR^{b}R^{c}, -SO₂R^{d}, -NR^{b}SO₂R^{d}, optionally substituted C₁₋₆ alkyl, optionally substituted C₁₋₆ alkoxy, optionally substituted C₂₋₈ alkenyl, optionally substituted C₂₋₈ alkynyl, optionally substituted -C₁₋₆ alkyl-R', optionally substituted -C₁₋₆ alkoxy-R', -O-R', -C(O)-R', -SO₂-R', -NR^{b}C(O)-R', optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl; preferably, R^{a} is present or absent, and when present, R^{a} is each independently selected from the group consisting of hydrogen, cyano, hydroxyl, mercapto, halogen, carboxyl, nitro, C₁₋₆ alkyl, C₁₋₆ alkoxy, halogenated C₁₋₆ alkyl; and more preferably, R^{a} is present or absent, and when present, R^{a} is each independently selected from hydrogen, C₁₋₆ alkyl and C₁₋₆ alkoxy;
R^{b}, R^{c} and R^{d} are each present or absent, and when present, R^{b}, R^{c} and R^{d} are each independently selected from the group consisting of hydrogen, hydroxyl, mercapto, optionally substituted C₁₋₆ alkyl, optionally substituted C₁₋₆ alkoxy, optionally substituted -C₁₋₆ alkyl-R', optionally substituted -C₁₋₆ alkoxy-R', -O-R', -C(O)-R', -SO₂-R', optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl; preferably, R^{b}, R^{c} and R^{d} are each present or absent, and when present, R^{b}, R^{c} and R^{d} are each independently selected from the group consisting of hydrogen, cyano, hydroxyl, mercapto, halogen, carboxyl, nitro, C₁₋₆ alkyl, C₁₋₆ alkoxy and halogenated C₁₋₆ alkyl; and more preferably, R^{b}, R^{c} and R^{d} are each present or absent, and when present, R^{b}, R^{c} and R^{d} are each independently selected from hydrogen, C₁₋₆ alkyl and C₁₋₆ alkoxy;
R' is selected from the group consisting of optionally substituted 3-8 membered cycloalkyl, optionally substituted 3-8 membered cycloalkenyl, optionally substituted 3-10 membered heterocyclyl, optionally substituted 6-10 membered aryl and optionally substituted 5-6 membered heteroaryl; and
substituents involved in the phrase "optionally substituted" are each independently selected from the group consisting of hydroxyl, mercapto, amino, carboxyl, cyano, nitro, halogen, C₁₋₆ alkyl, C₁₋₆ alkoxy C₁₋₆ alkyl, C₁₋₆ alkoxy, C₁₋₆ alkyl C₁₋₆ alkoxy, C₁₋₆ alkoxy C₁₋₆ alkoxy, C₁₋₆ alkylamino, (C₁₋₆ alkyl)₂amino, C₁₋₆ alkylester, C₁₋₆ alkylaminocarbonyl, (C₁₋₆ alkyl)₂aminocarbonyl, C₁₋₆ alkylcarbonyl, C₁₋₆ alkylcarbonyloxy, C₁₋₆ alkylcarbonylamino, C₁₋₆ alkylsulfonylamino, halogenated C₁₋₆ alkyl, halogenated C₁₋₆ alkoxy, C₁₋₆ alkylsulfonyl, C₁₋₆ alkylthio, 3-8 membered cycloalkyl, 6-10 membered aryl, 3-8 membered heterocyclyl, 5-6 membered heteroaryl and oxo.

6. The compound or the pharmaceutically acceptable salt, ester, stereoisomer or tautomer thereof according to any one of claims 1 to 5, wherein,
X¹ is N, X² is CR^{a}, and X³ is C=O; or, X¹ is CR^{a}, X² is N, and X³ is C=O; or, X¹ is C=O, X² is CR^{a}, and X³ is C=O; or, X¹ is CR^{a}, X² is CR^{a}, and X³ is C=O.

7. The compound or the pharmaceutically acceptable salt, ester, stereoisomer or tautomer thereof according to any one of claims 1 to 6, wherein, the ring in the group shown as formula (b) is represented by any one of the following formulae, and in these formulae, -̅-̅-̅-̅ represents a single bond or a double bond, 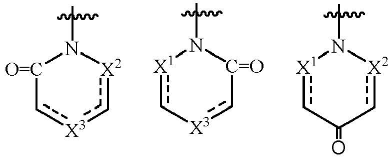 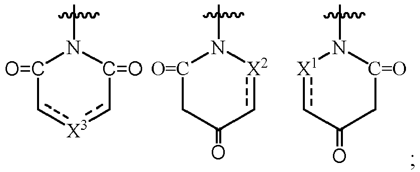 and
the ring in the group shown as formula (c) is represented by any one of the following formulae, and in these formulae, -̅-̅-̅-̅ represents a single bond or a double bond,

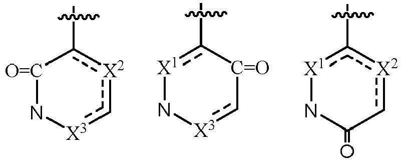

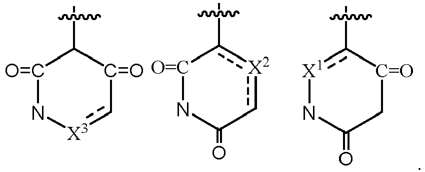

8. The compound or the pharmaceutically acceptable salt, ester, stereoisomer or tautomer thereof according to any one of claims 1 to 7, wherein,
Cy¹ is selected from the following groups optionally substituted with one or more R¹: 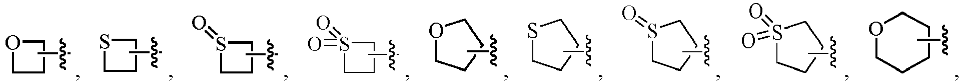 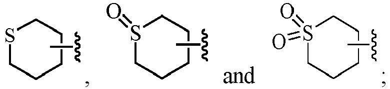 preferably, Cy¹ is selected from the following groups optionally substituted with one or more R¹: 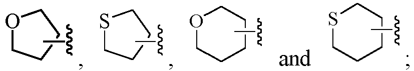 Cy² is selected from the following groups optionally substituted with one or more R²: 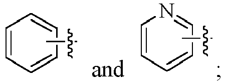 preferably, Cy² is selected from the following groups optionally substituted with one or more R²: 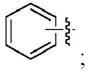 Cy³ is selected from the following groups optionally substituted with one or more R³: 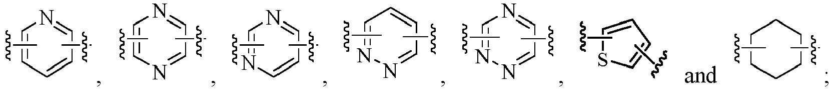 preferably, Cy³ is selected from the following groups optionally substituted with one or more R³: 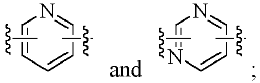 more preferably, Cy³ is selected from the following groups optionally substituted with one or more R³: 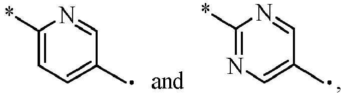 wherein * terminal is linked to N and · terminal is linked to L;
Cy⁴ is selected from the following groups optionally substituted with one or more R⁴: 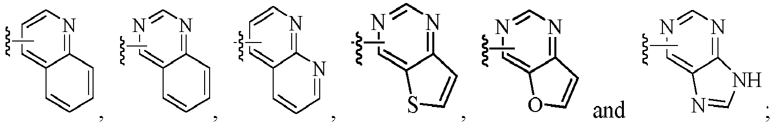 and
preferably, Cy⁴ is selected from the following group optionally substituted with one or more R⁴:

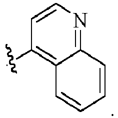

9. The compound or the pharmaceutically acceptable salt, ester, stereoisomer or tautomer thereof according to any one of claims 1 to 8, having a structure of general formula (V) or (VI), 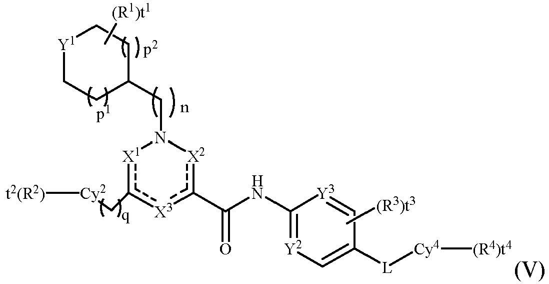 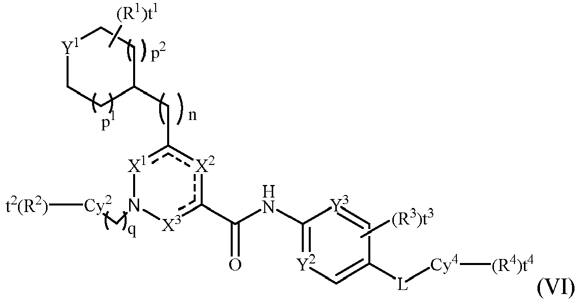
wherein, -̅-̅-̅-̅ represents a single bond or a double bond, X¹, X² and X³ are each independently selected from CR^{a}R^{a}, C=O and NR^{b}, and at least one of X¹, X² and X³ is C=O;
Y¹ is selected from O, S, S(O) and S(O)₂;
Y² and Y³ are each independently selected from C and N, and at least one of Y² and Y³ is N;
Cy⁴ is a group shown as 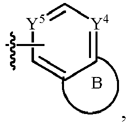 Y⁴ and Y⁵ are each independently selected from C and N, and at least one of Y⁴ and Y⁵ is N, and ring B is benzene ring or 5-6 membered heteroaromatic ring;
n is an integer of 0 to 2; q is an integer of 0 to 2;
t¹, t², t³ and t⁴ are each independently selected from integers of 1 to 5; and
p¹ and p² are each independently selected from integers of 0 to 2.

10. The compound or the pharmaceutically acceptable salt, ester, stereoisomer or tautomer thereof according to claim 9, wherein, X¹ is N, X² is CR^{a}, and X³ is C=O; or, X¹ is CR^{a}, X² is N, and X³ is C=O; or, X¹ is C=O, X² is CR^{a}, and X³ is C=O; or, X¹ is CR^{a}, X² is CR^{a}, and X³ is C=O.

11. The compound or the pharmaceutically acceptable salt, ester, stereoisomer or tautomer thereof according to any one of claims 1 to 10, wherein substituents involved in the phrase "optionally substituted" are each independently selected from the group consisting of hydroxyl, mercapto, amino, carboxyl, cyano, nitro, halogen, C₁₋₄ alkyl, C₁₋₄ alkoxy C₁₋₄ alkyl, C₁₋₄ alkoxy, C₁₋₄ alkyl C₁₋₄ alkoxy, C₁₋₄ alkoxy C₁₋₄ alkoxy, C₁₋₄ alkylamino, (C₁₋₄ alkyl)₂amino, C₁₋₄ alkylester, C₁₋₄ alkylaminocarbonyl, (C₁₋₄ alkyl)₂aminocarbonyl, C₁₋₄ alkylcarbonyl, C₁₋₄ alkylcarbonyloxy, C₁₋₄ alkylcarbonylamino, C₁₋₄ alkylsulfonylamino, halogenated C₁₋₄ alkyl, halogenated C₁₋₄ alkoxy, C₁₋₄ alkylsulfonyl, C₁₋₄ alkylthio, cyclopropyl, cyclobutyl, cyclopentyl, cyclohexyl, cycloheptyl, phenyl, naphthyl, oxa-cyclopropyl, oxa-cyclobutyl, oxa-cyclopentyl, oxa-cyclohexyl, oxa-cycloheptyl, pyrrolyl, furanyl, thienyl, oxazolyl, isoxazolyl, pyrazolyl, thiazolyl, pyridyl, pyrimidinyl, pyridazinyl and oxo.

12. The compound or the pharmaceutically acceptable salt, ester, stereoisomer or tautomer thereof according to any one of claims 1 to 11, having any one of the following structures:
| No. | Structure | No. | Structure |
|---|---|---|---|
| 1 | 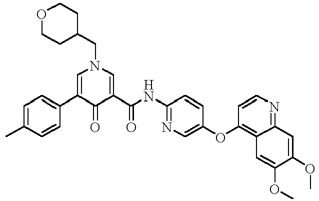 | 2 | 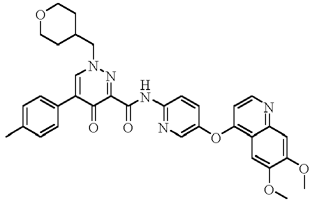 |
| 3 | 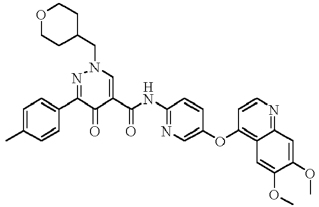 | 4 | 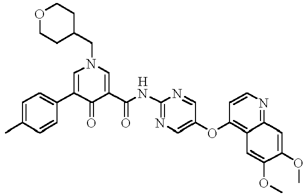 |
| 5 | 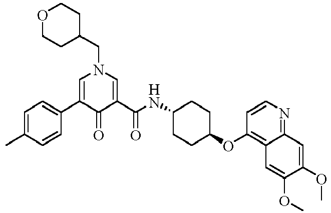 | 6 | 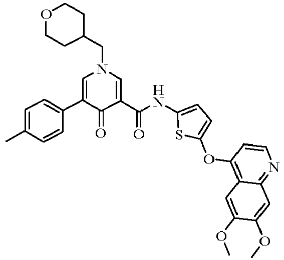 |
| 7 | 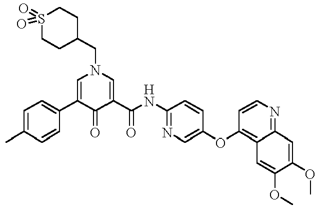 | 8 | 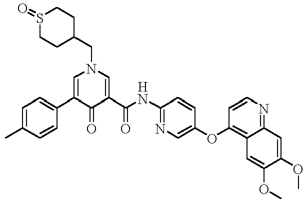 |
| 9 | 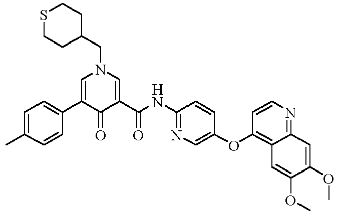 | 10 | 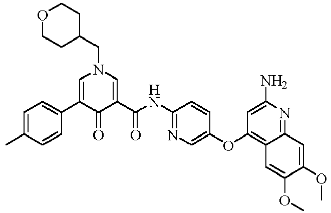 |
| 11 | 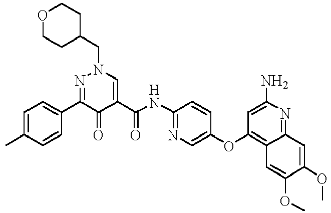 | 12 | 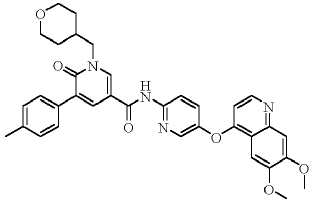 |
| 13 | 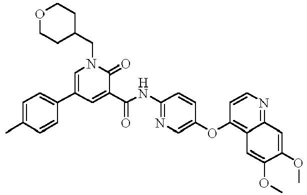 | 14 | 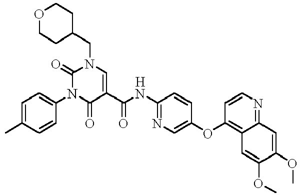 |
| 15 | 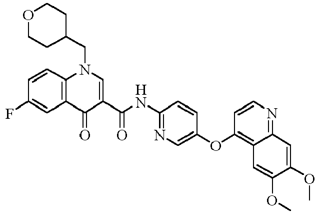 | 16 | 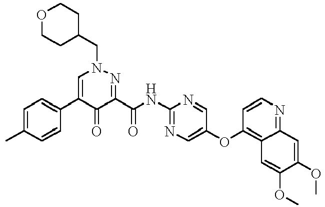 |
| 17 | 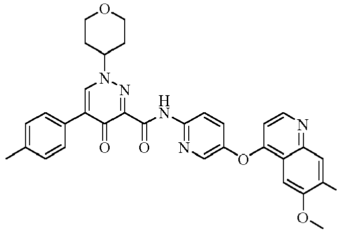 | 18 | 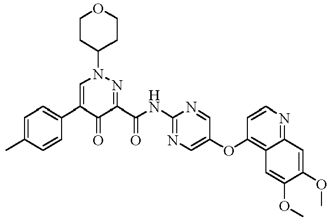 |
| 19 | 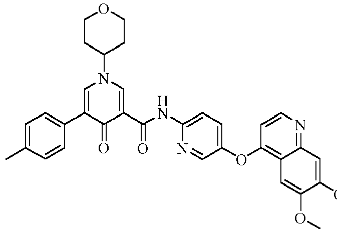 | 20 | 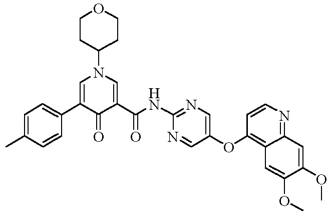 |
| 21 | 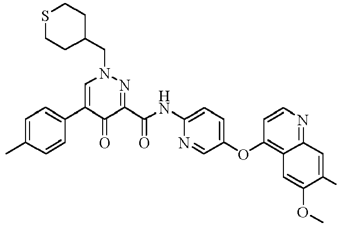 | 22 | 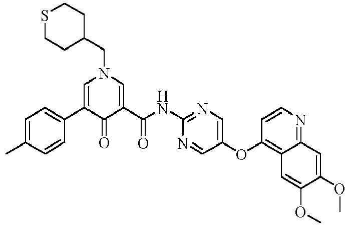 |
| 23 | 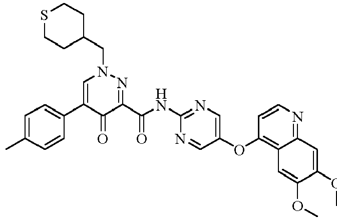 | 24 | 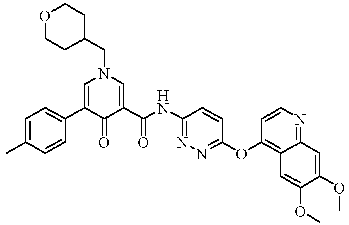 |
| 25 | 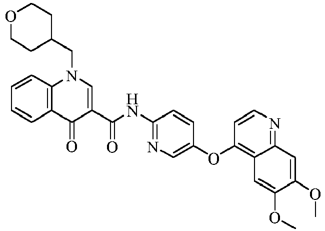 | 26 | 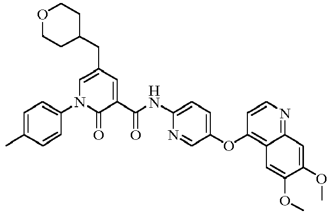 |
| 27 | 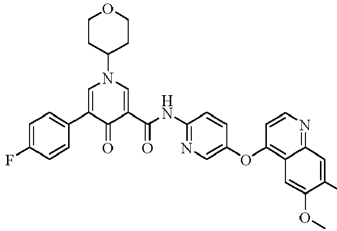 | 28 | 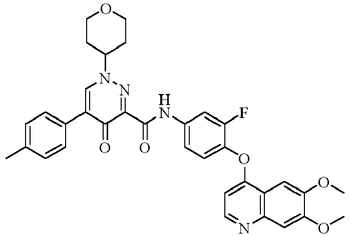 |
| 29 | 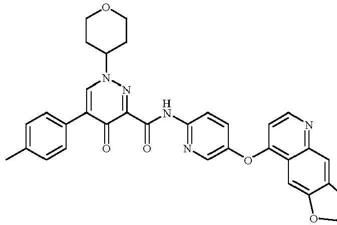 | 30 | 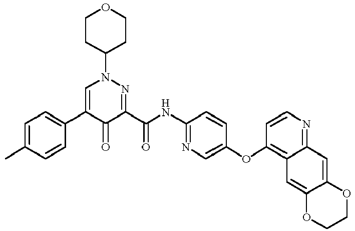 |
| 31 | 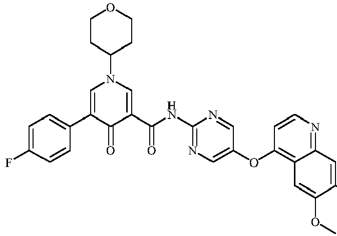 | 32 | 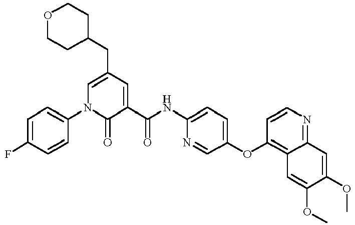 |
| 33 | 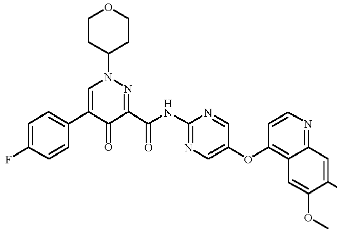 | 34 | 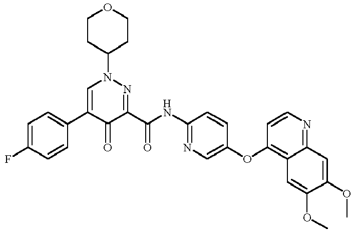 |
| 35 | 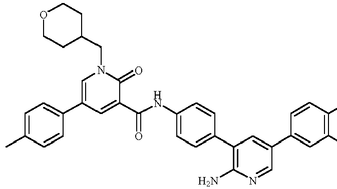 | 36 | 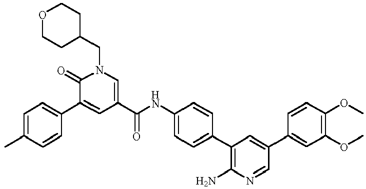 |
| 37 | 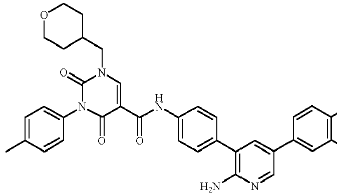 | 38 | 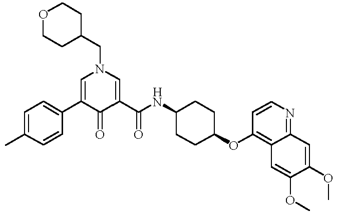 |
| 39 | 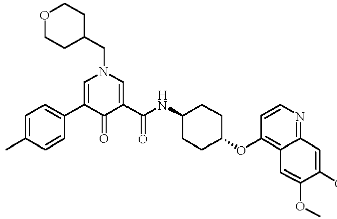 | 40 | 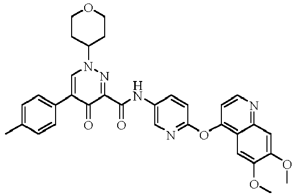 |
| 41 | 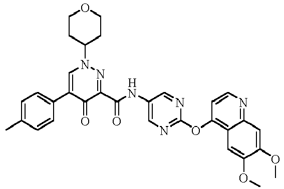 | 42 | 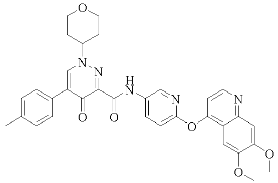 |
| 43 | 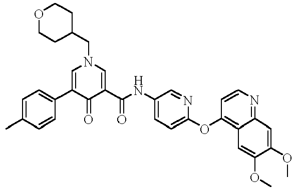 | 44 | 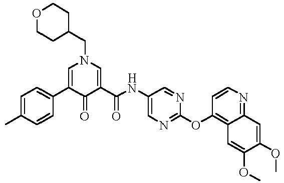 |
| 45 | 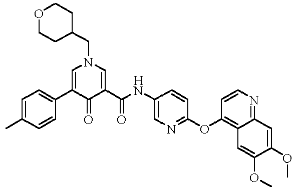 | 46 | 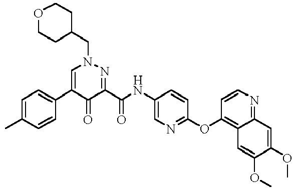 |
| 47 | 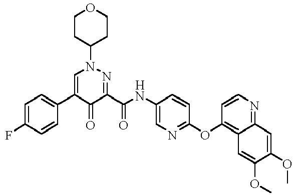 | 48 | 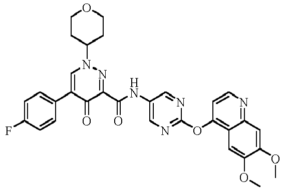 |
| 49 | 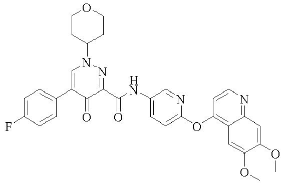 | | |

13. A pharmaceutical composition, comprising at least one of the compound and the pharmaceutically acceptable salt, ester, stereoisomer or tautomer thereof according to any one of claims 1 to 12.

14. The pharmaceutical composition according to claim 13, optionally comprising one or more pharmaceutical carriers.

15. The pharmaceutical composition according to claim 13, optionally comprising at least one second therapeutically active agent.

16. The pharmaceutical composition according to claim 15, wherein the second therapeutically active agent refers to at least one selected from the group consisting of antimetabolite, growth factor inhibitor, mitotic inhibitor, anti-tumor hormone, alkylating agent, metal preparation, topoisomerase inhibitor, hormone drug, immunomodulator, tumor suppressor gene, cancer vaccine, immune checkpoint- or tumor immunotherapy-related antibody, small molecule drug and cytotherapeutic agent.

17. A pharmaceutical formulation, comprising the compound or the pharmaceutically acceptable salt, ester, stereoisomer or tautomer thereof according to any one of claims 1 to 12, and optionally one or more pharmaceutical carriers.

18. Use of the compound or the pharmaceutically acceptable salt, ester, stereoisomer or tautomer thereof according to any one of claims 1 to 12, the pharmaceutical composition according to any one of claims 13 to 16, or the pharmaceutical formulation according to claim 17 for treating and/or preventing diseases mediated by abnormal expression of TAM family kinase receptors and/or CSF1R kinase receptors and/or ligands thereof, wherein the diseases mediated by abnormal expression of TAM family kinase receptors and/or CSF1R kinase receptors and/or ligands thereof include at least one of tumor, immune dysregulation, endometriosis, vascular disease/trauma, psoriasis, visual defect/lesion, kidney disease, rheumatoid arthritis, osteoporosis and related diseases.

19. Use of the compound or the pharmaceutically acceptable salt, ester, stereoisomer or tautomer thereof according to any one of claims 1 to 12, the pharmaceutical composition according to any one of claims 13 to 16, or the pharmaceutical formulation according to claim 17 for treating and/or preventing diseases mediated by abnormal expression of TAM family kinase receptors and/or ligands thereof, wherein the diseases mediated by abnormal expression of TAM family kinase receptors and/or ligands thereof include at least one of tumor, immune dysregulation, endometriosis, vascular disease/trauma, psoriasis, visual defect/lesion, kidney disease, rheumatoid arthritis, osteoporosis and related diseases.
